(19) Europäisches Patentamt
European Patent Office
Office européen des brevets

(11) EP 1 308 509 A1

(12) **EUROPEAN PATENT APPLICATION**
published in accordance with Art. 158(3) EPC

(43) Date of publication:
**07.05.2003 Bulletin 2003/19**

(21) Application number: **01955553.1**

(22) Date of filing: **01.08.2001**

(51) Int Cl.[7]: **C12N 15/09**

(86) International application number:
**PCT/JP01/06633**

(87) International publication number:
**WO 02/010371 (07.02.2002 Gazette 2002/06)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **01.08.2000 JP 2000232886**

(83) **Declaration under Rule 28(4) EPC (expert solution)**

(71) Applicant: **KYOWA HAKKO KOGYO CO., LTD.**
**Chiyoda-ku, Tokyo 100-8185 (JP)**

(72) Inventors:
- **SATOH, Mitsuo Tokyo Research Laboratories Machida-shi Tokyo 194-8533 (JP)**
- **SHIBATA, Kenji, T.R.L Kyowwa Hakko Kogyo Co. Ltd Machida-shi, Tokyo 194-8533 (JP)**
- **SEKINE, Susumu Tokyo Research Laboratories Kyowa Machida-shi Tokyo 194-8533 (JP)**
- **KOIKE, Masamichi Tokyo Research Lab. Kyowa Machida-shi Tokyo 194-8533 (JP)**
- **SAKURAI, Wataru Kita-ku, Tokyo 114-0024 (JP)**
- **FURUYA, Akiko Tokyo Research Laboratories Kyowa Machida-shi Tokyo 194-8533 (JP)**
- **MORI, Katsuhiro Tokyo Research Laboratories Kyowa Machida-shi Tokyo 194-8533 (JP)**
- **KATO, Yoko Tokyo Research Laboratories Kyowa Machida-shi Tokyo 194-8533 (JP)**

(74) Representative: **VOSSIUS & PARTNER**
**Siebertstrasse 4**
**81675 München (DE)**

# (54) NOVEL PHYSIOLOGICALLY ACTIVE PEPTIDE AND USE THEREOF

(57) The present invention provides a physiologically active peptide, a DNA encoding the peptide, an antibody which recognizes the peptide, and application methods thereof useful in screening and developing a therapeutic agent for diseases which accompany infection and inflammation, diseases which accompany abnormal differentiation and proliferation of smooth muscle cells, diseases which accompany abnormal activation of fibroblasts, diseases which accompany abnormal activation of a synovial tissue, diseases which accompany disorder of pancreatic β cells, diseases which accompany abnormality of osteoblasts or osteoclasts, diseases which accompany abnormal activation of immunocytes, diseases which accompany disorder of a blood vessel, diseases of an eye based on angiogenesis, diseases which accompany neopla, diseases in which linkage to gene regions of a major histocompatibility antigen is confirmed, and the like.

EP 1 308 509 A1

SEQUENCE LISTING

<110> KYOWA HAKKO KOGYO CO., LTD.

<120> AIF related peptides

<130> H 1180 EP
<160> 38
<170> PatentIn Ver. 2.0

<210> 1
<211> 39
<212> PRT
<213> Homo sapiens

<400> 1
```
Met Leu Glu Lys Leu Gly Val Pro Lys Thr His Leu Glu Leu Lys Lys
  1               5                  10                  15
Leu Ile Gly Glu Val Ser Ser Gly Ser Gly Glu Thr Phe Ser Tyr Pro
             20                  25                  30
Asp Phe Leu Arg Met Met Leu
         35
```

<210> 2
<211> 39
<212> PRT
<213> Mus musculus
<400> 2
```
Met Leu Glu Lys Leu Gly Val Pro Lys Thr His Leu Glu Leu Lys Arg
  1               5                  10                  15
Leu Ile Arg Glu Val Ser Ser Gly Ser Glu Glu Thr Phe Ser Tyr Ser
             20                  25                  30
Asp Phe Leu Arg Met Met Leu
         35
```

<210> 3
<211> 39
<212> PRT
<213> Sus scrofa
<400> 3
```
Met Leu Glu Lys Leu Gly Val Pro Lys Thr His Leu Glu Leu Lys Lys
  1               5                  10                  15
Leu Ile Lys Glu Val Ser Ser Gly Ser Gly Glu Thr Phe Ser Tyr Ser
             20                  25                  30
Ile Phe Leu Lys Met Met Leu
         35
```

<210> 4
<211> 39
<212> PRT
<213> Rattus norvegicus

<400> 4
```
Met Leu Glu Lys Leu Gly Val Pro Lys Thr His Leu Glu Leu Lys Lys
  1               5                  10                  15
Leu Ile Arg Glu Val Ser Ser Gly Ser Glu Glu Thr Phe Ser Tyr Ser
             20                  25                  30
Asp Phe Leu Arg Met Met Leu
         35
```

<210> 5

<211> 39
<212> PRT
<213> Homo sapiens

<400> 5
Met Met Glu Lys Leu Gly Val Pro Lys Thr His Leu Glu Met Lys Lys
1 5 10 15
Met Ile Ser Glu Val Thr Gly Gly Val Ser Asp Thr Ile Ser Tyr Arg
20 25 30
Asp Phe Val Asn Met Met Leu
35

<210> 6
<211> 39
<212> PRT
<213> Cyprinus carpio
<400> 6
Met Met Glu Lys Leu Gly Val Pro Lys Thr His Leu Glu Met Lys Lys
1 5 10 15
Met Ile Ser Glu Val Thr Gly Gly Cys Ser Asp Thr Ile Asn Tyr Arg
20 25 30
Asp Phe Val Lys Met Met Leu
35

<210> 7
<211> 39
<212> PRT
<213> Artificial Sequence

<220>
<221> MOD_RES
<222> (39)
<223> AMIDATION, Glycine amide

<400> 7
Leu Ser Pro His Thr Lys Leu Ser Tyr Gly Glu Gly Val Phe Leu Arg
1 5 10 15
Leu Ser Lys Ser Glu Gly Leu Met Glu Ile Lys Leu Met Glu Asp Leu
20 25 30
Met Val Phe Pro Lys Thr Xaa
35

<210> 8
<211> 39
<212> PRT
<213> Artificial Sequence

<220>
<221> MOD_RES
<222> (39)
<223> AMIDATION, Glycine amide

<400> 8
Leu Ser Pro His Thr Lys Leu Ser Tyr Arg Glu Glu Val Phe Leu Arg
1 5 10 15
Leu Ser Lys Ser Glu Gly Leu Met Glu Ile Lys Leu Met Glu Asp Leu
20 25 30
Met Val Phe Ser Arg Thr Xaa
35

<210> 9
<211> 39

```
<212> PRT
<213> Artificial Sequence

<220>
<221> MOD_RES
<222> (39)
<223> AMIDATION, Glycine amide

<400> 9
Val Thr Pro His Thr Lys Leu Val Tyr Ser Glu Ser Val Ile Met Asn
  1               5                  10                  15
Met Ser Lys Gly Glu Gly Leu Met Glu Ile Lys Leu Met Asp Asp Met
             20                  25                  30
Met Thr Phe Arg Lys Val Xaa
         35

<210> 10
<211> 23
<212> PRT
<213> Homo sapiens

<220>
<221> MOD_RES
<222> (39)
<223> AMIDATION, Leucine amide

<400> 10
Leu Ile Arg Glu Val Ser Ser Gly Ser Glu Glu Thr Phe Ser Tyr Ser
  1               5                  10                  15
Asp Phe Leu Arg Met Met Xaa
             20

<210> 11
<211> 117
<212> DNA
<213> Homo sapiens
<400> 11
atg ctg gag aaa ctt gga gtc ccc aag act cac cta gag cta aag aaa     48
Met Leu Glu Lys Leu Gly Val Pro Lys Thr His Leu Glu Leu Lys Lys
  1               5                  10                  15
tta att gga gag gtg tcc agt ggc tcc ggg gag acg ttc agc tac cct     96
Leu Ile Gly Glu Val Ser Ser Gly Ser Gly Glu Thr Phe Ser Tyr Pro
             20                  25                  30
gac ttt ctc agg atg atg ctg                                        117
Asp Phe Leu Arg Met Met Leu
         35

<210> 12
<211> 117
<212> DNA
<213> Mus musculus
<400> 12
atg ctg gag aaa ctt ggg gtt ccc aag acc cac cta gag ctg aag aga     48
Met Leu Glu Lys Leu Gly Val Pro Lys Thr His Leu Glu Leu Lys Arg
  1               5                  10                  15
tta att aga gag gtg tcc agt ggc tcc gag gag acg ttc agc tac tct     96
Leu Ile Arg Glu Val Ser Ser Gly Ser Glu Glu Thr Phe Ser Tyr Ser
             20                  25                  30
gac ttt ctc aga atg atg ctg                                        117
Asp Phe Leu Arg Met Met Leu
         35

<210> 13
<211> 117
```

<212> DNA
<213> Rattus norvegicus
<400> 13

```
atg ctg gag aaa ctt ggg gtt ccc aag acc cat cta gag ctg aag aaa    48
Met Leu Glu Lys Leu Gly Val Pro Lys Thr His Leu Glu Leu Lys Lys
 1               5                  10                  15
tta att aga gag gtg tcc agt ggc tcc gag gag acg ttc agt tac tct    98
Leu Ile Arg Glu Val Ser Ser Gly Ser Glu Glu Thr Phe Ser Tyr Ser
            20                  25                  30
gac ttt ctc aga atg atg ctg                                        117
Asp Phe Leu Arg Met Met Leu
        35
```

<210> 14
<211> 117
<212> DNA
<213> Homo sapiens
<400> 14

```
atg atg gag aag ctt ggt gtc ccc aag acc cac ctg gag atg aag aag    48
Met Met Glu Lys Leu Gly Val Pro Lys Thr His Leu Glu Met Lys Lys
 1               5                  10                  15
atg atc tca gag gtg aca gga ggg gtc agt gac act ata tcc tac cga    96
Met Ile Ser Glu Val Thr Gly Gly Val Ser Asp Thr Ile Ser Tyr Arg
            20                  25                  30
gac ttt gtg aac atg atg ctg                                        117
Asp Phe Val Asn Met Met Leu
        35
```

<210> 15
<211> 117
<212> DNA
<213> Mus musculus
<400> 15

```
atg atg gag aag ctg ggg gtc ccc aag acc cac ctg gag atg aag aag    48
Met Met Glu Lys Leu Gly Val Pro Lys Thr His Leu Glu Met Lys Lys
 1               5                  10                  15
atg atc tcg gag gtg aca ggg ggt gtc agt gac acc atc tcc tac cga    96
Met Ile Ser Glu Val Thr Gly Gly Val Ser Asp Thr Ile Ser Tyr Arg
            20                  25                  30
gac ttt gtg aat atg atg ctg                                        117
Asp Phe Val Asn Met Met Leu
        35
```

<210> 16
<211> 117
<212> DNA
<213> Cyprinus carpio
<400> 16

```
atg atg gag aag ttg ggt gtg cca aag act cac ctg gag atg aag aaa    48
Met Met Glu Lys Leu Gly Val Pro Lys Thr His Leu Glu Met Lys Lys
 1               5                  10                  15
atg atc tca gag gtg aca gga ggt tgc agc gac acc atc aac tac agg    96
Met Ile Ser Glu Val Thr Gly Gly Cys Ser Asp Thr Ile Asn Tyr Arg
            20                  25                  30
gac ttt gtg aaa atg atg ctt                                        117
Asp Phe Val Lys Met Met Leu
        35
```

<210> 17
<211> 27
<212> DNA
<213> Artificial Sequence
<220>
<223> Description of Artificial Sequence: Synthetic DNA

<400> 17
cttgtcgaca tgcaggtctc tgtcacg 27

<210> 18
<211> 28
<212> DNA
<213> Artificial Sequence
<220>
<223> Description of Artificial Sequence: Synthetic DNA
<400> 18
cttgtcgaca ctagttctct gtcatact 28

<210> 19
<211> 44
<212> DNA
<213> Artificial Sequence
<220>
<223> Description of Artificial Sequence: Synthetic DNA
<400> 19
cgcggccgca taacttcgta taatgtatgc tatacgaagt tatc 44

<210> 20
<211> 52
<212> DNA
<213> Artificial Sequence
<220>
<223> Description of Artificial Sequence: Synthetic DNA
<400> 20
tcgagataac ttcgtatagc atacattata cgaagttatg cggccgcggt ca 52

<210> 21
<211> 47
<212> DNA
<213> Artificial Sequence
<220>
<223> Description of Artificial Sequence: Synthetic DNA
<400> 21
gatctataac ttcgtataat gtatgctata cgaagttatg gatccat 47

<210> 22
<211> 45
<212> DNA
<213> Artificial Sequence
<220>
<223> Description of Artificial Sequence: Synthetic DNA
<400> 22
cgatggatcc ataacttcgt atagcataca ttatacgaag ttata 45

<210> 23
<211> 40
<212> PRT
<213> Artificial Sequence

<220>
<221> MOD_RES
<222> (40)
<223> AMIDATION, Leucine amide

<400> 23
Cys Met Met Glu Lys Leu Gly Val Pro Lys Thr His Leu Glu Met Lys
1 5 10 15
Lys Met Ile Ser Glu Val Thr Gly Gly Val Ser Asp Thr Ile Ser Tyr
20 25 30
Arg Asp Phe Val Asn Met Met Xaa

35 40

<210> 24
<211> 16
<212> PRT
<213> Artificial Sequence

<400> 24
Met Met Glu Lys Leu Gly Val Pro Lys Thr His Leu Glu Met Lys Cys
1 5 10 15

<210> 25
<211> 24
<212> PRT
<213> Artificial Sequence

<220>
<221> MOD_RES
<222> (25)
<223> AMIDATION, Leucine amide

<400> 25
Cys Leu Ile Gly Glu Val Ser Ser Gly Ser Gly Glu Thr Phe Ser Tyr
1 5 10 15
Pro Asp Phe Leu Arg Met Met Xaa
20

<210> 26
<211> 40
<212> PRT
<213> Artificial Sequence

<220>
<221> MOD_RES
<222> (40)
<223> AMIDATION, Leucine amide

<400> 26
Cys Met Leu Glu Lys Leu Gly Val Pro Lys Thr His Leu Glu Leu Lys
1 5 10 15
Lys Leu Ile Gly Glu Val Ser Ser Gly Ser Gly Glu Thr Phe Ser Tyr
20 25 30
Pro Asp Phe Leu Arg Met Met Xaa
35 40

<210> 27
<211> 40
<212> PRT
<213> Artificial Sequence

<220>
<221> MOD_RES
<222> (40)
<223> AMIDATION, Leucine amide

<400> 27
Cys Met Leu Glu Lys Leu Gly Val Pro Lys Thr His Leu Glu Leu Lys
1 5 10 15
Lys Leu Ile Arg Glu Val Ser Ser Gly Ser Glu Glu Thr Phe Ser Tyr
20 25 30
Ser Asp Phe Leu Arg Met Met Xaa
35 40

<210> 28

<211> 27
<212> DNA
<213> Artificial Sequence

<220>
<223> Description of Artificial Sequence: Symthetic DNA
<400> 28
aaagaattca tgagccaaac cagggat 27

<210> 29
<211> 31
<212> DNA
<213> Artificial Sequence

<220>
<223> Description of Artificial Sequence: Symthetic DNA
<400> 29
aaatttaagc ttatcagggc aactcagaga t 31

<210> 30
<211> 27
<212> DNA
<213> Artificial Sequence

<220>
<223> Description of Artificial Sequence: Symthetic DNA
<400> 30
aaagaattca tgtcgggcga gctcagc 27

<210> 31
<211> 31
<212> DNA
<213> Artificial Sequence

<220>
<223> Description of Artificial Sequence: Symthetic DNA
<400> 31
aaatttaagc ttatcagggc aggctagcaa t 31

<210> 32
<211> 444
<212> DNA
<213> Homo sapiens
<400> 32

```
atg agc caa acc agg gat tta cag gga gga aaa gct ttc gga ctg ctg    48
Met Ser Gln Thr Arg Asp Leu Gln Gly Gly Lys Ala Phe Gly Leu Leu
  1               5                  10                  15

aag gcc cag cag gaa gag agg ctg gat gag atc aac aag caa ttc cta    96
Lys Ala Gln Gln Glu Glu Arg Leu Asp Glu Ile Asn Lys Gln Phe Leu
             20                  25                  30

gac gat ccc aaa tat agc agt gat gag gat ctg ccc tcc aaa ctg gaa   144
Asp Asp Pro Lys Tyr Ser Ser Asp Glu Asp Leu Pro Ser Lys Leu Glu
         35                  40                  45

ggc ttc aaa gag aaa tac atg gag ttt gac ctt aat gga aat ggc gat   192
Gly Phe Lys Glu Lys Tyr Met Glu Phe Asp Leu Asn Gly Asn Gly Asp
     50                  55                  60

att gat atc atg tcc ctg aaa cga atg ctg gag aaa ctt gga gtc ccc   240
Ile Asp Ile Met Ser Leu Lys Arg Met Leu Glu Lys Leu Gly Val Pro
 65                  70                  75                  80
```

```
aag act cac cta gag cta aag aaa tta att gga gag gtg tcc agt ggc   288
Lys Thr His Leu Glu Leu Lys Lys Leu Ile Gly Glu Val Ser Ser Gly
                85                  90                  95

tcc ggg gag acg ttc agc tac cct gac ttt ctc agg atg atg ctg ggc   336
Ser Gly Glu Thr Phe Ser Tyr Pro Asp Phe Leu Arg Met Met Leu Gly
            100                 105                 110

aag aga tct gcc atc cta aaa atg atc ctg atg tat gag gaa aaa gcg   384
Lys Arg Ser Ala Ile Leu Lys Met Ile Leu Met Tyr Glu Glu Lys Ala
        115                 120                 125

aga gaa aag gaa aag cca aca ggc ccc cca gcc aag aaa gct atc tct   432
Arg Glu Lys Glu Lys Pro Thr Gly Pro Pro Ala Lys Lys Ala Ile Ser
    130                 135                 140

gag ttg ccc tga                                                   444
Glu Leu Pro
145
```

<210> 33
<211> 453
<212> DNA
<213> Homo sapiens
<400> 33

```
atg tcg ggc gag ctc agc aac agg ttc caa gga ggg aag gcg ttc ggc    48
Met Ser Gly Glu Leu Ser Asn Arg Phe Gln Gly Gly Lys Ala Phe Gly
  1               5                  10                  15

ttg ctc aaa gcc cgg cag gag agg agg ctg gcc gag atc aac cgg gag    96
Leu Leu Lys Ala Arg Gln Glu Arg Arg Leu Ala Glu Ile Asn Arg Glu
            20                  25                  30

ctt ctg tgt gac cag aag tac agt gat gaa gag aac ctt cca gaa aag   144
Leu Leu Cys Asp Gln Lys Tyr Ser Asp Glu Glu Asn Leu Pro Glu Lys
        35                  40                  45

ctc aca gcc ttc aaa gag aag tac atg gag ttt gac ctg aac aat gaa   192
Leu Thr Ala Phe Lys Glu Lys Tyr Met Glu Phe Asp Leu Asn Asn Glu
    50                  55                  60

ggc gag att gac ctg atg tct tta aag agg atg atg gag aag ctt ggt   240
Gly Glu Ile Asp Leu Met Ser Leu Lys Arg Met Met Glu Lys Leu Gly
65                  70                  75                  80

gtc ccc aag acc cac ctg gag atg aag aag atg atc tca gag gtg aca   288
Val Pro Lys Thr His Leu Glu Met Lys Lys Met Ile Ser Glu Val Thr
                85                  90                  95

gga ggg gtc agt gac act ata tcc tac cga gac ttt gtg aac atg atg   336
Gly Gly Val Ser Asp Thr Ile Ser Tyr Arg Asp Phe Val Asn Met Met
            100                 105                 110

ctg ggg aaa cgg tcg gct gtc ctc aag tta gtc atg atg ttt gaa gga   384
Leu Gly Lys Arg Ser Ala Val Leu Lys Leu Val Met Met Phe Glu Gly
        115                 120                 125

aaa gcc aac gag agc agc ccc aag cca gtt ggc ccc cct cca gag aga   432
Lys Ala Asn Glu Ser Ser Pro Lys Pro Val Gly Pro Pro Pro Glu Arg
    130                 135                 140

gac att gct agc ctg ccc tga                                       453
Asp Ile Ala Ser Leu Pro
145         150
```

```
<210>  34
<211>  147
<212>  PRT
<213>  Homo sapiens
<400>  34
Met Ser Gln Thr Arg Asp Leu Gln Gly Gly Lys Ala Phe Gly Leu Leu
 1               5                  10                  15
Lys Ala Gln Gln Glu Glu Arg Leu Asp Glu Ile Asn Lys Gln Phe Leu
            20                  25                  30
Asp Asp Pro Lys Tyr Ser Ser Asp Glu Asp Leu Pro Ser Lys Leu Glu
        35                  40                  45
Gly Phe Lys Glu Lys Tyr Met Glu Phe Asp Leu Asn Gly Asn Gly Asp
    50                  55                  60
Ile Asp Ile Met Ser Leu Lys Arg Met Leu Glu Lys Leu Gly Val Pro
65                  70                  75                  80
Lys Thr His Leu Glu Leu Lys Lys Leu Ile Gly Glu Val Ser Ser Gly
                85                  90                  95
Ser Gly Glu Thr Phe Ser Tyr Pro Asp Phe Leu Arg Met Met Leu Gly
            100                 105                 110
Lys Arg Ser Ala Ile Leu Lys Met Ile Leu Met Tyr Glu Glu Lys Ala
        115                 120                 125
Arg Glu Lys Glu Lys Pro Thr Gly Pro Pro Ala Lys Lys Ala Ile Ser
    130                 135                 140
Glu Leu Pro
145

<210>  35
<211>  151
<212>  PRT
<213>  Homo sapiens
<400>  35
Met Ser Gly Glu Leu Ser Asn Arg Phe Gln Gly Gly Lys Ala Phe Gly
 1               5                  10                  15
Leu Leu Lys Ala Arg Gln Glu Arg Arg Leu Ala Glu Ile Asn Arg Glu
            20                  25                  30
Leu Leu Cys Asp Gln Lys Tyr Ser Asp Glu Glu Asn Leu Pro Glu Lys
        35                  40                  45
Leu Thr Ala Phe Lys Glu Lys Tyr Met Glu Phe Asp Leu Asn Asn Glu
    50                  55                  60
Gly Glu Ile Asp Leu Met Ser Leu Lys Arg Met Met Glu Lys Leu Gly
65                  70                  75                  80
Val Pro Lys Thr His Leu Glu Met Lys Lys Met Ile Ser Glu Val Thr
                85                  90                  95
Gly Gly Val Ser Asp Thr Ile Ser Tyr Arg Asp Phe Val Asn Met Met
            100                 105                 110
Leu Gly Lys Arg Ser Ala Val Leu Lys Leu Val Met Met Phe Glu Gly
        115                 120                 125
Lys Ala Asn Glu Ser Ser Pro Lys Pro Val Gly Pro Pro Pro Glu Arg
    130                 135                 140
Asp Ile Ala Ser Leu Pro
145                 150

<210>  36
<211>  39
<212>  PRT
<213>  Artificial Sequence

<220>
<221> MOD_RES
<222> (39)
<223> AMIDATION, Leucine amide
```

<400> 36
Met Leu Glu Lys Leu Gly Val Pro Lys Thr His Leu Glu Leu Lys Lys
1 5 10 15
Leu Ile Gly Glu Val Ser Ser Gly Ser Gly Glu Thr Phe Ser Tyr Pro
20 25 30
Asp Phe Leu Arg Met Met Xaa
35

<210> 37
<211> 39
<212> PRT
<213> Artificial Sequence

<220>
<221> MOD_RES
<222> (39)
<223> AMIDATION, Leucine amide

<400> 37
Met Leu Glu Lys Leu Gly Val Pro Lys Thr His Leu Glu Leu Lys Arg
1 5 10 15
Leu Ile Arg Glu Val Ser Ser Gly Ser Glu Glu Thr Phe Ser Tyr Ser
20 25 30
Asp Phe Leu Arg Met Met Leu
35

<210> 38
<211> 39
<212> PRT
<213> Artificial Sequence

<220>
<221> MOD_RES
<222> (39)
<223> AMIDATION, Leucine amide

<400> 38

Met Met Glu Lys Leu Gly Val Pro Lys Thr His Leu Glu Met Lys Lys
1 5 10 15
Met Ile Ser Glu Val Thr Gly Gly Val Ser Asp Thr Ile Ser Tyr Arg
20 25 30

Asp Phe Val Asn Met Met Leu
35

## Description

### TECHNICAL FIELD

The present invention relates to a novel physiologically active peptide or derivatives thereof having an activity to change the concentration of intracellular calcium ions, a DNA encoding the peptide, an antibody which recognizes the peptide, and a diagnostic agent, a medicament and a therapeutic agent which use them.

### BACKGROUND OF THE INVENTION

The living body is constituted by a large number of cells and performs life activities through close connection among the cells. Physiologically active substances such as hormones, neurotransmitters, cell growth factors and the like relate to the close intercellular connection, and among the physiologically active substances, there are many substances in which signal transduction is carried out by changing the concentration of intracellular calcium ions.
The role of a calcium ion as an intracellular signal transduction substance was found in skeletal muscles by Ebashi *et al*. in 1960's [*Ann. Rev. Physiol*., 38, 293 (1976)].
Thereafter, it was shown by Ozawa *et al*. that a calcium ion does not only relate to muscle contraction but also relates to other enzyme reactions [*J. Biochem*., 61, 531 (1967)]. To date, it has been found that a calcium ion relates to various physiological phenomena such as excitation of nerves [*Ann. Rev. Physiol*., 60, 327 (1998)], secretion of physiologically active substances from secretory glands [*Cell Calcium,* 24, 367 (1998)] and cell growth or differentiation [*Cellular Signaling*, 9, 483 (1997)]. As the mechanism of introducing a calcium ion into cytoplasm, Michell suggested that phosphatidylinositol metabolic turnover plays an important role in changing the concentration of calcium ions in cytoplasm [*Biochim. Biophys. Acta*, 415, 81 (1975)]. Thereafter, it was found that a receptor of inositol 1,4,5-triphosphate (IP3) is present in endoplasmic reticulum which is an intracellular pool of calcium ions and that calcium ions are released via the receptor, so that a calcium ion is an intracellular signal transduction substance called second messenger *[Nature,* 342, 32 (1989)].
Thus, since dynamic changes of calcium ions inside the cells relates to many basic physiological phenomena, it is considered that change of the concentration of intracellular calcium ions in the living body is one of a very important method for treating human diseases. Accordingly, a substance having the activity to change the intracellular calcium concentration for specific cells is useful as a therapeutic agent for human diseases. For example, it is useful in screening and developing therapeutic agents for diseases which accompany infection and inflammation, diseases which accompany abnormal differentiation proliferation of smooth muscle cells, diseases which accompany abnormal activation of fibroblasts, diseases which accompany abnormal activation of a synovial tissue, diseases which accompany disorder of pancreatic β cells, diseases which accompany abnormality of osteoblasts or osteoclasts, diseases which accompany abnormal activation of immunocytes, diseases which accompany disorder of a blood vessel, diseases of an eye based on angiogenesis, diseases which accompany neoplasia, diseases in which linkage relation to gene regions of a major histocompatibility antigen is confirmed, and the like.
Although peptides having the activity to change the intracellular calcium concentration have been known, the presence of a novel physiologically active peptide is fully expected, and a possibility of providing a new therapeutic agent for human diseases is suggested.

### DISCLOSURE OF THE INVENTION

The present invention provides a physiologically active peptide, a DNA encoding the peptide, an antibody which recognizes the peptide, and application methods thereof useful in screening and developing a therapeutic agent for diseases which accompany infection and inflammation such as microbial infection, HIV infection, chronic hepatitis B, rheumatoid arthritis, sepsis, graft versus host diseases, insulin-dependent diabetes mellitus, glomerulonephritis, Crohn's disease, traumatic brain damage, inflammatory bowel diseases, *etc.* ; diseases which accompany abnormal differentiation and proliferation of smooth muscle cells such as arteriosclerosis, re-stricture, *etc.* ; diseases which accompany abnormal activation of fibroblasts such as psoriasis, *etc.*; diseases which accompany abnormal activation of a synovial tissue such as rheumatic arthritis, *etc.*; diseases which accompany disorder of pancreatic β cells such as diabetes mellitus, *etc.*; diseases which accompany abnormality of osteoblasts or osteoclasts such as osteoporosis, *etc.;* diseases which accompany abnormal activation of immunocytes such as allergy, atopy, asthma, pollinosis, airway oversensitivity, autoimmune diseases, *etc.*; diseases which accompany disorder of a blood vessel such as myocardial infarction, cerebral infarction, peripheral obstruction, angina pectoris, hypertension, diabetes mellitus, arteriosclerosis, SLE, *etc.*; diseases of an eye based on angiogenesis such as diabetic retinopathy, retinopathy of prematurity, senile macular degeneration, neovascular glaucoma, *etc.*; diseases which accompany neopla such as acute myelomonocytic leukemia, malignant tumor, *etc.;* diseases in which linkage to gene regions of a major histocompatibility antigen is confirmed such as insulin-dependent diabetes mellitus, rheumatoid arthritis, tetanic spondylitis, myasthenia gravis, IgA deficiency, Hashimoto's disease, Basedow's disease, Behcet's disease, *etc.*; and the like.

In order to solve the above problems, the present inventors have conducted intensive studies and, as a result, succeeded in obtaining novel physiologically active peptides. Thus, the present invention has been accomplished.

The present invention provides the following (1) to (52).

(1) A peptide represented by the following formula (I):

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence selected from the group consisting of the following (a) to (l)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;
(h) an amino acid sequence comprising amino acids of positions 17 to 39 in the amino acid sequence represented by SEQ ID NO:2;
(i) an amino acid sequence comprising amino acids of positions 17 to 39 in the amino acid sequence represented by SEQ ID NO:3;
(j) an amino acid sequence comprising amino acids of positions 17 to 39 in the amino acid sequence represented by SEQ ID NO:4;
(k) an amino acid sequence comprising amino acids of positions 17 to 39- in the amino acid sequence represented by SEQ ID NO:5;
(l) an amino acid sequence comprising amino acids of positions 17 to 39 in the amino acid sequence represented by SEQ ID NO:6.

(2) A peptide, which has an activity to change a concentration of an intracellular calcium ion, represented by the following formula (I):

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence in which one or more amino acids are substituted, deleted or added in the amino acid sequence selected from the group consisting of the following (a) to (l)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;

(h) an amino acid sequence comprising amino acids of positions 17 to 39 in the amino acid sequence represented by SEQ ID NO:2;

(i) an amino acid sequence comprising amino acids of positions 17 to 39- in the amino acid sequence represented by SEQ ID NO:3;

(j) an amino acid sequence comprising amino acids of positions 17 to 39 in the amino acid sequence represented by SEQ ID NO:4;

(k) an amino acid sequence comprising amino acids of positions 17 to 39 in the amino acid sequence represented by SEQ ID NO:5;

(l) an amino acid sequence comprising amino acids of positions 17 to 39 in the amino acid sequence represented by SEQ ID NO:6.

(3) A peptide, which has an activity to change a concentration of an intracellular calcium ion, represented by the following formula (I):

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence having a homology of 60% or more with the amino acid sequence selected from the group consisting of the following (a) to (f)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6.

(4) The peptide according to (2) or (3), wherein the activity to change a concentration of an intracellular calcium ion is an activity to increase a concentration of an intracellular calcium ion, or a pharmaceutically acceptable salt thereof.

(5) The peptide according to (2) or (3), wherein the cell is derived from a bone or a spleen, or a pharmaceutically acceptable salt thereof.

(6) A peptide comprising an amino acid sequence selected from the group consisting of the amino acid sequences represented by SEQ ID NOs:36, 37, 38 and 10.

(7) A DNA which encodes the peptide according to any one of (1) to (6).

(8) A DNA which comprises the nucleotide sequence represented by SEQ ID NO:11, 12, 13, 14, 15 or 16.

(9) A DNA which comprises a nucleotide sequence comprising nucleotides of positions 49 to 117 in the nucleotide sequence represented by SEQ ID NO:11, 12, 13, 14, 15 or 16.

(10) A DNA which hybridizes with the DNA according to any one of (7) to (9) under stringent conditions and has an activity to change a concentration of an intracellular calcium ion.

(11) The DNA according to (10), wherein the activity to change a concentration of an intracellular calcium ion is an activity to increase a concentration of an intracellular calcium ion.

(12) The DNA according to (10), wherein the cell is derived from a bone or a spleen.

(13) A recombinant DNA which is obtainable by inserting the DNA according to any one of (7) to (12) into a vector.

(14) A transformant which is obtainable by introducing the recombinant DNA according to (13) into a host cell.

(15) A process for producing a peptide, which comprises culturing the transformant according to (14) in a medium to thereby form and accumulate the peptide according to (1) to (6) in the culture, and recovering the peptide from the culture.

(16) An antibody which recognizes the peptide according to any one of (1) to (6).

(17) The antibody according to (16), which recognizes the amino acid sequence represented by SEQ ID NO:1 and recognizes the amino acid sequence represented by SEQ ID NO:5.

(18) The antibody according to (16) or (17), which recognizes the peptide represented by SEQ ID NO:36 and recognizes the peptide represented by SEQ ID NO:38.

(19) The antibody according to (16), which recognizes the amino acid sequence represented by SEQ ID NO:1 but does not recognize the amino acid sequence represented by SEQ ID NO:5.

(20) The antibody according to (16) or (19), which recognizes the peptide represented by SEQ ID NO:36 but does not recognize the peptide represented by SEQ ID NO:38.

(21) The antibody according to (16), which recognizes the amino acid sequence represented by SEQ ID NO:5 but does not recognize the amino acid sequence represented by SEQ ID NO:1.

(22) The antibody according to (16) or (21), which recognizes the peptide represented by SEQ ID NO:38 but does not recognize the peptide represented by SEQ ID NO:36.

(23) The antibody according to (16), which is an antibody having an activity to inhibit the peptide according to any one of (1) to (7).

(24) The antibody according to (23), wherein the peptide is represented by SEQ ID NO:36 or 38.

(25) A monoclonal antibody which is produced by a hybridoma KM2952 (FERN BP-7657).

(26) A monoclonal antibody which is produced by a hybridoma KM3022 (FERM BP-7658).

(27) A monoclonal antibody which is produced by a hybridoma KM3030 (FERM BP-7659).

(28) A monoclonal antibody which is produced by a hybridoma KM2947 (FERN BP-7656).

(29) An oligonucleotide which corresponds to a sequence comprising continuous 5 to 60 nucleotides in the nucleotide sequence contained in the DNA according to any one of (7) to (12), or an oligonucleotide which corresponds to a sequence complementary to the oligonucleotide.

(30) A method for detecting expression of a gene encoding the peptide according to any one of (1) to (6) by hybridization, which comprises using the DNA according to any one of (7) to (12).

(31) A method for detecting mutation of a gene encoding the peptide according to any one of (1) to (6) by hybridization, which comprises using the DNA according to any one of (7) to (12).

(32) An immunological detection method of the peptide according to any one of (1) to (6), which comprises using the antibody according to any one of (16) to (28).

(33) A tissue immunostaining method, which detecting the peptide according to any one of (1) to (6) by using the antibody according to any one of (16) to (28).

(34) A method for detecting expression of a gene encoding the peptide according to any one of (1) to (6) by polymerase chain reaction, which comprises using the oligonucleotide according to (29).

(35) A method for detecting expression of a gene encoding the peptide according to any one of (1) to (6) by polymerase chain reaction, which comprises using the oligonucleotide according to (29).

(36) A method for inhibiting transcription of a gene or translation of a mRNA encoding the peptide according to any one of (1) to (6), which comprises using the DNA according to any one of (7) to (12) or the oligonucleotide according to (29).

(37) A method for obtaining a promoter region of a gene encoding the peptide according to any one of (1) to (6), which comprise using the DNA according to any one of (7) to (12) or the oligonucleotide according to (29).

(38) A method for diagnosing diseases which accompany infection and inflammation, diseases which accompany abnormal differentiation proliferation of a smooth muscle cell, diseases which accompany abnormal activation of a fibroblast, diseases which accompany abnormal activation of a synovial tissue, diseases which accompany disorder of a pancreatic β cell, diseases which accompany abnormality of an osteoblast or an osteoclast, diseases which accompany abnormal activation of an immunocyte, diseases which accompany disorder of a blood vessel, diseases of an eye based on angiogenesis, diseases which accompany neopla or diseases in which linkage relation to a gene region of a major histocompatibility antigen is confirmed, which comprises using the method according to any one of (30) to (35).

(39) A medicament which comprises the peptide according to any one of (1) to (6) as an active ingredient.

(40) A medicament which comprises the DNA according to any one of (7) to (12) as an active ingredient.

(41) A medicament which comprises the antibody according to any one of (16) to (28) as an active ingredient.

(42) A medicament which comprises the oligonucleotide according to (29) as an active ingredient.

(43) The medicament according to any one of (39) to (42), which is a medicament for diagnosing, treating or preventing diseases which accompany infection and inflammation, diseases which accompany abnormal differentiation proliferation of a smooth muscle cell, diseases which accompany abnormal activation of a fibroblast, diseases which accompany abnormal activation of a synovial tissue, diseases which accompany disorder of a pancreatic β cell, diseases which accompany abnormality of an osteoblast or an osteoclast, diseases which accompany abnormal activation of an immunocyte, diseases which accompany disorder of a blood vessel, diseases of an eye based on angiogenesis, diseases which accompany neopla or diseases in which linkage relation to a gene region of a major histocompatibility antigen is confirmed.

(44) A method for screening a receptor which specifically interacts with the peptide according to any one of (1) to (6), which comprises using the peptide.

(45) A receptor which specifically interacts with the peptide according to any one of (1) to (6) which is obtainable by the screening method according to (44).
(46) A knockout non-human animal in which expression of a gene encoding the peptide according to any one of (1) to (6) is partially or completely suppressed.
(47) A knockout non-human animal in which the activity of the peptide according to any one of (1) to (6) is partially or completely suppressed.
(48) A method for screening an agonist for the peptide according to any one of (1) to (6), which comprises using the peptide.
(49) An agonist for the peptide according to any one of (1) to (6), which is identified by the method according to (48).
(50) A method for screening an antagonist for the peptide according to any one of (1) to (6), which comprises using the peptide.
(51) An antagonist for the peptide according to any one of (1) to (6), which is identified by the method according to (50).
(52) A method for analyzing expression of a gene encoding the peptide according to any one of (1) to (6), which comprises using the transformant according to (14).

The peptide of the present invention include:

(1) a peptide represented by the following formula (I):

$$R^1\text{-A-}R^2 \qquad \text{(I)}$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence selected from the group consisting of the following (a) to (1)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;
(h) an amino acid sequence comprising amino acids of positions 17 to 39 in the amino acid sequence represented by SEQ ID NO:2;
(i) an amino acid sequence comprising amino acids of positions 17 to 39 in the amino acid sequence represented by SEQ ID NO:3;
(j) an amino acid sequence comprising amino acids of positions 17 to 39 in the amino acid sequence represented by SEQ ID NO:4;
(k) an amino acid sequence comprising amino acids of positions 17 to 39 in the amino acid sequence represented by SEQ ID NO:5;
(l) an amino acid sequence comprising amino acids of positions 17 to 39 in the amino acid sequence represented by SEQ ID NO:6,

(2) a peptide, which has an activity to change a concentration of an intracellular calcium ion, represented by the following formula (I):

$$R^1\text{-A-}R^2 \qquad \text{(I)}$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence in which one or more amino acid are substituted, deleted or added in the amino acid sequence selected from the group consisting of the following (a) to (l)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;
(h) an amino acid sequence comprising amino acids of positions 17 to 39 in the amino acid sequence represented by SEQ ID NO:2;
(i) an amino acid sequence comprising amino acids of positions 17 to 39 in the amino acid sequence represented by SEQ ID NO:3;
(j) an amino acid sequence comprising amino acids of positions 17 to 39 in the amino acid sequence represented by SEQ ID NO:4;
(k) an amino acid sequence comprising amino acids of positions 17 to 39 in the amino acid sequence represented by SEQ ID NO:5;
(l) an amino acid sequence comprising amino acids of positions 17 to 39 in the amino acid sequence represented by SEQ ID NO:6,

(3) a peptide, which has an activity to change a concentration of an intracellular calcium ion, represented by the following formula (I):

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence having a homology of 60% or more with the amino acid sequence selected from the group consisting of the following (a) to (f)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6,

(4) the peptide according to (2) or (3), wherein the activity to change a concentration of an intracellular calcium ion is an activity to increase a concentration of an intracellular calcium ion, or a pharmaceutically acceptable salt thereof, and
(5) the peptide according to (2) or (3), wherein the cell is derived from a bone or a spleen, or a pharmaceutically acceptable salt thereof. Furthermore, . pharmaceutically acceptable salts of the peptide of the present invention are also included in the peptide of the present invention. Examples include a peptide comprising an amino acid sequence selected from the group consisting of the amino acid sequences represented by SEQ ID NOs:36, 37, 38 and 10.

The activity to change a concentration of an intracellular calcium ion against cells means an activity to increase or decrease a concentration of a calcium ion as an intracellular signal transduction substance, and the cell includes a bone, a splenocyte and the like.
In the definition of each group in formula (I), the alkanoyl include straight or branched alkanoyls having 1 to 20 carbon atoms, such as formyl, acetyl, propionyl, butyryl, isobutyryl, valeryl, isovaleryl, pivaloyl, hexanoyl, peptanoyl, lauroyl, eicosanoyl and the like.

In the definition of each group in formula (I), the alkanoyl include straight or branched alkenoyls having 1 to 20 carbon atoms, such as formyl, acetyl, propionyl, butyryl, isobutyryl, valeryl, isovaleryl, pivaloyl, hexanoyl, peptanoyl, lauroyl, eicosanoyl and the like.

The aryl moiety of the aroyl and the aryloxycarbonyl include aryls having 6 to 15 carbon atoms, such as phenyl, naphthyl and the like.

The heteroaryl moiety of heteroarylcarbonyl and heteroaryloxycarbonyl include furyl, thienyl, pyrrolyl, pyrazolyl, imidazolyl, oxazolyl, thiazolyl, pyridyl, pyridazinyl, pyrimidinyl, pyrazinyl, indolyl, indazolyl, benzimidazolyl, quinolyl, isoquinolyl, cinnolyl, quinazolinyl, quinoxalinyl, naphthyridinyl and the like.

The alkyl moiety of the alkoxycarbonyl and alkoxy include straight or branched alkyls having from 1 to 20 carbon atoms, such as methyl, ethyl, propyl, isopropyl, butyl, pentyl, hexyl, heptyl, decyl, dodecyl, eicosyl and the like.

The substituents in substituted alkanoyl, the . substituted alkoxycarbonyl and the substituted alkoxy are the same or different and 1 to 3 substituent(s), such as hydroxy; carboxy; alicyclic alkyl having 3 to 8 carbon atoms, e.g., cyclopropyl, cyclobutyl, cyclopentyl, cyclohexyl, cycloheptyl, cyclooctyl, *etc.*; substituted or unsubstituted phenyl; fluorenyl; and the like. The substituents in substituted phenyl are the same or different and 1 to 3 substituent(s), such as alkyl, alkoxy, hydroxy, nitro, sulfo, cyano, halogen and the like, and the halogen includes fluorine, chlorine, bromine and iodine. The alkyl moiety of the alkyl and the alkoxy as the substituents in the substituted phenyl is the same as the above alkyl moiety of the alkoxycarbonyl and the alkoxy.

The substituents in the substituted aroyl, the substituted aryloxycarbonyl, the substituted heteroarylcarbonyl and the substituted heteroaryloxycarbonyl are the same or different and 1 to 3 substituent(s) having the same meanings as the above substituents in the substituted phenyl.

The substituents in the substituted amino are the same or different and 1 or 2 substituent(s), such as substituted or unsubstituted alkyl, substituted or unsubstituted aryl and the like. The alkyl has the same meaning as the above alkyl moiety of the alkoxy and the like, and the substituents in the substituted alkyl have the same meanings as the above substituents in the substituted alkoxy and the like. The aryl has the same meaning as the above aryl moiety of the aroyl or the aryloxycarbonyl, and the substituents in the substituted aryl have the same meanings as the above substituents in the substituted aroyl or the substituted aryloxycarbonyl.

In formula (I), the amino acid residue which constitutes A is not limited to 20 L-amino acids known as essential amino acids, and it may include D-amino acids or β-amino acids other than α-amino acids, so long as they have the activity as a physiologically active peptide to change a concentration of an intracellular calcium ion, as an object of the present invention. Preferred amino acid residues as the constituting component other than the essential amino acids include L-tert-leucine (tLeu) and L-2-aminoadipic acid (Aad). Also, a side chain functional group of the amino acid residue which constitutes A may be chemically modified or protected. Examples of the amino acid residue whose side chain functional group is chemically modified or protected include an aspartic acid residue or a glutamic acid residue whose side chain carboxyl group is protected by benzyl ester, a carboxymethylated cysteine residue and the like.

In the amino acid sequence of formula (I), one or more amino acid substitution, deletion or addition means that one or more amino acids are substituted, deleted or added to at any one or plural positions in the amino acid sequence. The substitution, deletion and addition may be caused in the same amino acid sequence simultaneously. Also, the amino acid residue substituted or added can be natural or non-natural. The natural amino acid residue includes L-alanine, L-asparagine, L-aspartic acid, L-glutamine, L-glutamic acid, glycine, L-histidine, L-isoleucine, L-leucine, L-lysine, L-methionine, L-phenylalanine, L-proline, L-serine, L-threonine, L-tryptophan, L-tyrosine, L-valine and L-cysteine.

Hereinafter, examples of amino acid residues which are substituted with each other are shown. The amino acid residues in the same group can be substituted with each other.

Group A:

leucine, isoleucine, norleucine, valine, norvaline, alanine, 2-aminobutanoic acid, methionine, O-methylserine, t-butylglycine, t-butylalanine, cyclohexylalanine;

Group B:

aspartic acid, glutamic acid, isoaspartic acid, isoglutamic acid, 2-aminoadipic acid, 2-aminosuberic acid;

Group C:

asparagine, glutamine;

Group D:

lysine, arginine, ornithine, 2,4-diaminobutanoic acid, 2,3-diaminopropionic acid;

Group E:

proline, 3-hydroxyproline, 4-hydroxyproline;

Group F:

serine, threonine, homoserine;

Group G:

phenylalanine, tyrosine.

In order to provide the peptide of the present invention with the activity to change a concentration of an intracellular calcium ion, it is preferable that the amino acid sequence of A in the formula has a homology of at least 60% or more, generally 80% or more, particularly 95% or more, with the amino acid sequence represented by SEQ ID NO:1, 2, 3, 4, 5 or 6, when calculated using BLAST *(J. Mol. Biol.*, 215, 403 (1990)], FASTA *[Methods in Enzymology,* 183, 63-98 (1990)] or the like.
As amino acid sequences having a homology with the amino acid sequence represented by SEQ ID NO:1, The amino acid sequences represented by SEQ ID NOs:2, 3, 4, 5 and 6 are exemplified. These amino acids mutually have a homology of 60% or more.
The pharmaceutically acceptable salt includes an acid addition salts, metal salts, organic base addition salts and the like. The acid addition salts include inorganic acid salts such as hydrochloride, sulfate, phosphate and the like, and organic acid salts such as acetate, maleate, fumarate, tartarate, citrate and the like. The metal salts include alkali metal salts such as sodium salts, potassium salts, *etc*.; alkaline earth metal salts such as magnesium salts, calcium salts, *etc.;* aluminum salts, zinc salts and the like. The organic base addition salts include salts formed with a primary amine such as methylamine, ethylamine, aniline, *etc.* , a secondary amine such as dimethylamine, diethylamine, pyrrolidine, piperidine, morpholine, piperazine, *etc*.; or a tertiary amine such as trimethylamine, triethylamine, N,N-dimethylaniline, pyridine, *etc*.; ammonium salts; and the like.
The DNA of the present invention includes:

(a) a DNA encoding the peptide of the present invention defined above;
(b) a DNA comprising the nucleotide sequence represented by SEQ ID NO:11, 12, 13, 14, 15 or 16;
(c) a DNA which comprises a nucleotide sequence comprising nucleotides of positions 49 to 117 in the nucleotide sequence represented by SEQ ID NO:11, 12, 13, 14, 15 or 16;
(d) a DNA encoding a peptide which hybridizes with the DNA described in any one of the above (a) to (c) under stringent conditions and has an activity to change a concentration of an intracellular calcium ion;
(e) the DNA according to the above (d), wherein the cell is derived from a bone or a spleen; and
(f) the DNA according to the above (d) or (e), wherein the activity to change a concentration of an intracellular calcium ion is an activity to increase a concentration of an intracellular calcium ion.

In the present invention, a DNA which hybridizes under stringent conditions is a DNA obtained, e.g., by a method such as colony hybridization, plaque hybridization or Southern blot hybridization using the DNA of the present invention such as the DNA comprising the nucleotide sequence represented by SEQ ID NO:11 or a partial fragment thereof as the probe. The DNA includes a DNA which can be identified by carrying out hybridization at 65°C in the presence of 0.7 to 1.0 M sodium chloride using a filter to which colony- or plaque-derived DNA fragments are immobilized, and then washing the filter at 65°C using 0.1-fold to 2-fold SSC solution (composition of the 1-fold SSC solution comprising 150 mM sodium chloride and 15 mM sodium citrate). The hybridization can be carried out according to the method described in *Molecular Cloning, A Laboratory Manual*, Second Edition, Cold Spring Harbor Laboratory Press (1989) (hereinafter referred to as *"Molecular Cloning,* Second Edition"), *Current Protocols in Molecular Biology,* John Wiley & Sons, 1987-1997 (hereinafter referred to as "Current Protocols in Molecular Biology")*DNA Cloning 1: Core Techniques, A Practical Approach,* Second Edition, Oxford University (1995) or the like. The hybridizable DNA includes a DNA having a homology of at least 50% or more, preferably a DNA having a homology of 70% or more, and more preferably a DNA having a homology of 90% or more, with the nucleotide sequence selected from the nucleotide sequences represented by SEQ ID NOs:11, 12, 13, 14, 15 and 16 and nucleotide sequences of nucleotides 49-117 in the nucleotide sequences represented by SEQ ID NOs:11, 12, 13, 14, 15 and 16.
The present invention is explained below in detail.

1. Method for producing the peptide of the present invention

The method for producing the peptide of the present invention includes a method by chemical synthesis, a genetically method, and the like

(1) Method for producing the peptide by chemical synthesis

For example, the physiologically active peptide can be obtained by synthesizing a peptide by the usual peptide synthesis method described in *Fundamentals and Experiments on Peptide Synthesis (Peptide no Kiso to Jikken)*, written by Nobuo Izumiya, Tetsuo Kato and Michinori Waki: Maruzen; *Experimental Chemical Course (Jikken Kagaku Koza),* 4th Edition, 22- Organic Synthesis (Yuki Gosei) IV, Acid-Amino Acid-Peptide, written by Saburo Aimoto, Shoichi Kusumoto, Kuniaki Tatsuta, Yoshihiro Hayakawa, Kagesaku Yamamoto and Tateaki Wakamiya: Maruzen; *International Journal of Peptide Protein Research,* 35, 161-214 (1990); *Solid-Phase Peptide Synthesis, Methods in Enzymology,* vol. 289, edited by Gregg B. Fields, Academic Press, (1997); *Peptide Synthesis Protocols, Methods in Enzymology,* vol. 35, edited by Michael W. Pennington and Ben M. Dunn, Humana Press (1994); and the like, and purifying the peptide. Specific synthesis method includes an azide method, an acid chloride method, an acid anhydride method, a mixed acid anhydride method, a DCC method, an active ester method, a carboimidazol method, an oxidation-reduction method and the like. Furthermore, any of a solid phase synthesis method and a liquid phase synthesis method can be applied. That is, an objective peptide is synthesized by condensing the amino acids constituting the peptide of the present invention and a remaining part and, if a product has a protective group, by eliminating the protective group.
Furthermore, when the side chain, the peptide-amino terminal and the peptide-carboxyl terminal of the amino acid residues constituting the physiologically active peptide of the present invention is chemically modified or protected, the peptide can be produced by the known method, such as chemical modification after the peptide synthesis, peptide synthesis using a chemically modified amino acid, appropriate selection of reaction conditions at final deprotection of the peptide synthesis, or the like *(Fundamentals and Experiments on Peptide Synthesis (Peptide no Kiso to Jikken)*, written by Nobuo Izumiya, Tetsuo Kato and Michinori Waki: Maruzen; *Sequel of Development of Medicament (Zoku Iyakuhin no Kaihatsu),* vol. 14, Peptide Synthesis, edited by Haruaki Yajima, Hirokawa Shoten; *Biocyemistry Experimental Course (Seikagaku Jikken Koza*)-1-Chemistry of Protein IV-Chemical Modification and Peptide Synthesis, edited by The Japanese Biochemical Society, Tokyo Kagaku Dojin; and *Chemical Modification of Protein (Tanpakushitsu no Kagaku Shushoku)*, the first and second volumes, Motonori Ohno, Yuichi Kaneoka, Fumio Sakiyama and Hiroshi Maeda.
Also, the peptide of the present invention can be synthesized by an automatic peptide synthesizer. The peptide can be synthesized by a peptide synthesizer using amino acids with appropriately protected side chains such as Nα-Fmoc-amino acid, Nα-Boc-amino acid and the like, on a commercially available peptide synthesizer, for example, a peptide synthesizer manufactured by Shimadzu Corporation, a peptide synthesizer manufactured by Advanced ChemTech Inc., USA (hereinafter referred to as "ACT") or the like, according to the synthesis program of each synthesizer. Protected amino acids and carrier resins used as the starting materials are available from ABI, Shimadzu Corporation, Kokusan Kagaku, Nova Biochem, Watanabe Kagaku, ACT, Ana Spec Inc., Peptide Institute and the like
The peptide of the present invention can be purified by the usual purification methods such as solvent extract, distillation, column chromatography, liquid chromatography, recrystallization and the like in combination.

(2)Method for producing the peptide according to genetically technique

The peptide of the present invention, for example, the peptide represented by formula (I) wherein $R^1$ is a hydrogen atom and $R^2$ is hydroxy can be produced by expressing the DNA of the present invention in a host cell, for example, by the following method, using a method described *in Molecular Cloning,* Second Edition; *Current Protocols* in *Molecular Biology* or the like.
Also, when the peptide of the present invention represented by formula (I) other than the peptide wherein $R^1$ is a hydrogen atom and $R^2$ is hydroxy can be produced by expressing the DNA of the present invention in a host cell, it can be obtained as it is. Also, the peptide of the present invention represented by formula (I) wherein $R^1$ is a hydrogen atom and $R^2$ is hydroxy can be produced by the following method, and then the side chain of the peptide is chemically or genetically modified.
A DNA fragment having an appropriate length containing a part encoding the peptide is prepared based on a full-length DNA, if necessary.
The DNA fragment or full-length DNA is inserted into the downstream of a promoter in an appropriate expression vector to thereby produce a recombinant vector.

The recombinant vector is introduced into a host cell suitable for the expression vector to thereby obtain a transformant which produces the peptide of the present invention.

Any bacteria, yeasts, animal cells, insect cells, plant cells, and the like can be used as the host cell, so long as it can express the desired gene.

The expression vector includes those which can replicate autonomously in the above host cell or can be integrated into a chromosome and contain a promoter at such a position that the DNA encoding the peptide of the present invention can be transcribed.

When a procaryote, such as a bacterium or the like, is used as the host cell, it is preferred that the used recombinant DNA containing the DNA encoding the peptide of the present invention can replicate autonomously in the procaryote, and at the same time contains a promoter, a ribosome-binding sequence, the DNA of the present invention and a transcription termination sequence. A gene regulating the promoter may also be contained.

The expression vector includes pBTrp2, pBTac1 and pBTac2 (all available from Boehringer Manheim), pKK223-3 (manufactured by Pharmacia), pSE280 (manufactured by Invitrogen), pGEMEX-1 (manufactured by Promega), pQE-8 (manufactured by QIAGEN), pKYP10 (Japanese Published . Unexamined Patent Application No. 110600/83), pKYP200 [*Agricultural Biological Chemistry,* 48, 669 (1984)], pLSA1 [*Agricultural Biological Chemistry,* 53, 277 (1989)], pGEL1 [*Proc. Natl. Acad. Sci. USA*, 82, 4306 (1985)], pBluescript II SK(-) (manufactured by Stratagene), pTrs30 [prepared from *Escherichia coli* JM109/pTrS30 (FERM BP-5407)], pTrs32 [prepared from *Escherichia coli* JM109/pTrS32 (FERM BP-5408)], pGHA2 [prepared from *Escherichia coli* IGHA2 (FERM B-400), Japanese Published Unexamined Patent Application No.221091/85], pGKA2 [prepared from *Escherichia coli* IGKA2 (FERM BP-6798), Japanese Published Unexamined Patent Application No.221091/85], pTerm2 (U.S. 4,686,191, U.S. 4,939,094, U.S. 5,160,735), pSupex, pUB110, pTP5, pC194, pEG400 [*J. Bacteriol*., 172, 2392 (1990)], pGEX (manufactured by Pharmacia), pET system (manufactured by Novagen), pSupex and the like.

Any promoter can be used, so long as it can function in the host cell. Examples include promoters derived from *Escherichia coli,* phage and the like, such as trp promoter ($P_{trp}$), *lac* promoter, $P_L$ promoter, $P_R$ promoter, T7 promoter and the like. Also, artificially designed and modified promoters, such as a promoter in which two $P_{trp}$ are linked in tandem ($P_{trp}\times2$), *tac* promoter, lacT7 promoter letI promoter and the like, can be used.

It is preferred to use a plasmid in which the space between Shine-Dalgarno sequence, which is the ribosome binding sequence, and the initiation codon is adjusted to an appropriate distance (for example, 6 to 18 bases).

The nucleotides in the nucleotide sequence of the DNA encoding the peptide of the present invention are substituted so as to have suitable codons for expression of the host to thereby improve the productivity of the desired peptide of the present invention.

A transcription termination sequence is not always necessary for expression of the DNA of the present invention. However, it is preferred to arrange the transcription terminating sequence just downstream of the structural gene.

The host cell includes microorganisms belonging to the genera *Escherichia, Serratia, Bacillus, Brevibacterium, Corynebacterium, Microbacterium, Pseudomonas* and the like. Examples include *Escherichia coli* XL1-Blue, *Escherichia coli* XL2-Blue, *Escherichia coli* DH1, *Escherichia coli* MC1000, *Escherichia coli* KY3276, *Escherichia coli* W1485, *Escherichia coli* JM109, *Escherichia coli* HB101, *Escherichia coli* No. 49, *Escherichia coli* W3110, *Escherichia coli* NY49, *Serratia ficaria, Serratia fonticola, Serratia liquefaciens, Serratia marcescens, Bacillus subtilis, Bacillus amyloliquefaciens, Brevibacterium immariophilum* ATCC 14068, *Brevibacterium saccharolyticum* ATCC 14066, *Brevibacterium flavum* ATCC 14067, *Brevibacterium lactofermentum* ATCC 13869, *Corynebacterium glutamicum* ATCC 13032, *Corynebacterium acetoacidophilum* ATCC 13870, *Microbacterium ammoniaphilum* ATCC 15354, *Pseudomonas* sp. D-0110 and the like.

As the method for introducing the recombinant DNA, any method for introducing the DNA into the above host cells can be used. Examples include a method using a calcium ion [*Proc. Natl. Acad. Sci. USA*, 69, 2110 (1972)], a protoplast method (Japanese Published Unexamined Patent Application No. 248394/88), methods described in *Gene*, 17, 107 (1982) and *Molecular & General Genetics,* 168, 111 (1979) and the like.

When yeast is used as the host cell, the expression vector includes YEp13 (ATCC 37115), YEp24 (ATCC 37051), YCp50 (ATCC 37419) and the like.

Any promoter can be used, so long as it can function in a yeast strain. Examples include a promoter of a gene in glycolytic pathway such as hexose kinase, etc., PHO5 promoter, PGK promoter, GAP promoter, ADH promoter, gal 1 promoter, gal 10 promoter, a heat shock polypeptide promoter, MF$\alpha$1 promoter, CUP 1 promoter and the like.

The host cell includes microorganisms belonging to the genera *Saccharomyces, Schizosaccharomyces, Kluyveromyces, Trichosporon, Schwanniomyces, Pichia, Candida* and the like. Examples include *Saccharomyces cerevisiae, Schizosaccharomyces pombe, Kluyveromyces lactis, Trichosporon pullulans, Schwanniomyces alluvius* and the like.

As the method for introducing the recombinant DNA, any method for introducing the DNA into yeast can be used. Examples include electroporation *[Methods. Enzymol.,* 194, 182 (1990)], the spheroplast method [*Proc. Natl.*

*Acad. Sci. USA,* 75, 1929 (1978)], the lithium acetate method (*J. Bacteriology*, 153, 163 (1983)], the method described in *Proc. Natl. Acad. Sci. USA,* 75, 7929 (1978) and the like..

When an animal cell is used as the host cell, the expression vector includes pcDNAI and pcDM8 (available from Funakoshi), pAGE107 [Japanese Published Unexamined Patent Application No. 22979/91, *Cytotechnology,* 3, 133 (1990)], pAS3-3 (Japanese Published Unexamined Patent Application No. 227075/90), pcCDM8 [*Nature,* 329, 840 (1987)], pcDNAI/Amp (manufactured by Invitrogen), pREP4 (manufactured by Invitrogen), pAGE103 [*J. Biochemistry,* 101, 1307 (1987)], pAGE210 and the like.

Any promoter can be used, so long as it can function in an animal cell. Examples include a promoter of IE (immediate early) gene of cytomegalovirus (CMV), an early promoter of SV40, a promoter of retrovirus, a metallothionein promoter, a heat shock promoter, SRα promoter and the like. Also, the enhancer of the IE gene of human CMV can be used together with the promoter

The host cell includes human Namalwa cell, monkey COS cell, Chinese hamster CHO cell, HBT5637 (Japanese Published Unexamined Patent Application No. 299/88) and the like.

As the method for introducing the recombinant DNA, any method can be used, so long as it is a method for introducing the DNA into an animal cell. Examples include electroporation [*Cytotechnology,* 3, 133 (1990)], a calcium phosphate method (Japanese Published Unexamined Patent Application No. 227075/90), lipofection [*Proc. Natl. Acad. Sci. USA*, 84, 7413 (1987)], a method described in *Virology,* 52, 456 (1973) and the like.

When an insect cell is used as the host cell, the peptide of the present invention can be expressed by a method described in, for example, *Current Protocols in Molecular Biology; Baculovirus Expression Vectors, A Laboratory Manual,* W.H. Freeman and Company, New York (1992); *Bio/Technology*, 6, 47 (1988) or the like.

A vector for introducing a recombinant gene and baculovirus are co-transfected into an insect cell to thereby obtain a recombinant virus in a supernatant in the culture of the insect cell, and then an insect cell is infected with the recombinant virus to thereby express the peptide of the present invention.

The vector for introducing a gene used in the method includes pVL1392, pVL1393 and pBlueBacIII (all manufactured by Invitrogen) and the like.

The baculovirus includes *Autographa californica* nuclear polyhedrosis virus which infects insects of the family Barathra and the like.

The insect cell includes *Spodoptera frugiperda* ovary cells Sf9 and Sf21 [*Baculovirus Expression Vectors, A Laboratory Manual,* W.H. Freeman and Company, New York (1992)], *Trichoplusia ni* ovary cell High 5 (manufactured by Invitrogen) and the like.

The method for co-transfecting the vector for transferring a recombinant DNA and the baculovirus for the preparation of the recombinant virus includes a calcium phosphate method (Japanese Published Unexamined Patent Application No. 227075/90), lipofection [*Proc. Natl. Acad. Sci. USA*, 84, 7413 (1987)] and the like.

When a plant cell is used as the host cell, the expression vector includes Ti plasmid, a tobacco mosaic virus vector and the like.

When a plant cell is used as the host cell, the expression vector includes Ti plasmid, a tobacco mosaic virus vector and the like.

Any promoter can be used, so long as it can be expressed in a plant cell. Examples include 35S promoter of cauliflower mosaic virus (CaMV), rice actin 1 promoter and the like.

Any promoter can be used, so long as it can be expressed in a plant cell. Examples include 35S promoter of cauliflower mosaic virus (CaMV), rice actin 1 promoter and the like.

The host cell includes plant cells such as tobacco, potato, tomato, carrot, soybean, rape, alfalfa, rice, wheat, barley, *etc.,* and the like.

As the method for introducing the recombinant DNA, any method can be used, so long as it is a method for introducing DNA into a plant cell. Examples include the *Agrobacterium* method (Japanese Published Unexamined Patent Application No. 140885/84, Japanese Published Unexamined Patent Application No. 70080/85, WO 94/00977), electroporation (Japanese Published Unexamined Patent Application No. 251887/85), the particle gun method (Japanese Patents 2606856 and 2517813) and the like.

As the method for expressing the gene, secretion production, fusion protein expression and the like can be carried out according to the method described in *Molecular Cloning,* Second Edition in addition to direct expression.

When the gene is expressed by using yeast, an animal cell, an insect cell or a plant cell, the peptide encoded by the DNA of the present invention to which a sugar or a sugar chain is added can be obtained.

The protein of the present invention can be produced by culturing the thus obtained transformant in a medium to thereby form and accumulate the peptide encoded by the DNA of the present invention in the culture, and recovering it from the culture. The method for culturing the transformant in a medium is carried out according to the usual method as used in culturing of the host

As a medium for culturing the transformant obtained by using, as the host, prokaryote such as *Escherichia coli* or the like or eukaryote such as yeast or the like, the medium may be either a natural medium or a synthetic medium, so long as it contains a carbon source, a nitrogen source, inorganic salts and the like which can be assimilated by the organism and the transformant can be cultured efficiently.

Any carbon source can be used, so long as the organism can be assimilated. Examples include carbohydrates such as glucose, fructose, sucrose, molasses containing them, starch, starch hydrolysate, *etc.,* organic acids such as acetic acid, propionic acid, *etc.,* alcohols such as ethanol, propanol, *etc.*, and the like.

The nitrogen source includes ammonia, various ammonium salts of inorganic acids or organic acids such as ammonium chloride, ammonium sulfate, ammonium acetate, ammonium phosphate, etc., other nitrogen-containing compounds, peptone, meat extract, yeast extract, corn steep liquor, casein hydrolysate, soybean meal and soybean meal hydrolysate, various fermented cells and hydrolysates thereof, and the like.

The inorganic salts include potassium dihydrogen phosphate, dipotassium hydrogen phosphate, magnesium phosphate, magnesium sulfate, sodium chloride, ferrous sulfate, manganese sulfate, copper sulfate, calcium carbonate and the like.

Culturing is usually carried out under aerobic conditions by shaking culture, submerged spinner culture under aeration or the like. The culturing temperature is preferably from 15 to 40°C, and the culturing time is generally from 16 hours to 7 days. The pH is preferably maintained at 3.0 to 9.0 during culturing. The pH can be adjusted by using an inorganic or organic acid, an alkali solution, urea, calcium carbonate, ammonia or the like.

Also, antibiotics such as ampicillin, tetracycline and the like can be added to the medium during culturing, if necessary.

When a microorganism transformed with a recombinant vector using an inducible promoter as a promoter is cultured, an inducer can be added to the medium, if necessary. For example, isopropyl-β-D-thiogalactopyranoside (IPTG) or the like can be added to the medium when a microorganism transformed with a recombinant vector using *lac* promoter is cultured, or indoleacrylic acid or the like can by added thereto when a microorganism transformed with a recombinant vector using *trp* promoter is cultured.

The medium for culturing a transformant obtained using an animal cell as the host includes generally used RPMI 1640 medium [*The Journal of the American Medical Association,* 199, 519 (1967)], Eagle's MEM [*Science,* 122, 501 (1952)], modified Dulbecco's MEM [*Virology,* 8, 396 (1959)], 199 Medium [*Proceeding of the Society for the Biological Medicine,* 73, 1 (1950)], and other media to which fetal calf serum or the like has been added to the above media and the like.

Culturing is generally carried out at pH 6 to 8 and at 30 to 40°C in the presence of 5% $CO_2$ for 1 to 7 days.

Furthermore, antibiotics such as kanamycin, penicillin and the like can be added to the medium during culturing, if necessary.

The medium for culturing a transformant obtained using an insect cell as the host includes generally used TNM-FH medium (manufactured by Pharmingen), Sf-900 II SFM (manufactured by Life Technologies), ExCell 400 and ExCell 405 (both manufactured by JRH Biosciences), Grace's Insect Medium [Grace, T.C.C., *Nature,* 195, 788 (1962)] and the like.

Culturing is generally carried out at pH 6 to 7 and at 25 to 30°C for 1 to 5 days.

Furthermore, antibiotics such as gentamicin and the like may be added to the medium during culturing, if necessary.

A transformant obtained by using a plant cell as the host can be used as the cell or after differentiating to a plant cell or organ. The medium used in culturing of the transformant includes Murashige and Skoog (MS) medium, White medium, media to which a plant hormone such as auxin, cytokinine or the like has been added, and the like.

A transformant obtained by using a plant cell as the host can be used as the cell or after differentiating to a plant cell or organ. The medium used in culturing of the transformant includes Murashige and Skoog (MS) medium, White medium, media to which a plant hormone such as auxin, cytokinine or the like has been added, and the like.

Culturing is carried out generally at a pH 5 to 9 and at 20 to 40°C for 3 to 60 days.

Furthermore, antibiotics such as kanamycin, hygromycin and the like can be added to the medium during culturing, if necessary.

As described above, the peptide of the present invention can be produced by culturing a transformant derived from a microorganism, an animal cell or a plant cell containing a recombinant DNA to which a DNA encoding the peptide has been inserted, according to the usual culturing method to form and accumulate the peptide, and recovering the peptide from the culture.

The process for producing the peptide of the present invention includes a method of intracellular production in a host cell, a method of extracellular secretion from a host cell, or a method of production on an outer membrane of the host cell. The method can be selected by changing the used host cell or the structure of the produced peptide encoded by the DNA of the present invention.

When the peptide of the present invention is produced in a host cell or on an outer membrane of the host cell, the peptide can be secreted extracellularly according to the method of Paulson *et al.* [*J. Biol. Chem.*, 264, 17619 (1989)], the method of Lowe *et al.* [*Proc. Natl. Acad. Sci.*, *USA*, 86, 8227 (1989); *Genes Develop*., 4, 1288 (1990)], or methods described in Japanese Published Unexamined Patent Application Nos. 336963/93, 823021/94, and the like.

That is, the peptide of the present invention can be secreted extracellularly by expressing it in the form that a signal peptide has been added just before the active site of the peptide in the peptide encoded by the DNA of the present invention according to the recombinant DNA technique.

Furthermore, the amount of production can be increased using a gene amplification system, such as a dihydrofolate reductase gene or the like according to the method described in Japanese Published Unexamined Patent Application No. 227075/90.

Moreover, the peptide of the present invention can be produced by redifferentiating animal or plant cells to which the gene has been introduced to prepare a gene-introduced animal individual (transgenic non-human animal) or plant individual (transgenic plant) and using the individuals.

When the transformant is the animal individual or plant individual, the peptide can be produced by breeding or cultivating it so as to form and accumulate the peptide, and recovering the peptide from the animal individual or plant individual.

The process for producing the peptide of the present invention using the animal individual includes a process for producing the protein in an animal developed by introducing a gene according to known methods [*American Journal of Clinical Nutrition,* 63, 639S (1996), *American Journal of Clinical Nutrition,* 63, 627S (1996), *Bio/Technology*, 9, 830 (1991)].

In the animal individual, for example, the peptide of the present invention can be produced by breeding a transgenic non-human animal to which the DNA of the present invention has been introduced to form and accumulate the peptide encoded by the DNA in the animal, and recovering the peptide from the animal. The peptide thus produced can be accumulated in milk (Japanese Published Unexamined . Patent Application No. 309192/88), egg and the like of the animal. Any promoter can be used, so long as it can be expressed in the animal. Suitable examples include an α-casein promoter, a β-casein promoter, a β-lactoglobulin promoter, a whey acidic protein promoter, and the like, which are specific for mammary glandular cells.

The method for producing the peptide of the present invention using the plant individual includes a method for producing the peptide of the present invention by cultivating a transgenic plant to which the DNA the present invention is introduced, by a known method *[Tissue Culture (Soshiki Baiyo)*, *20* (1994), *Tissue Culture (Soshiki Baiyo)*, *21* (1994), *Trends Biotechnol*., 15, 45 (1997)] to form and accumulate the peptide encoded by the DNA in the plant, and recovering the peptide from the plant.

When the peptide produced by the transformant of the present invention is expressed as a soluble product in cells, the cells are collected by centrifugation after culturing, suspended in an aqueous buffer, and disrupted using an ultrasonicator, a French press, a Manton Gaulin homogenizer, a Dynomill, or the like to obtain a cell-free extract. From the supernatant obtained by centrifuging the cell-free extract, a purified product can be obtained by the usual method used for isolating and purifying an enzyme, for example, solvent extraction, salting-out using ammonium sulfate or the like, desalting, precipitation using an organic solvent, anion exchange chromatography using a resin, such as diethylaminoethyl (DEAE)-Sepharose, DIAION HPA-75 (manufactured by Mitsubishi Chemical) or the like, cation exchange chromatography using a resin, such as S-Sepharose FF (manufactured by Pharmacia) or the like, hydrophobic chromatography using a resin, such as butyl sepharose, phenyl sepharose or the like, gel filtration using a molecular sieve, affinity chromatography, chromatofocusing, or electrophoresis such as isoelectronic focusing or the like, alone or in combination thereof.

When the peptide is expressed as an inclusion body in the host cells, the cells are collected in the same manner, disrupted and centrifuged to recover the peptide as the precipitate fraction. Next, the inclusion body of the peptide is solubilized with a protein-denaturing agent. The solubilized solution is diluted or dialyzed to reconstitute the normal tertiary structure of the peptide, which is subjected to the purification and isolation method similar to the above to obtain the purified product of the peptide.

When the peptide of the present invention or derivatives thereof are extracellularly secreted, the peptide or the derivatives can be collected in the culture supernatant. Specifically, the culture supernatant is obtained by treating the culture in a treatment similar to the above, such as centrifugation or the like, and a purified product can be obtained from the supernatant using a purification and isolation method similar to the above.

Examples of the peptide obtained by the above method include peptides wherein A comprises the amino acid sequence represented by SEQ ID NO:1, 2, 3, 4, 5 or 6 or amino acid positions 17-39 in SEQ ID NO:1, 2, 3, 4, 5 or 6.

Furthermore, the peptide of the present invention can be produced in the form of salts according to the conditions in the above production method. The salts of the peptide of the present invention are preferably pharmaceutically acceptable acid addition salts, metal salts, and organic base addition salts. The acid addition salts include inorganic acid salts such as hydrochloride, sulfate, phosphate and the like, and organic acid salts such as acetate, maleate, fumarate, tartarate, citrate and the like. The metal salts include alkali metal salts such as sodium salts, potassium salts, *etc.*; alkaline earth metal salts such as magnesium salts, calcium salts, etc.; aluminum salts, zinc salts and the like. The organic base addition salts include salts formed with a primary amine such as methylamine, ethylamine, aniline,

etc., a secondary amine such as dimethylamine, diethylamine, pyrrolidine, piperidine, morpholine, piperazine, *etc.*; or a tertiary amine such as trimethylamine, triethylamine, N,N-dimethylaniline, pyridine, etc.; ammonium salts; and the like.

The salts can be prepared using an appropriate acid such as hydrochloric acid or the like or an appropriate base such as sodium hydroxide. For example, the salts can be prepared by a treatment according to the standard protocol in water or liquid containing an inactive water-miscible organic solvent such as methanol, ethanol or dioxane. Also, the treatment temperature is 0 to 100°C, preferable room temperature. Moreover, the biochemical and physicochemical properties of the peptide of the present invention can be analyzed by mass spectrometry, nuclear magnetic resonance, electrophoresis, high performance liquid chromatography or the like.

2. Preparation of DNA of the present invention

The DNA of the present invention can be prepared from human or non-human animal tissue or cell, for example, by the following method using the method described in *Molecular Cloning,* Second Edition, *Current Protocols in Molecular Biology* or the like.

The tissue or cells of human or a non-human animal includes the bone marrow, the spleen, macrophages, neutrophils, smooth muscle cells and the like.

A total RNA or mRNA is prepared from the human or non-human animal tissue or cell.

cDNA libary is produced from the prepared total RNA or mRNA.

Based on the amino acid sequence of the peptide of the present invention, degenerative primers are produced and a gene fragment encoding the peptide of the present invention is obtained by PCR using the produced cDNA library as the template.

The cDNA libary is screened by using the obtained gene fragment as a probe to thereby obtain the DNA of the present invention encoding the peptide of the present invention.

The mRNA of the human or non-human animal tissue or cell can be one on the market or be prepared from the human or non-human animal tissue or cell as follows. The method for preparing a total RNA from the human or non-human animal tissue or cell includes the guanidine thiocyanatecesium trifluoroacetate method *[Methods in Enzymology,* 154, 3 (1987)], the acidic guanidine thiocyante-phenol-chloroform (AGPC) method [*Analytical Biochemistry*, 162, 156 (1987), *Experimetal Medicine (Jikken Igaku),* 9, 1937 (1991)] and the like.

The method for preparing mRNA from a total RNA includes the oligo (dT) immobilized cellulose column method (*Molecular Cloning,* Second Edition) and the like.

Also, mRNA can be prepared by a kit such as Fast Track mRNA Isolation Kit (manufactured by Invitrogen), Quick Prep mRNA Purification Kit (manufactured by Pharmacia) or the like.

A cDNA library is prepared using the prepared tissue or cell mRNA of human or a non-human animal. The method for preparing the cDNA library include the methods described in *Molecular Cloning,* Second Edition, *Current Protocols in Molecular Biology, A Laboratory Manual*, 2nd Ed. (1989), a method using a commercially available kit such as Superscript Plasmid System for cDNA Synthesis and Plasmid Cloning (manufactured by Life Technologies) or ZAP-cDNA Synthesis Kit (manufactured by STRATAGENE), and the like.

As the cloning vector for preparing the cDNA library, any of a phage vector, plasmid vector and the like so long as it can replicate autonomously in *Escherichia coli* K12 strain. Examples include ZAP Express [manufactured by STRATAGENE, *Strategies,* 5, 58 (1992)], pBluescript II SK(+) *[Nucleic Acids Research,* 17, 9494 (1989)], Lambda zapII (manufactured by STRATAGENE), λgt10 and λgt11 [*DNA Cloning: A Practical Approach,* I, 49 (1985)], λTriplEx (manufactured by Clontech), λExCell (manufactured by Pharmacia), pT7T318U (manufactured by Pharmacia), pcD2 [*Mol. Cell. Biol*., 3, 280 (1983)], pUC18 *[Gene,* 33, 103 (1985)] and the like.

Any host microorganism can be used, so long as it belongs to *Escherichia coli.* Examples include *Escherichia coli* XL1-Blue MRF' [manufactured by STRAGAGENE, *Strategies,* 5, 81 (1992)], *Escherichia coli* C600 [*Genetics,* 39, 440 (1954)], *Escherichia coli* Y1088 *[Science,* 222, 778 (1983)], *Escherichia coli* Y1090 *[Science,* 222, 778 (1983)], *Escherichia coli* NM522 [*J. Mol. Biol*., 166, 1 (1983)], *Escherichia coli* K802 *[J. Mol. Blol*., 16, 118 (1966)], *Escherichia coli* JM105 [*Gene,* 38, 275 (1985)] and the like.

The cDNA library can be used as such in the succeeding analysis, and in order to obtain a full length cDNA as efficient as possible by decreasing the ratio of an infull length cDNA, a cDNA library prepared using the oligo cap method *[Gene,* 138, 171 (1994); *Gene,* 200, 149 (1997); *Protein, Nucleic Acid, Protein (Tanpakushitsu, Kakusan, Koso),* 41, 603 (1996); *Experimental Medicine (Jikken Igaku),* 11, 2491 (1993); *cDNA Cloning,* Yodo-sha (1996); *Methods for Preparing Gene Libraries (Idenshi Libarry no Sakuseiho),* Yodo-sha (1994)] can be used in the following analysis.

Based on the amino acid sequence of the peptide of the present invention, degenerative primers specific for the 5'-terminal and 3'-terminal nucleotide sequences of a nucleotide sequence presumed to encode the amino acid sequence are prepared, and DNA is amplified by PCR [*PCR Protocols*, Academic Press (1990)] using the produced cDNA library as the template to thereby obtain a gene fragment encoding the peptide of the present invention.

It can be confirmed that the obtained gene fragment is a DNA encoding the peptide of the present invention by a method usually used for analyzing a nucleotide, such as the dideoxy method of Sanger *et al.* [*Proc. Natl. Acad. Sci. USA,* 74, 5463 (1977)], a nucleotide sequence analyzer such as ABIPRISM 377 DNA Sequencer (manufactured by PE Biosystems) or the like.

The DNA of the present invention can be obtained by carrying out colony hybridization or plaque hybridization *(Molecular Cloning,* Second Edition) for the cDNA or cDMA library synthesized from the mRNA contained in the human or non-human animal tissue or cell, using the gene fragment as a DNA probe.

Furthermore, the DNA of the present invention can also be obtained by carrying out screening by PCR using the cDNA or cDNA library synthesized from the mRNA contained in the human or non-human animal tissue or cell as the template and using the primers used for obtaining the gene fragment encoding the peptide of the present invention.

The nucleotide sequence of the DNA of the present invention is analyzed from its terminus and determined by a method usually used for analyzing a nucleotide, such as the dideoxy method of Sanger *et al.* [*Proc. Natl. Acad. Sci. USA,* 74, 5463 (1977)], a nucleotide sequence analyzer such as ABIPRISM 377 DNA Sequencer (manufactured by PE Biosystems) or the like.

A gene encoding the peptide of the present invention can also be determined from genes in databases by searching nucleotide sequence databases such as GenBank, EMBL, DDBJ and the like using a homology retrieving program such as BLAST based or the like on the determined cDNA nucleotide sequence.

The DNA of the present invention can also be obtained by chemically synthesizing it with a DNA synthesizer such as DNA Synthesizer model 392 manufactured by Perkin Elmer or the like using the phosphoamidite method, based on the determined DNA nucleotide sequence.

3. Production of antibody which recognizes the peptide of the present invention

Antibodies which recognize the peptide of the present invention such as a polyclonal antibody, a monoclonal antibody and the like can be produced by using, as an antigen, a purified product of the peptide of the present invention or a partial amino acid sequence of the peptide of the present invention, and immunizing it to an animal.

As the animal immunized, a rabbit, a goat, a 3- to 20-weeks-old rat, a mouse, a hamster and the like can be used.

As the antigen for producing the antibody of the present invention, any of the peptide of the present invention and a partial peptide thereof as described above can be used. The antigen is preferably a peptide comprising the amino acid sequence represented by SEQ ID NO: 1, 2, 3, 4, 5 or 6 wherein a cystein residue is added to the N-terminal and the C-terminal is amidated. For example, as a peptide comprising the amino acid sequence represented by SEQ ID NO:1 wherein the C-terminal is aminated, a peptide comprising the amino acid sequence represented by SEQ ID NO: 26 is exemplified. Also, as a peptide comprising the amino acid sequence represented by SEQ ID NO:5 wherein the C-terminal is aminated, a peptide comprising the amino acid sequence represented by SEQ ID NO:23 is exemplified. Furthermore, as a peptide comprising amino acids of positions 1-15 in the amino acid sequence represented by SEQ ID NO:5 wherein the C-terminal is aminated, a peptide comprising the amino acid sequence represented by SEQ ID NO:24 is exemplified, and as a peptide comprising amino acids of positions 17-39 wherein the C-terminal is aminated, a peptide comprising the amino acid sequence represented by SEQ ID NO:25.

A dose of the antigen is preferably 50 to 100 μg per animal.

The antigen is preferably a peptide to which a carrier protein such as keyhole limpet hemocyanin, bovine thyroglobulin or the like is covalently bound. The peptide used as the antigen can be synthesized by a peptide synthesizer.

The antigen may be administered every 1 to 2 weeks for 3 to 10 times after the first administration. A blood sample is collected from the veinvenous plexus of the ocular fundus 3 to 7 days after each administration and tested, for example, by an enzyme immunoassay *[Enzyme-linked Immunosorbent Assay (ELISA),* published by Igaku Shoin (1976), *Antibodies - A Laboratory Manual,* Cold Spring Harbor Laboratory (1988)] or the like on the reactivity of the serum with the antigen used for the immunization.

A serum is obtained from a non-human mammal animal showing a sufficient antibody titer in its serum against the antigen used for the immunization, and a polyclonal antibody can be obtained by separating and purifying the serum.

The method for the separation and purification includes centrifugation; salting out by 40-50% saturated ammonium sulfate; caprylic acid precipitation *[Antibodies, A Laboratory manual,* Cold Spring Harbor Laboratory (1988)], chromatography using a DEAE-sepharose column, an anion exchange column, a protein A- or G-column, a gel filtration column, *etc.*; and the like, which may be carried out alone or in combination.

(2) Production of monoclonal antibody

(a) Production of antibody-producing cell

A mouse or rat in which its serum showed a sufficient antibody titer for the peptide of the present invention used in the immunization is submitted for use as a supply source of the antibody-producing cell.

Three to seven days after the final administration of the cell used as the antigen into the mouse or rat which showed the antibody titer, the spleen is excised.

The spleen is cut to pieces in MEM medium (manufactured by Nissui Pharmaceutical), the cells are unbound using a pair of forceps and centrifuged at 1,200 rpm for 5 minutes, and then the supernatant is discarded.

Splenocytes in the thus obtained precipitation fraction are treated with Tris-ammonium chloride buffer (pH 7.65) for 1 to 2 minutes to eliminate erythrocytes and then washed three times with MEM medium, and the resulting splenocytes are used as the antibody-producing cells.

(b) Production of myeloma cells

Cells of a cell line obtained from the non-human animal are used as the myeloma cells. Examples include 8-azaguanine-resistant mouse (BALB/c-derived) myeloma cell lines P3-X63Ag8-U1 (hereinafter referred to as "P3-U1") [*Curr. Topics. Microbiol. Immunol.*, 81, 1 (1978); *Europ. J. Immunol.*, 6, 511 (1976)], SP2/0-Ag14 (SP-2) [*Nature,* 276, 269 (1978)], P3-X63-Ag8653 (653) [*J. Immunol.*, 123, 1548 (1979)], P3-X63-Ag8 (X63) [*Nature,* 256, 495 (1975)] and the like. These cell lines are subcultured in 8-azaguanine medium [produced by supplementing RPMI-1640 medium with glutamine (1.5 mM), 2-mercaptoethanol ($5\times10^{-5}$ M), gentamicin (10 μg/ml) and fetal calf serum (FCS) (manufactured by CSL; 10%) (hereinafter referred to as "normal medium") and further supplementing the resulting medium with 8-azaguanine (15 μg/ml)] and cultured for 3 to 4 days before cell fusion in the normal medium, and $2\times10^{7}$ or more of the cells are used in the fusion.

(c) Production of hybridoma

The antibody-producing cells obtained in (a) and the myeloma cells obtained in (b) are washed well with MEM or PBS (1.83 g of disodium phosphate, 0.21 g of monopotassium phosphate and 7.65 g of sodium chloride per liter of distilled water, pH 7.2), and mixed in a cell number proportion of antibody-producing cells : myeloma cell = 5 to 10 : 1, the mixture is centrifuged at 1,200 rpm for 5 minutes and then the supernatant is discarded.

The cells of the resulting precipitation fraction are thoroughly disintegrated, 0.2 to 1 ml of a mixture solution containing 2 g of polyethylene glycol-1000 (PEG-1000), 2 ml of MEM and 0.7 ml of dimethyl sulfoxide (DMSO) is added to the cells per $10^8$ antibody-producing cells under stirring at 37°C, at then 1 to 2 ml of MEM is added several times at 1- to 2-minute intervals.

After the addition, the total volume is adjusted to 50 ml by adding MEM. After centrifugation of the thus produced solution at 900 rpm for 5 minutes, the supernatant is discarded. The cells of the resulting precipitation fraction are loosened gently and then suspended in 100 ml of HAT medium [produced by supplementing the normal medium with hypoxanthine ($10^{-4}$ M), thymidine ($1.5\times10^{-5}$ M) and aminopterin ($4\times10^{-7}$ M)] by repeated drawing up and discharging using a measuring pipette.

The suspension is dispensed in 100 μl into each well of a 96-well incubation plates and incubated in a 5% $CO_2$ incubator at 37°C for 7 to 14 days.

After the incubation, a part of the culture supernatant is taken from each well, and a hybridoma which specifically reacts with the peptide of the present invention is selected by the enzyme immunoassay described in, for example, *Antibodies, A Laboratory Manual,* Cold Spring Harbor Laboratory, Chapter 14 (1988) or the like.

Examples of the enzyme immunoassay are described below.

The peptide of the present invention or a part fragment of the peptide used as the antigen in the immunization is coated onto an appropriate plate, and is allowed to react with a hybridoma culture supernatant or the purified antibody obtained in (d) described below, as a first antibody, and then with an anti-rat or anti-mouse immunoglobulin antibody as a second antibody labeled with biotin, an enzyme, a chemiluminescent substance, a radioactive compound or the like, and then a reaction suitable for the label is carried out to select one which specifically reacts with the peptide of the present invention as a hybridoma which produces the monoclonal antibody of the present invention.

Using the hybridoma, cloning is repeated twice by limiting dilution [using HT medium (HAT medium minus aminopterin) for the first cloning and the normal medium for the second cloning], and a line for which a high antibody titer is constantly observed is selected as a hybridoma which produces the monoclonal antibody of the present invention.

The antibody-producing cells obtained in (a) and the myeloma cells obtained in (b) are washed well with MEM medium or PBS (1.83 g of disodium phosphate, 0.21 g of monopotassium phosphate and 7.65 g of sodium chloride per liter of distilled water, pH 7.2), and mixed in a cell number proportion of antibody-producing cells : myeloma cell = 5 to 10 : 1, the mixture is centrifuged at 1,200 rpm for 5 minutes and then the supernatant is discarded.

The spleen is finely cut and broken up into pieces with tweezers in MEM medium (manufactured by Nissui Pharmaceutical) and is centrifuged at 1,200 rpm for 5 minutes, and the supernatant is discarded.

The spleen cells in the resulting precipitate are treated with a tris-ammonium chloride buffer (pH 7.65) for 1 to 2 minutes to remove blood erythrocytes and then washed with MEM medium three times, and the resulting spleen cells are used as antibody-producing cells.

(d) Production of monoclonal antibody

The hybridoma cells producing the monoclonal antibody which recognizes the peptide of the present invention obtained in (c) are administered by intraperitoneal injection into 8- to 10-weeks-old mice or nude mice treated with pristane [0.5 ml of 2,6,10,14-tetramethylpentadecane (pristane) is intraperitoneally administered, followed by feeding for 2 weeks] at a dose of 5 to $20 \times 10^6$ cells/animal. The hybridoma causes ascites tumor in 10 to 21 days.

The ascitic fluid is collected from the mice and centrifuged at 3,000 rpm for 5 minutes to remove the solid matter.

The monoclonal antibody can be purified and obtained from the resulting supernatant in the same manner as the method for obtaining the polyclonal antibody.

The subclass of the purified monoclonal antibody can be determined using a mouse monoclonal antibody typing kit or a rat monoclonal antibody typing kit. The amount of the peptide can be determined by the Lowry method or by absorbance at 280 nm.

The antibody obtained by using a peptide comprising the amino acid sequence represented by SEQ ID NO: 23 as the antigen specifically recognizes the amino acid sequence represented by SEQ ID NO:5 and neutralizes activity of a peptide comprising the amino acid sequence represented by SEQ ID NO:5 wherein the C-terminal side is amidated, e.g., a peptide comprising the amino acid sequence represented by SEQ ID NO:38. Examples include monoclonal antibodies KM2947 and KM2946 produced by hybridomas KM2947 and KM2946, respectively.

The antibody obtained by using a peptide comprising the amino acid sequence represented by SEQ ID NO: 26 as the antigen specifically recognizes the amino acid sequence represented by SEQ ID NO:1 and neutralizes activity of a peptide comprising the amino acid sequence represented by SEQ ID NO:1 in which the C-terminal side is amidated, e.g., a peptide comprising the amino acid sequence represented by SEQ ID NO:36. Examples include monoclonal antibodies KM3030 and KM3032 produced by hybridomas KM3030 and KM3032, respectively.

The antibody obtained by using a peptide comprising the amino acid sequence represented by SEQ ID NO: 24 as the antigen recognizes both of SEQ ID NO:1 and SEQ ID NO:5. Examples include a monoclonal antibody KM2952 produced by a hybridoma KM2952.

The antibody obtained by using a peptide comprising the amino acid sequence represented by SEQ ID NO: 25 as the antigen specifically recognizes the amino acid sequence represented by SEQ ID NO:1. Examples include a monoclonal antibody KM3022 produced by a hybridoma KM3022.

Herein, the hybridomas KM2947, KM2952, KM3022 and KM3030 have been deposited on Jury 12, 2001, in International Patent Organism Depositary, National Instituted of Advanced Industrial Science and Technology (AIST Tsukuba Central 6, 1-1, Higashi 1-Chome Tsukuba-shi, Ibaraki-ken 305-8566 Japan) as FERM BP-7656, FERM BP-7657, FERM BP-7658 and FERM BP-7659, respectively.

The monoclonal antibodies obtained in the above can be used in diagnosis, treatment and prevention depending on the properties of the antibodies. That is, a monoclonal antibody having a neutralizing activity can be used as an agent for preventing and treating diseases to which the peptide of the present invention relates described below. Also, degrees of the diseases can be diagnosed by detecting the peptide of the present invention through a sandwich ELISA or the like using monoclonal antibodies having different reactivity.

4. Production method of the oligonucleotide of the present invention

Based on the information of the nucleotide sequence of the DNA obtained in the above 2, an oligonucleotide having a sequence corresponding to continuous 5 to 60 nucleotides, preferably 10 to 40 nucleotides, in the nucleotide sequence of the DNA of the present invention, e.g., the nucleotide sequence represented by SEQ ID NO:11, 12, 13, 14, 15 or 16, or an oligonucleotide corresponding to a sequence complementary to the oligonucleotide (hereinafter referred to as "antisense oligonucleotide") can be produced using a conventional method or a DNA synthesizer.

The oligonucleotide of the present invention includes oligonucleotides such as oligo DNA, oligo RNA and the like, derivatives of the oligonucleotides (hereinafter referred to as "oligonucleotide derivatives") and the like.

The oligonucleotide or antisense oligonucleotide includes a sense primer corresponding to a nucleotide sequence at the 5'-terminal side and an antisense primer corresponding to a nucleotide sequence at the 3'-terminal side and the like in a partial nucleotide sequence of a mRNA to be detected. In this case, uracil in mRNA corresponds to thymidine in the oligonucleotide primers.

As the sense primer and antisense primer, oligonucleotides in which melting points (Tm) and the number of nucleotides of both are not extremely varied and which have a nucleotide number of 5 to 60 nucleotides, preferably from 10 to 50 nucleotides, are exemplified.

The oligonucleotide derivatives include oligonucleotide derivatives in which a phosphodiester bond in the oligonucleotide is converted into a phosphorothioate bond, oligonucleotide derivatives in which a phosphodiester bond in the oligonucleotide is converted into an N3'-P5' phosphoamidate bond; oligonucleotide derivatives in which a ribose and phosphodiester bond in the oligonucleotide is converted into a peptide-nucleic acid bond, oligonucleotide derivatives in which uracil in the oligonucleotide is substituted with C-5 propynyluracil, oligonucleotide derivatives in which uracil in the oligonucleotide is substituted with C-5 thiazoleuracil, oligonucleotide derivatives in which cytosine in the oligonucleotide is substituted with C-5 propynylcytosine, oligonucleotide derivatives in which cytosine in the oligonucleotide is substituted with phenoxazine-modified cytosine, oligonucleotide derivatives in which ribose in the oligonucleotide is substituted with 2'-O-propylribose, oligonucleotide derivatives in which ribose in the oligonucleotide is substituted with 2'-methoxyethoxyribose, and the like [*Cell Engineering*, 16, 1463 (1997)].

5. Measuring method of intracellular calcium ion concentration

It can be confirmed that the peptide of the present invention or a salt thereof is a physiologically active peptide having an activity to change a concentration of an intracellular calcium ion, by adding the peptide of the present invention or a pharmaceutically acceptable salt thereof to a system in which an organ derived from the living body or cells constituting an organ are cultured, and measuring changes in an intracellular signal transduction, e.g., changes in the concentration of an intracellular calcium ion, in the cell under culturing.

The organ derived from the living body or the organ-constituting cell include a bone, organs such as the spleen and the like, cells containing the organs and the like.

The method for measuring the concentration of an intracellular calcium ion includes 1) a method using a calcium indicator such as fura-2, indo-1 or the like [R.Y. Tsien, *Nature,* 290, 527 (1981); R.Y. Tsien *et al., J. Cell. Biol*., 94, 325 (1982)], 2) a method using a calcium divalent ion-sensitive microelectrode [C.C. Ashley and A.K. Campbell, Eds., Detection and Measurement of Free $Ca^{2+}$ in Cells, Elsevier, North-Holland, Amsterdam (1979)], 3) a method using fluorescence resonance energy transfer (FRET), 4) a method using a calcium ion-sensitive photoprotein, aequorin [E.B. Ridgway *et al*.; Biochem. Biophys. Commun., 29, 229 (1967)] and the like.

6. Use of peptide, DNA, oligonucleotide or antibody of the present invention

(1) Method for detecting expression of a gene encoding the peptide of the present invention using the DNA or oligonucleotide of the present invention

Expression of a mRNA encoding the peptide of the present invention can be detected using the DNA or oligonucleotide of the present invention by carrying out Northern hybridization (*Molecular Cloning,* Second Edition), PCR or RT (reverse-transcribed)-PCR [both in *PCR Protocols,* Academic Press (1990)] (both of them will also be called "PCR") or the like.

(2) Method for detecting mutation of a gene encoding the peptide of the present invention using the DNA or oligonucleotide of the present invention

Mutation of a gene encoding the peptide of the present invention can be detected by carrying out Southern hybridization (*Molecular Cloning,* Second Edition), PCR or the like using the oligonucleotide of the present invention as the probe.

(3)Method for immunologically detecting the peptide of the present invention using the antibody of the present invention

The peptide of the present invention or a tissue containing the peptide can be immunologically detected using the antibody of the present invention by carrying out an antigen-antibody reaction. The detection method can be used for the diagnosis of diseases caused by a mutation or a change in the expression of a gene encoding the peptide of the present invention, such as diseases which accompany infection and inflammation, diseases which accompany abnormal differentiation proliferation of smooth muscle cells, diseases which accompany abnormal activation of fibroblasts, diseases which accompany abnormal activation of a synovial tissue, diseases which accompany disorder of pancreatic β cells, diseases which accompanies abnormality of osteoblasts or osteoclasts, diseases which accompany abnormal activation of immunocytes, diseases which accompany disorder of a blood vessel, diseases of an eye based on angiogenesis, diseases which accompany neopla, diseases in which linkage relation to gene regions of a major histocompatibility antigen is confirmed, and the like. In addition, the detection method can also be used for the determination of the peptide.

The immunological detection method includes ELISA using a microtiter plate, an immunofluorescent method, Western blotting, a tissue immunostaining and the like.

The immunological determination method includes sandwich ELISA in which two monoclonal antibodies having different epitopes which react with the peptide of the present invention in a liquid phase, radioimmunoassay using the peptide of the present invention labeled with a radioisotope such as $^{125}I$ or the like and an antibody which recognizes the peptide of the present invention, and the like.

(4)Method for inhibiting transcription or translation of a gene encoding the peptide of the present invention using the DNA or oligonucleotide of the present invention

The DNA of the present invention can inhibit transcription or translation of a gene encoding the peptide of the present invention using an antisense RNA/DNA technique [*Bioscience and Industry,* 50, 322 (1992), *Chemistry (Kagaku)*, 46, 681 (1991), *Biotechnology,* 9, 358 (1992), *Trends in Biotechnology,* 10, 87 (1992), *Trends in Biotechnology,* 10, 152 (1992), *Cell Engineering (Saibo Kogaku)*, 16, 1463 (1997)], a triple helix technique *[Trends in Biotechnology,* 10, 132 (1992)], a ribozyme technique *[Current Opinion in Chemical Biology*, 3, 274 (1999), *FEMS Microbiology Reviews,* 23, 257 (1999), *Frontiers in Bioscience,* 4, D497 (1999), *Chemistry & Biology,* 6, R33 (1999), *Nucleic Acids Research,* 26, 5237 (1998), *Trends in Biotechnology,* 16, 438 (1998)], a decoy DNA method [*Nippon Rinsho - Japanese Journal of Clinical Medicine,* 56, 563 (1998), *Circulation Research,* 82, 1023 (1998), *Experimental Nephrology,* 5, 429 (1997), *Nippon Rinsho - Japanese Journal of Clinical Medicine,* 54, 2583 (1996)] and the like.

For example, production of the peptide of the present invention can be inhibited by administering the DNA or oligonucleotide of the present invention. That is, transcription of a gene encoding the peptide of the present invention and translation of mRNA encoding the peptide of the present invention each can be inhibited by using the DNA or oligonucleotide of the present invention. The method for administering the DNA or oligonucleotide of the present invention include a method in which it is directly administered to a patient and a method in which the DNA or oligonucleotide of the present invention is introduced into a cell collected from a patient and then the cell is returned into the living body. For example, the expression of the peptide of the present invention is controlled inside the body of a patient by administering the DNA or oligonucleotide of the present invention to the living body via an appropriate virus vector sch as retrovirus, adenovirus, adeno-associated virus, herpes simplex virus, lentivirus, Sendai virus or the like, and then administering it into the living body, or by via an artificial vehicle structure such as a liposome.

(5)Method for obtaining promoter region of a gene encoding the peptide of the present invention using the DNA or oligonucleotide of the present invention

The promoter region of the gene can be obtained by a known method [*New Cell Technology Experimentation Protocols (Shin Saibo Kogaku Jikken Protocols),* edited by Division of Carcinostatic Research, Institute of Medical Science, The University of Tokyo, published by Shujun-sha (1993)] using the DNA or oligonucleotide of the present invention as the probe.

The promoter region includes all promoter regions which are playing a role in the transcription of genes encoding the peptide of the present invention in mammal cells. Specific examples include promoter regions which play a role in the transcription of genes encoding the peptide of the present invention in the human bone marrow, the spleen, smooth muscle cells, macrophages or neutrophils. The promoter can also be used in the screening method described below.

The promoter region includes all promoter regions which are playing a role in the transcription of genes encoding the peptide of the present invention in mammal cells. Specific examples include promoter regions which play a role in the transcription of genes encoding the peptide of the present invention in the human bone marrow, the spleen, smooth muscle cells, macrophages or neutrophils. The promoter can also be used in the screening method described below.

(6)Method for diagnosing various diseases

The peptide of the present invention has an activity to change a concentration of an intracellular calcium ion as an intracellular signal transduction substance in a bone, the spleen or cells containing these tissues derived from the living body. Also, the gene encoding the peptide of the present invention is expressed in the bone marrow, the spleen, smooth muscle cells, macrophages or neutrophils, and relates to various diseases.

Thus, diseases which accompany infection and inflammation, diseases which accompany abnormal differentiation proliferation of smooth muscle cells, diseases which accompany abnormal activation of fibroblasts, diseases which accompany abnormal activation of a synovial tissue, diseases which accompany disorder of pancreatic β cells, diseases which accompany abnormality of osteoblasts or osteoclasts, diseases which accompany abnormal activation of immunocytes, diseases which accompany disorder of a blood vessel, diseases of an eye based on angiogenesis, diseases which accompany neopla, diseases in which linkage relation to gene regions of a major histocompatibility antigen is confirmed and the like can be diagnosed by the method for detecting expression or mutation of a gene encoding the peptide of the present invention using the DNA or oligonucleotide of the present invention, described in the above 6(1) and 6(2), or the immunological detection method using antibodies described in the above 6(3).

The DNA encoding the peptide of the present invention, specifically the DNA encoding a peptide comprising the amino acid sequence represented by SEQ ID NO:1, is a part of a gene encoding human AIF-1 whose function is unclear by homology analysis.

It is known that expression of AIF-1 increases specifically in the host-side macrophage and neutrophil infiltrated into a region where a chronic rejection reaction occurs by allograft transplantation of the heart, that expression of the AIF-1 gene in macrophage and neutrophil increases when stimulated with interferon γ (IFNγ), that macrophage infiltrated into the pancreas in an insulin-dependent diabetes mellitus model highly expresses the AIF-1 gene, and that the AIF-1 gene is positioned in the human major histocompatibility antigen HLA class III region where a large number of genes concerned in immune inflammation are present in the chromosome. Accordingly, diseases which accompany infection and inflammation, diseases which accompany abnormal activation of fibroblasts, diseases which accompany abnormal activation of a synovial tissue, diseases which accompany disorder of pancreatic β cells and diseases which accompany abnormal activation of immunocytes can be diagnosed by detecting or determining the peptide of the present invention or a DNA encoding the peptide.

It is also known that increased expression of the AIF-1 gene is observed for 1 to 3 days in a rat blood vessel treated with percutaneous transluminal coronary angioplasty (PTCA) but it is returned after 7 days, and that expression of the AIF-1 gene increases by the stimulation of bovine fetal serum, IFNγ, interleukin 1β (IL-1β), platelet-derived growth factor (PDGF) or transforming growth factor β (TGFβ) when human smooth muscle cells are cultured in vitro and stimulated with various types of cytokine. Accordingly, diseases which accompany abnormal differentiation proliferation of smooth muscle cells, diseases which accompany disorder of a blood vessel, diseases of an eye based on angiogenesis and diseases which accompany abnormal activation of fibroblasts can be diagnosed by detecting or determining the peptide of the present invention or a DNA encoding the peptide.

In addition, it is known that the AIF-1 gene is positioned in the human major histocompatibility antigen HLA class III region where a large number of genes concerned in immune inflammation are present in the chromosome, and that the gene regions of the human major histocompatibility antigen relates to diseases closely related to immune inflammation reactions. Accordingly, diseases in which linkage to gene regions of a major histocompatibility antigen is confirmed can be diagnosed by detecting or determining the peptide of the present invention or a DNA encoding the peptide.

The peptide of the present invention has an activity to change a concentration of an intracellular calcium ion, which is an intracellular signal transduction substance, in living body-derived bone, spleen or cells constituting these tissues, so that it is considered that the peptide relates to diseases which accompany neopla, diseases which accompany abnormality of osteoblasts or osteoclasts, diseases which accompany infection and inflammation, diseases which accompany abnormal activation of fibroblasts, diseases which accompany abnormal activation of a synovial tissue, diseases which accompany disorder of pancreatic β cells and diseases which accompany abnormal activation of immunocytes. Accordingly, the diseases can be diagnosed by detecting or determining the peptide of the present invention or a DNA encoding the peptide.

The diseases which accompany infection and inflammation include microbial infection, HIV infection, chronic hepatitis B, rheumatoid arthritis, sepsis, graft versus host diseases, insulin-dependent diabetes mellitus, glomerulonephritis, Crohn's disease, traumatic brain damage, inflammatory bowel disease and the like.

The diseases which accompany abnormal activation of fibroblasts include psoriasis and the like.

The diseases which accompany abnormal activation of a synovial tissue include rheumatic arthritis and the like.

The diseases which accompany disorder of pancreatic β cells, diabetes mellitus and the like.

The diseases which accompany abnormal activation of immunocytes include allergy, atopy, asthma, pollinosis, airway oversensitivity, autoimmune disease and the like.

The diseases which accompany abnormal differentiation and proliferation of smooth muscle cells include arteriosclerosis, re-stricture and the like.

The diseases which accompany disorder of a blood vessel include myocardial infarction, cerebral infarction, peripheral obstruction, angina pectoris, hypertension, diabetes mellitus, arteriosclerosis, SLE and the like.

The diseases of an eye based on angiogenesis include diabetic retinopathy, retinopathy of prematurity, senile macular degeneration, neovascular glaucoma and the like.

The diseases in which linkage to gene regions of a major histocompatibility antigen is confirmed include insulin-dependent diabetes mellitus, rheumatoid arthritis, tetanic spondylitis, myasthenia gravis, IgA deficiency, Hashimoto's disease, Basedow's disease, Behcet's disease and the like.

The diseases which accompany neopla include acute myelomonocytic leukemia, malignant tumor and the like.

The diseases which accompany abnormality of osteoblasts or osteoclasts include osteoporosis and the like.

Diseases relating to the peptide of the present invention can be diagnosed using the method for detecting expression or mutation of a gene encoding the peptide of the present invention.

Particularly, since RT-PCR is a convenient method, it is particularly useful as the detection method. The detection method can also be used for the determination of expressed quantities of genes.

(7)Method for treating or preventing various diseases using the peptide, DNA, oligonucleotide or antibody of the present invention

Diseases relating to the peptide of the present invention can be treated by a method for inhibiting transcription of a gene or translation of a mRNA encoding the peptide of the present invention using the DNA or oligonucleotide of the present invention described in the above 6(4).

Also, diseases relating to the peptide of the present invention can be treated by using the antibody of the present invention, preferably the antibody which neutralizes the activity of the peptide of the present invention obtained in the above 3.

In addition, diseases relating to the peptide of the present invention can be prevented or treated by ' administering the peptide of the present invention to a patient as a vaccine to thereby increase antiserum for the peptide of the present invention in the body of the patient.

The diseases relating to the peptide of the present invention include the diseases described in the above 6 (6). That is, specific examples include diseases which accompany infection and inflammation, diseases which accompany abnormal differentiation and proliferation of smooth muscle cells, diseases which accompany abnormal activation of fibroblasts, diseases which accompany abnormal activation of a synovial tissue, diseases which accompany disorder of pancreatic β cells, diseases which accompany abnormality of osteoblasts or osteoclasts, diseases which accompany abnormal activation of immunocytes, diseases which accompany disorder of a blood vessel, diseases of an eye based on angiogenesis, diseases which accompany neopla, diseases in which linkage relation to gene regions of a major histocompatibility antigen is confirmed and the like.

The method administrating the peptide, DNA, oligonucleotide or antibody of the present invention include 1) a method in which the peptide, DNA, oligonucleotide or antibody of the present invention is directly administered to a patient, 2) a method of gene therapy, 3) a method in which a DNA encoding the peptide of the present invention is inserted into a cell and expressed and then the cell is transplanted into a patient, and the like. They are described below in detail.

The method for treating a disease whose cause is a mutation or change in expression of a gene encoding the peptide of the present invention includes a method wherein a gene which controls expression of the peptide of the present invention is expressed inside the body of a patient by introducing the recombinant vector of the present invention appropriately prepared for gene therapy into a cell collected from the patient, and then returning the cell into the living body; administering it together with an appropriate virus vector such as a retrovirus, an adenovirus, an adeno-associated virus, a herpes simplex virus, a lentivirus, a Sendai virus or the like and administering it into the living body; or including it in an artificial vehicle structure such as a liposome or the like and then administering it into the living body.

In order to use a vector for gene therapy prepared by inserting a DNA encoding the peptide of the present invention into a virus vector such as a retrovirus, an adenovirus or the like or other vector for gene therapy, as a gene therapy agent or a preventive agent, it can be produced by mixing the vector for gene therapy with a base material which is used in a gene therapy agent [*Nature Genet*., 8, 42 (1994)].

As the base material used in the gene therapy agent, any base material generally used in injections can be used. Examples include distilled water, a salt solution such as sodium chloride or a mixture of sodium chloride with an inorganic salt and the like, a sugar solution such as mannitol, lactose, dextran, glucose and the like, an amino acid solution such as glycine, arginine and the like, a mixed solution such as an organic acid solution, a salt solution with a glucose solution and the like. Also, according to a conventional method, the base materials may be made into injections as solutions, suspensions or dispersants using auxiliary agents such as an osmotic pressure controlling agent; a pH adjusting agent; plant oil, e.g., sesame oil, soybean oil, *etc.*; lecithin; a surfactant, e.g., a nonionic surfactant; and the like. The injections can also be made into preparations which are dissolved when used, by a treatment such as disintegration, freeze-drying or the like. The gene therapy agent of the present invention can be used in the treatment directly when it is a liquid, or, when it is a solid, by dissolving it just before the gene therapy in the base material which is sterilized, if necessary. The administration method of the gene therapy agent of the present invention include a method in which the agent is topically administered so that it is absorbed in the treating region of a patient.

In addition, the DNA can also be transported into the desired treating region by a non-viral gene transfer method.

The non-viral gene transfer method known in the field include the calcium phosphate coprecipitation method [*Virology,* 52, 456-467 (1973); *Science,* 209, 1414-1422 (1980)], the microinjection method [*Proc. Natl. Acad. Sci. USA,* 77, 5399-5403 (1980); *Proc. Natl. Acad. Sci. USA*, 77, 7380-7384 (1980); *Cell,* 27, 223-231 (1981); *Nature,* 294, 92-94 (1981)], the membrane fusion-mediated transfer method via liposome [*Proc. Natl. Acad. Sci. USA*, 84, 7413-7417 (1987); *Biochemistry*, 28, 9508-9514 (1989); *J. Biol. Chem.*, 264, 12126-12129 (1989); *Hum. Gene Ther.,* 3, 267-275 (1992); *Science*, 249, 1285-1288 (1990); *Circulation,* 83, 2007-2011 (1992)], the direct DNA incorporation and receptor-mediated DNA transfer methods [*Science,* 247, 1465-1468 (1990); *J. Biol. Chem.*, 266, 14338-14342 (1991); *Proc. Natl. Acad. Sci. USA*, 87, 3655-3659 (1991); *J. Biol. Chem.*, 264, 16985-16987 (1989); *BioTechniques,* 11, 474-485 (1991); *Proc. Natl. Acad. Sci. USA*, 87, 3410-3414 (1990); *Proc. Natl. Acad. Sci. USA,* 88, 4255-4259 (1991); *Proc. Natl. Acad. Sci. USA*, 87, 4033-4037 (1990); *Proc. Natl. Acad. Sci. USA*, 88, 8850-8854 (1991); *Hum. Gene Ther.*, 3, 147-154 (1991)] and the like.

In the membrane fusion-mediated transfer method via liposome, it has been reported based on a study about tumor that a topical gene of a target tissue can be topically incorporated or expressed by directly administering a liposome preparation to the tissue [*Hum. Gene TAer.*, 3, 399 (1992)].

(8)Medicament comprising the peptide, DNA, oligonucleotide or antibody of the present invention

The medicament comprising the peptide, DNA, oligonucleotide or antibody of the present invention can diagnose, treat or prevent diseases relating to the peptide of the present invention, using the method described in the above 6(6) or 6(7).

The diseases relating to the peptide of the present invention include the diseases described in the above 6 (6). Specific examples include diseases which accompany infection and inflammation, diseases which accompanies abnormal differentiation proliferation of smooth muscle cells, diseases which accompany abnormal activation of fibroblasts, diseases which accompany abnormal activation of a synovial tissue, diseases which accompany disorder of pancreatic β cells, diseases which accompany abnormality of osteoblasts or osteoclasts, diseases which accompany abnormal activation of immunocytes, diseases which accompany disorder of a blood vessel, disease of an eye based on angiogenesis, diseases which accompany neopla, diseases in which linkage relation to gene regions of a major histocompatibility antigen is confirmed, and the like.

Also, even if a mutation or change in expression of a gene encoding the peptide of the present invention is not the direct cause of diseases, the peptide, DNA, oligonucleotide or antibody of the present invention is useful as a medicament for the above diseases when the symptomatic therapy is possible.

The medicament comprising the peptide, DNA, oligonucleotide or antibody of the present invention can be used as a therapeutic agent by administering the compounds alone, but generally, it is preferable to provide it as a pharmaceutical preparation produced by any method well known in the technical field of pharmaceutics, by mixing the peptide, DNA, oligonucleotide or antibody of the present invention with one or at least two pharmaceutically acceptable carriers.

Preferably, an aseptic solution prepared by dissolving the compound in an aqueous carrier such as water or an aqueous solution of sodium chloride, glycine, glucose, human albumin or the like is used. In addition, in order to bring the pharmaceutical preparation solution close to physiological conditions, pharmaceutically acceptable additives such as buffer agents, tonicity agents and the like, e.g., sodium acetate, sodium chloride, sodium lactate, potassium chloride, sodium citrate and the like, can also be added. Also, it can be used by freeze-drying and storing and then dissolving in an appropriate solvent when used.

In addition, it is possible to administer the DNA of the present invention into the living body by inserting it into a virus vector or the like described in the above 6(4).

It is preferable to use the most effective route of administration in carrying out the treatment. Examples include oral administration and parenteral administration such as buccal, airway, rectal, subcutaneous, intramuscular, intravenous and the like. The dosage forms include sprays, capsules, tablets, granules, syrups, emulsions, suppositories, injections, ointments, tapes and the like.

Preferred pharmaceutical preparations suitable for the oral administration include emulsions, syrups, capsules, tablets, powders, granules and the like. For example, liquid preparations such as emulsions, syrups and the like can be produced using, as additives, water; saccharides such as sucrose, sorbitol, fructose, *etc.*; glycols such as polyethylene glycol, propylene glycol, *etc.*; oils such as sesame oil, olive oil, soybean oil, *etc.*; antiseptics such as p-hydroxybenzoates, *etc.*; flavors such as strawberry flavor, peppermint, *etc.*; and the like. Capsules, tablets, powders, granules and the like can be produced using, as additives, fillers such as lactose, glucose, sucrose, mannitol, *etc.*; disintegrating agents such as starch, sodium alginate, *etc.*; lubricants such as magnesium stearate, talc, *etc.*; binders such as polyvinyl alcohol, hydroxypropylcellulose, gelatin, *etc.*; surfactants such as fatty acid ester, *etc.*; plasticizers such as glycerol, etc.; and the like.

Preferred pharmaceutical preparations suitable for parenteral administration include injections, suppositories, sprays and the like. For example, injections are prepared using a carrier comprising a salt solution, a glucose solution or a mixture of both, or the like. Suppositories are prepared using a carrier such as cacao butter, hydrogenated fat, carboxylic acid or the like. Also, sprays are prepared using the peptide, DNA, oligonucleotide or antibody as it is, or using a carrier which can disperse the peptide, DNA, oligonucleotide or antibody as fine particles to facilitate the absorption, or the like. The carrier include lactose, glycerol and the like. Depending on the properties of the peptide, DNA, oligonucleotide or antibody and the carrier to be used, it is possible to prepare pharmaceutical preparations such as aerosols, dry powders and the like. In addition, the components exemplified as the additives of the oral preparation can also be added to the parenteral preparation.

The dose or administration frequency varies depending on the intended therapeutic effect, administration method, treating period, age, body weight and the like, but is usually 10 μg/kg to 8 mg/kg per day per adult.

(9)Method for screening a receptor which specifically binds to the peptide of the present invention using the peptide

A receptor which specifically binds to the peptide can be screened by using a method for identifying a substance which directly binds to the peptide of the present invention, or the like. The method includes a method using a labeled ligand, and the like. Specifically, the screening can be carried out according to a method in which an endothelin receptor was identified using $^{125}$I-labeled endothelin (T. Sakurai *et al., Nature*, 348, 732, 1990) or the like. The receptor can be applied to a therapeutic agent for diseases relating to the peptide of the present invention, or to studies on the signal transduction systems and biological functions relating to the peptide of the present invention.

(10)Method for analyzing expression of a gene encoding the peptide of the present invention using the transformant of the present invention

A substance which controls transcription of the gene encoding the peptide of the present invention, a substance which relates to the transcription controlling function by the peptide of the present invention or a gene which is subjected to transcription control by the peptide of the present invention can be screened by coexisting the transformant of the present invention with various substances to be tested and analyzing the expressed level of a gene in the transformant.

(11)Preparation of knockout non-human animal using the DNA of the present invention

A mutant clone in which a gene encoding the peptide of the present invention on the chromosome in the embryonic stem cell of an objective non-human animal such as cow, sheep, goat, pig, horse, mouse, domestic fowl or the like is inactivated or substituted with any sequence by a homologous recombination technique [e.g., *Nature,* 326, 6110, 295 (1987)] is prepared using a recombinant vector ' comprising the DNA of the present invention [e.g., *Nature,* 350, 6315, 243 (1991)]. A chimeric individual comprising the embryonic stem cell and normal cell can be prepared using a mutant clone of an embryonic stem cell by an injection chimera method, an aggregation chimera method or the like to a blastocyst of an animal fertilized egg. When the chimera individual is crossed with a normal individual, individuals having any mutation in a gene encoding the peptide of the present invention on the chromosome in cells of the whole body can be obtained, and when the individuals are further crossed, a knockout non-human animal can be obtained as an individual in which expression of the gene encoding the peptide of the present invention is partially or completely inhibited from homologous individuals in which both of the homologous chromosomes are mutated.

Also, it is possible to prepare a knockout non-human animal by introducing a mutation into any position of a chromosomal gene encoding the peptide of the present invention. For example, it is possible to modify activity of a product by introducing a mutation into the translation region of the chromosomal gene encoding the peptide of the present invention by substitution, deletion, insertion or the like of a nucleotide. It is also possible to modify the degree of expression, period, tissue specificity and the like by introducing similar mutation into its expression controlling region. In addition, it is possible to control expressing period, expressing site, expressing quantity and the like more positively by its combination with Cre-loxP system. An example in which a desired gene was deleted in only a specific region in the brain using the promoter expressing in the region [*Cell*, 87, 7, 1317 (1996)] and an example in which a desired gene was organ-specifically deleted in an intended period using a Cre-expressing adenovirus *[Science,* 278, 5335 (1997)] are known.

Accordingly, a knockout non-human animal in which expression of the chromosomal gene encoding the peptide of the present invention can be controlled in an optional period and tissue or which has any insertion, deletion or substitution in its translation region or expression controlling region can be prepared in the same manner.

The knockout non-human animal can induce symptoms of various diseases caused by the peptide of the present invention during any period at any degree or in any region.

Thus, the knockout non-human animal of the present invention becomes a remarkably useful animal model in treating or preventing various diseases caused by the peptide of the present invention. Particularly, it is markedly useful as an evaluation model for the therapeutic agents, the preventive agents, physiologically functional food, healthy food and the like.

(12)Method for screening agonist or antagonist for the peptide of the present invention using the peptide of the present invention

In the method for screening agonist or antagonist of a physiologically active peptide using the peptide, the screening can be carried out by subjecting the peptide to radioisotope labeling with $^{125}I$ or the like or fluorescence labeling with fluorescein or the like, and adding a compound to be tested to a system which can measure binding activity of the peptide to various cells, animal tissues and the like. Also, the screening can be carried out by adding a compound to be tested to a system which can measure changes in various cells, animal tissues and the like caused by the peptide (e.g., changes in intracellular calcium ion concentration).

The present invention is described by the following examples more specifically, but the examples merely show simple exemplification of the present invention and the scope of the present invention is not limited thereto.

BRIEF DESCRIPTION OF THE DRAWINGS

Fig. 1 shows a result of the measurement of physiological activities of angiotensin II and bradykinin using a) the adrenal gland, b) the womb, c) a blood vessel and d) the spleen of an apoaequorin transgenic mouse. The RLU in the drawing represents a relative luminescence unit (the same shall apply hereinafter).

The drawing shows that (1) RPMI1640, (2) angiotensin II at a final concentration of 1 μmol/l, (3) bradykinin at a final concentration of 10 μmol/l, (4) A23187 at a final concentration of 1 μmol/l and (5) Triton X-100 at a final concentration of 2% were added at the time indicated by each arrow.

Fig. 2 shows a result of the measurement of physiological activities of endothelin and calcitonin using a) a blood vessel and b) the heart of an apoaequorin transgenic mouse.

The drawing shows that (1) RPMI1640, (2) endothelin at a final concentration of 10 nmol/l, (3) calcitonin at a final concentration of 10 nmol/l, (4) A23187 at a final concentration of 1 μmol/l and (5) Triton X-100 at a final concentration of 2% were added at the time indicated by each arrow.

Fig. 3 shows a result of the measurement of physiological activities of carbachol, α-methylserotonin, ATP and phenylephrine using a) the thymus, b) a blood vessel and c) the spleen of an apoaequorin transgenic mouse.

The drawing shows that (1) RPMI1640, (2) carbachol at a final concentration of 20 μmol/l, (3) α-methylserotonin at a final concentration of 20 μmol/l, (4) ATP at a final concentration of 100 μmol/l, (5) phenylephrine at a final concentration of 20 μmol/l, (6) A23187 at a final concentration of 1 μmol/l and (7) Triton X-100 at a final concentration of 2% were added at the time indicated by each arrow.

Fig. 4 shows a result of the measurement of the physiological activity of Peptide 1 produced in Example 1 using the spleen of an apoaequorin transgenic mouse.

The drawing shows that (1) RPMI1640, (2) Peptide 1 at a final concentration of 10 μmol/l, (3) ATP at a final concentration of 100 μmol/l, (4) A23187 at a final concentration of 1 μmol/l and (5) Triton X-100 at a final concentration of 2% were added at the time indicated by each arrow.

Fig. 5 shows a result of the measurement of the physiological activity of Peptide 2 produced in Example 1 using the spleen of an apoaequorin transgenic mouse.

The drawing shows that (1) RPMI1640, (2) Peptide 2 at a final concentration of 10 μmol/l, (3) ATP at a final concentration of 100 μmol/l, (4) A23187 at a final concentration of 1 μmol/l and (5) Triton X-100 at a final concentration of 2% were added at the time indicated by each arrow.

Fig. 6 shows a result of the measurement of the physiological activity of Peptide 3 produced in Example 1 using the spleen of an apoaequorin transgenic mouse.

The drawing shows that (1) RPMI1640, (2) Peptide 3 at a final concentration of 10 μmol/l, (3) ATP at a final concentration of 100 μmol/l, (4) A23187 at a final concentration of 1 μmol/l and (5) Triton X-100 at a final concentration of 2% were added at the time indicated by each arrow.

Fig. 7 shows a result of the measurement of the physiological activity of Peptide 1S produced in Example 1 using the spleen of an apoaequorin transgenic mouse.

The drawing shows that (1) RPMI1640, (2) Peptide 1S at a final concentration of 10 μmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 8 shows a result of the measurement of the physiological activity of Peptide 2S produced in Example 1 using the spleen of an apoaequorin transgenic mouse.

The drawing shows that (1) RPMI1640, (2) Peptide 2S at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 9 shows a result of the measurement of the physiological activity of Peptide 3S produced in Example 1 using the spleen of an apoaequorin transgenic mouse.

The drawing shows that (1) RPMI1640, (2) Peptide 3S at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 10 shows a result of the measurement of the physiological activity of Peptide 1 produced in Example 1 using the cranial bone of an apoaequorin transgenic mouse.

The drawing shows that (1) RPMI1640, (2) Peptide 1 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 11 shows a result of the measurement of the physiological activity of Peptide 2 produced in Example 1 using the cranial bone of an apoaequorin transgenic mouse.

The drawing shows that (1) RPMI1640, (2) Peptide 2 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 12 shows a result of the measurement of the physiological activity of Peptide 3 produced in Example 1 using the cranial bone of an apoaequorin transgenic mouse.

The drawing shows that (1) RPMI1640, (2) Peptide 3 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 13 shows a result of the measurement of the physiological activity of Peptide 1S produced in Example 1 using the cranial bone of an apoaequorin transgenic mouse.

The drawing shows that (1) RPMI1640, (2) Peptide 1S at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 14 shows a result of the measurement of the physiological activity of Peptide 2S produced in Example 1 using the cranial bone of an apoaequorin transgenic mouse.

The drawing shows that (1) RPMI1640, (2) Peptide 2S at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 15 shows a result of the measurement of the physiological activity of Peptide 3S produced in Example 1 using the cranial bone of an apoaequorin transgenic mouse.

The drawing shows that (1) RPMI1640, (2) Peptide 3S at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 16 shows a result of the measurement of the physiological activity of Peptide 1 produced in Example 1 using the thymus of an apoaequorin transgenic mouse.

The drawing shows that (1) RPMI1640, (2) Peptide 1 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 17 shows a result of the measurement of the physiological activity of Peptide 2 produced in Example 1 using the thymus of an apoaequorin transgenic mouse.

The drawing shows that (1) RPMI1640, (2) Peptide 2 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 18 shows a result of the measurement of the physiological activity of Peptide 3 produced in Example 1 using the thymus of an apoaequorin transgenic mouse.

The drawing shows that (1) RPMI1640, (2) Peptide 3 at a final concentration of 10 µmol/l, (3) ATP at a final concentration of 100 µmol/l, (4) A23187 at a final concentration of 1 µmol/l and (5) Triton X-100 at a final concentration of 2% were added at the time indicated by each arrow.

Fig. 19 shows a result of the measurement of the physiological activity of Peptide 1 produced in Example 1 using the spleen of an apoaequorin transgenic mouse.

The drawing shows that (1) RPMI1640, (2) Peptide 1 at a final concentration of 10 nmol/l, (3) Peptide 1 at a final concentration of 100 nmol/l, (4) Peptide 1 at a final concentration of 1 μmol/l, (5) Peptide 1 at a final concentration of 10 μmol/l, (6) ATP at a final concentration of 100 μmol/l, (7) A23187 at a final concentration of 1 μmol/l and (8) Triton X-100 at a final concentration of 2% were added at the time indicated by each arrow.

Fig. 20 shows a result of the measurement of the physiological activity of Peptide 2 produced in Example 1 using the spleen of an apoaequorin transgenic mouse.

The drawing shows that (1) RPMI1640, (2) Peptide 2 at a final concentration of 10 nmol/l, (3) Peptide 2 at a final concentration of 100 nmol/l, (4) Peptide 2 at a final concentration of 1 μmol/l, (5) Peptide 2 at a final concentration of 10 μmol/l, (6) ATP at a final concentration of 100 μmol/l, (7) A23187 at a final concentration of 1 μmol/l and (8) Triton X-100 at a final concentration of 2% were added at the time indicated by each arrow.

Fig. 21 shows a result of the measurement of the physiological activity of Peptide 3 produced in Example 1 using the spleen of an apoaequorin transgenic mouse.

The drawing shows that (1) RPMI1640, (2) Peptide 3 at a final concentration of 10 nmol/l, (3) Peptide 3 at a final concentration of 100 nmol/l, (4) Peptide 3 at a final concentration of 1 μmol/l, (5) Peptide 3 at a final concentration of 10 μmol/l, (6) ATP at a final concentration of 100 μmol/l, (7) A23187 at a final concentration of 1 μmol/l and (8) Triton X-100 at a final concentration of 2% were added at the time indicated by each arrow.

Fig. 22 shows a result of the measurement of the physiological activity of Peptide 2 short produced in Example 1 using the spleen of an apoaequorin transgenic mouse.

The drawing shows that (1) RPMI1640, (2) Peptide 2 short at a final concentration of 10 nmol/l, (3) Peptide 2 short at a final concentration of 100 nmol/l, (4) Peptide 2 short at a final concentration of 1 μmol/l, (5) Peptide 2 short at a final concentration of 10 μmol/l, (6) ATP at a final concentration of 100 μmol/l, (7) A23187 at a final concentration of 1 μmol/l and (8) Triton X-100 at a final concentration of 2% were added at the time indicated by each arrow.

Fig. 23 shows the binding activity of each culture supernatant of monoclonal antibodies KM2947, KM2952, KM3022 and KM3030 to an antigen peptide. The black column shows a reaction of a plate on which the antigen peptide was not coated with the culture supernatants, and the oblique column shows a reaction of a plate coated with the antigen peptide with the culture supernatants.

Fig. 24 shows detection of Peptide 1 and Peptide 3 by Western blotting using KM2952.

Fig. 25 shows binding activity of KM3022 to an antigen peptide. Each column shows reaction of Peptide 1C, Peptide 1C23, Peptide 2C, Peptide 3C or KNP2N with purified KM3022.

Fig. 26 shows effect of KM3030 on the physiological activity of Peptide 1 produced in Example 1. The physiological activity of Peptide 1 was measured using the spleen of an apoaequorin transgenic mouse.

a) shows a result of the activity measurement of Peptide 1. The drawing shows that (1) RPMI1640, (2) Peptide 1 at a final concentration of 8 μmol/l and (3) Triton X-100 at a final concentration of 2% were added at the time indicated by each arrow.

b) shows a result of the measurement when KM3030 was added alone to the measuring system. The drawing shows that (1) RPMI1640, (2) KM3030 at a final concentration of 1.8 mg/l and (3) Triton X-100 at a final concentration of 2% were added at the time indicated by each arrow.

c) shows a result of the measurement when Peptide 1 and KM3030 were allowed to react at room temperature for 30 minutes. The drawing shows that (1) RPMI1640, (2) Peptide 1 at a final concentration of 6 μmol/l and KM3030 at a final concentration of 1.8 mg/ml and (3) Triton X-100 at a final concentration of 2% were added at the time indicated by each arrow.

Fig. 27 shows effect of KM2947 on the physiological activity of Peptide 3 produced in Example 1. The physiological activity of Peptide 3 was measured using the spleen of an apoaequorin transgenic mouse.

a) shows a result of the activity measurement of Peptide 3. The drawing shows that (1) RPMI1640, (2) Peptide 3 at a final concentration of 8 μmol/l and (3) Triton X-100 at a final concentration of 2% were added at the time indicated by each arrow.

b) shows a result of the measurement when KM2347 was added alone to the measuring system. The drawing shows that (1) RPMI1640, (2) KM2947 at a final concentration of 2.0 mg/ml and (3) Triton X-100 at a final concentration of 2% were added at the time indicated by each arrow.

c) shows a result of the measurement when Peptide 3 and KM2947 were allowed to react at room temperature for 30 minutes. The drawing shows that (1) RPMI1640, (2) Peptide 3 at a final concentration of 8 μmol/l and KM2947 at a final concentration of 2.0 mg/ml and (3) Triton X-100 at a final concentration of 2% were added at the time indicated by each arrow.

Fig. 28 shows a result of the measurement of physiological activities of ATP and A23187 using the splenocyte of an apoaequorin transgenic mouse.

a) shows a result of the measurement when RPMI1640 was added. The drawing shows that (1) RPMI1640 was added at the time indicated by the arrow.
b) shows a result of the measurement of ATP activity. The drawing shows that (1) ATP was added at a final concentration of 100 μmol/l at the time indicated by the arrow.
c) shows a result of the measurement of the activity of A23197. The drawing shows that (1) A23187 at a final concentration of 1 μmol/l and (2) Triton X-100 were added at the time indicated by each arrow.

Fig. 29 shows a result of the measurement of physiological activities of Peptide 1, Peptide 2 and Peptide 3 produced in Example 1 using the splenocyte of apoaequorin transgenic mouse. a) shows a result of the activity measurement of Peptide 1. b) shows a result of the activity measurement of Peptide 2. c) shows a result of the activity measurement of Peptide 3. In each case, each peptide was added to the measuring system at a final concentration of 10 μmol/l at the time indicated by each arrow.
Fig. 30 shows expression of a fusion protein of MBP with AIF-1 or AIF-2. Each lane shows a transformant introduced with a gene of the fusion protein of MBP with AIF-1 or AIF-2, and mock shows a transformant introduced with an empty vector pMAL-p2X into which the fusion protein gene was not introduced. The drawing shows a result of a case in which a) Coomassie Brilliant Blue staining, b) staining by Western blotting using anti-MBP antibody or c) staining by Western blotting using KM2946 was carried out after electrophoresis of culture media of the transformants.
Fig. 31 shows isolation of AIF-1 or AIF-2 from purified MBP fusion protein. Blood coagulation factor Xa (FXa) was added to the purified MBP fusion protein, and a peptide between MBP and AIF-1 or AIF-2 was cut out. Each lane shows a sample after 0, 3.5 or 6.0 hours after the addition of FXa. mock shows a sample which was not treated with FXa. The drawing shows a result of a case in which staining by Western blotting using KM3032 or KM2946 was carried out after electrophoresis of the samples.
Fig. 32 shows construction of plasmid pAGE107AEQ.
Fig. 33 shows construction of plasmid pBSAEQpA.
Fig. 34 shows construction of plasmid pBSKS(+)CAG promoter.
Fig. 35 shows construction of plasmid pCAG-AEQ.
Fig. 36 shows construction of plasmid ploxp#1.
Fig. 37 shows construction of plasmid ploxp.
Fig. 38 is an illustration showing construction of plasmid ploxpHPRT.
Fig. 39 shows construction of plasmid ploxpHPRT2.
Fig. 40 shows construction of plasmid pCAG-AEQ-pHPRTp.
Fig. 41 shows a result of comparison of promoter activities of respective plasmids using luminescence quantity of aequorin by A23187 stimulation as the index.
The drawing shows a result when CHOdhfr(-)DG44 cell to which (1) pCAG-AEQ, (2) pAGE107AEQ or (3) pBSAEQpA was introduced was used.
RLU represents a relative luminescence unit.
Fig. 42 shows a result of comparison of apoaequorin expression quantities of a plasmid pCAG-AEQ-pHPRTp transformant clone 21 and a mutant clone 21 Δhprt produced by removing the expression unit of hprt gene from the clone 21.

## BEST MODE FOR CARRYING OUT THE INVENTION

### Example 1

Production of peptides:

Peptides in which C-termini of the amino acid sequences represented by SEQ ID NOs:1, 2 and 5 were amidated and their control peptides was produced. Hereinafter, the thus produced peptides in which C-termini of the amino acid sequences represented by SEQ ID NOs:1, 2 and 5 were amidated are called Peptide 1, Peptide 2 and Peptide 3, respectively, and are represented by SEQ ID NOs:36, 37 and 38, respectively.
Also, as control peptides of Peptide 1, Peptide 2 and Peptide 3, peptides designed in such a manner that they have the same amino acid compositions as those of Peptides 1, 2 and 3 but the arrangement of amino acids in their sequences is randomly changed were produced and named Peptide 1S, Peptide 2S and Peptide 3S, respectively, and are represented by SEQ ID NOs:7, 8 and 9, respectively. Also, a partial peptide of the C-terminal side of Peptide 2 is called Peptide 2 short which is represented by SEQ ID NO:10.

Abbreviations of the amino acids and their protecting groups used in the present invention were used according to the recommendation by IUPAC-IUB Joint Commission on Biochemical Nomenclature (*European Journal of Biochemistry, 138*: 9 (1984)).

Unless otherwise indicated, the following abbreviations represent the following amino acids.

Arg: L-Argnine
Asn: L-Asparagine
Asp: L-Aspartic acid
Asx: L-Aspartic acid or L-asparagine
Gln: L-Glutamine
Glu: L-Glutamic acid
Glx: L-Glutamic acid or L-glutamine
Gly: Glycine
His: L-Histidine
Ile: L-Isoleucine
Leu: L-Leucine
Lys: L-Lysine
Met: L-Methionine
Phe: L-Phenylalanine
Pro: L-Proline
Ser: L-Serine
Thr: L-Threonine
Tyr: L-Tyrosine
Val: L-Valine

The following abbreviations represent protecting groups of corresponding amino acids and side chain-protecting amino acids.

Fmoc: 9-Fluorenylmethyloxycarbonyl
tBu: t-Butyl
Trt: Trityl
Pmc: 2,2,5,7,8-Pentamethylchromane-6-sulfonyl
Boc: t-Butyloxycarbonyl

Fmoc-Arg(Pmc)-OH: Nα-9-Fluorenylmethyloxycarbonyl-$N^g$-2,2,5,7,8-pentamethylchromane-6-sulfonyl-L-arginine Fmoc-Asn(Trt)-OH: Nα-9-Fluorenylmethyloxycarbonyl-Nγ-trityl-L-asparagine Fmoc-Asp(OtBu)-OH: Nα-9-Fluorenylmethyloxycarbonyl-L-asapartic acid β-t-butyl ester Fmoc-GIn(Trt)-OH: Nα-9-Fluorenylmethyloxycarbonyl-Nε-trityl-L-glutamine Fmoc-Cys(Trt)-OH: Nα-9-Fluorenylmethyloxycarbonyl-S-trityl-L-cysteine Fmoc-Glu(OtBu)-OH: Nα-9-Fluorenylmethyloxycarbonyl-L-glutamic acid γ-t-butyl ester Fmoc-His(Trt)-OH: Nα-9-Fluorenylmethyloxycarbonyl-$N^{im}$-trityl-L-histidine Fmoc-Lys(Boc)-OH: Nα-9-Fluorenylmethyloxycarbonyl-Nε-t-butyloxycarbonyl-L-lysine Fmoc-Ser(tBu)-OH: Nα-9-Fluorenylmethyloxycarbonyl-O-t-butyl-L-serine Fmoc-Thr(tBu)-OH: Nα-9-Fluorenylmethyloxycarbonyl-O-t-butyl-L-threonine Fmoc-Tyr(tBu)-OH: Nα-9-Fluorenylmethyloxycarbonyl-O-t-butyl-L-tyrosine

The following abbreviations represent the following corresponding reaction solvent, reaction reagent and the like. HBTU: 2-(1H-benzotriazol-1-yl)-1,1,3,3-tetramethyluronium hexafluorophosphate, HOBT: N-hydroxybenzotriazole, DMF: N,N-dimethylformamide, NMP: N-methylpyrrolidone, TFA: trifluoroacetic acid, DIEA: diisopropylethylamine

In the following Examples, physicochemical properties of compounds were measured by the following methods.

Mass spectrometry was carried out by the MALDI-TOFMS method using a mass spectrometer REFLEX manufactured by Bulker, or by the FAB-MS method using JMS-HX110A manufactured by JEOL. Amino acid analysis was carried out in a manner similar to the method of Cohen, S.A. et al. [*Analytical Biochemistry,* 222, 19 (1994)]. Hydrolysis was carried out at 110°C for 20 hours in a steam of hydrochloric acid, and amino acid composition of the hydrolysate was analyzed using Waters AccQ-Tag amino acid analyzer (manufactured by Waters).

Seven peptides were produced specifically as follows.

(1)Synthesis of Peptide 1 (SEQ ID NO:36: H-Met-Leu-Glu-Lys-Leu-Gly-Val-Pro-Lys-Thr-His-Leu-Glu-Leu-Lys-Lys-Leu-Ile-Gly-Glu-Val-Ser-Ser-Gly-Ser-Gly-Glu-Thr-Phe-Ser-Tyr-Pro-Asp-Phe-Leu-Arg-Met-Met-Leu-$NH_2$)

Into the reaction vessel of an automatic synthesizer (manufactured by Shimadzu Corporation), 100 mg of a carrier resin (NovaSyn TGR resin, manufactured by Nova Biochem) to which 20 μmol of Fmoc-NH had been linked was placed, 900 μl of DMF was added thereto, followed by stirring for 1 minute, the solution was discharged and then the following steps were carried out according to the synthesis program of Shimadzu Corporation.

(a) To the reaction vessel, 900 μl of 30% piperidine-DMF solution was added, the mixture was stirred for 4 minutes, the solution was discharged, and this step was repeated once more.
(b) The carrier resin was washed with 900 μl of DMF for 1 minute, the solution was discharged, and this step was repeated 5 times.
(c) Fmoc-Leu-OH (200 μmol), HBTU (200 μmol), HOBt monohydrate (200 μmol) and DIEA (400 μmol) were mixed in DMF (1040 μl), the resulting solution was added to the resin, the mixture was stirred for 30 minutes, and the solution was discharged.
(d) The carrier resin was washed with 900 μl of DMF for 1 minute, the solution was discharged, and this step was repeated 5 times.

Thus, Fmoc-Leu-NH was synthesized on the carrier.
Next, after the steps of (a) and (b), condensation reaction was carried out using Fmoc-Met-OH in the step of (c), and Fmoc-Met-Leu-NH was synthesized on the carrier after the washing step of (d).
Subsequently, (a) to (d) were repeated using Fmoc-Met-OH, Fmoc-Arg(Pmc)-OH, Fmoc-Leu-OH, Fmoc-Phe-OH, Fmoc-Asp(OtBu)-OH, Fmoc-Pro-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Gly-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Gly-OH, Fmoc-Ile-OH, Fmoc-Leu-OH, Fmoc-Lys(Boc)-OH, Fmoc-Lys(Boc)-OH, Fmoc-Leu-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Leu-OH and Fmoc-Met-OH in this order in the step (c), the deprotection and washing steps of (a) and (b) were carried out, washing was carried out using methanol and butyl ether in this order, and drying was carried out for 12 hours under a reduced pressure to obtain the carrier resin to which a side chain protected peptide was linked. Herein, a mixed solvent of 240 μl of NMP and 800 μl of DMF was used instead of 900 μl of DMF at the time of the condensation of Fmoc-Phe-OH and Fmoc-Pro-OH. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added, and allowed to stand at room temperature for 8 hours to remove the side chain protecting group and cut out the peptide from the resin. The resin was filtered, about 10 ml of ether was added to the resulting solution, and the resulting precipitate was recovered by centrifugation and decantation to give 82.0 mg of the product as a crude peptide. The synthesis at the same scale was repeated to further give 87.3 mg of the crude peptide.
The crude product was dissolved in 16 ml of an aqueous acetic acid solution and purified by HPLC using a reverse phase column (manufactured by SHISEIDO, CAPCELL PAK C18, 30 mm I.D. x 250 mm). Elution was carried out by a linear density gradient method in which an aqueous solution of 90% acetonitrile containing 0.1% TFA was added to an aqueous solution of 0.1% TFA, and detected at 220 nm to give a fraction containing Peptide 1. The fraction was freeze-dried to give 37.3 mg of TFA salt of Peptide 1.
The resulting TFA salt was again dissolved in an aqueous acetic acid solution and applied to an anionic ion exchange column (manufactured by Asahi Chemical Industry, Asahipak ES-502N, 21.5 mm I.D. × 100 mm), and Peptide 1 was eluted with water containing 1% acetic acid and 20% acetonitrile. The elute was freeze-dried to give 29.9 mg of Peptide 1 as a acetate.
Mass spectrometry (TOFMS); m/z = 4381.4 (monoisotopic mass, $M+H^+$)
Amino acid analysis; Asx 1.1 (1), Ser 4.0 (4), Glx 4.3 (4), Gly 4.4 (4), His 1.0 (1), Arg 1.1 (1), Thr 2.1 (2), Pro 2.1 (2), Tyr 1.1 (1), Val 2.0 (2), Met 3.1 (3), Lys 4.2 (4), Ile 1.1 (1), Leu 7.3 (7), Phe 2.1 (2)

(2)Synthesis of Peptide 2 (SEQ ID NO:37: H-Met-Leu-Glu-Lys-Leu-Gly-Val-Pro-Lys-Thr-His-Leu-Glu-Leu-Lys-Arg-Leu-Ile-Arg-Glu-Val-Ser-Ser-Gly-Ser-Glu-Glu-Thr-Phe-Ser-Tyr-Ser-Asp-Phe-Leu-Arg-Met-Met-Leu-$NH_2$)

Using 60 mg of a carrier resin (NovaSyn TGR resin, manufactured by Nova Biochem) to which 12.6 μmol of Fmoc-NH was linked, as the starting material, the carrier resin was condensed with Fmoc-Leu-OH Fmoc-Met-OH, Fmoc-Met-OH, Fmoc-Arg(Pmc)-OH, Fmoc-Leu-OH, Fmoc-Phe-OH, Fmoc-Asp(OtBu)-OH, Fmoc-Ser(tBu)-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Glu(OtBu)-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Arg(Pmc)-OH, Fmoc-Ile-OH, Fmoc-Leu-OH, Fmoc-Arg(Pmc)-OH, Fmoc-Lys(Boc)-OH, Fmoc-Leu-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Leu-OH and Fmoc-Met-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 66.7 mg of crude peptide was obtained, dissolved in an aqueous acetic acid solution and purified by HPLC using a reverse phase column to give 14.4 mg of Peptide 2.
Mass spectrometry (TOFMS); m/z = 4569.8 (monoisotopic mass, $M+H^+$)
Amino acid analysis; Asx 1.0 (1), Glx 5.2 (5), Ser 4.8 (5), Gly 2.2 (2), His 1.0 (1), Arg 2.9 (3), Thr 1.8 (2), Pro 1.0 (1), Tyr 1.0 (1), Val 2.0 (2), Met 3.0 (3), Ile 0.9 (1), Leu 7.1 (7), Phe 2.1 (2), Lys 2.9 (3)

(3)Synthesis of Peptide 3 (SEQ ID NO:38: H-Met-Met-Glu-Lys-Leu-Gly-Val-Pro-Lys-Thr-His-Leu-Glu-Met-Lys-Lys-Met-Ile-Ser-Glu-Val-Thr-Gly-Gly-Val-Ser-Asp-Thr-Ile-Ser-Tyr-Arg-Asp-Phe-Val-Asn-Met-Met-Leu-$NH_2$)

Using 60 mg of a carrier resin (NovaSyn TGR resin, manufactured by Nova Biochem) to which 12.6 μmol of Fmoc-NH was linked, as the starting material, the carrier resin was condensed with Fmoc-Leu-OH, Fmoc-Met-OH, Fmoc-Met-OH, Fmoc-Asn(Trt)-OH, Fmoc-Val-OH, Fmoc-Phe-OH, Fmoc-Asp(OtBu)-OH, Fmoc-Arg(Pmc)-OH, Fmoc-Tyr(tBu)-OH, Fmoc-Ser(tBu)-OH, Fmoc-Ile-OH, Fmoc-Thr(tBu)-OH, Fmoc-Asp(OtBu)-OH, Fmoc-Ser(tBu)-OH, Fmoc-Val-OH, Fmoc-Gly-OH, Fmoc-Gly-OH, Fmoc-Thr(tBu)-OH, Fmoc-Val-OH, Fmoc-Glu(OtBu)-OH, Fmoc-Ser(tBu)-OH, Fmoc-Ile-OH, Fmoc-Met-OH, Fmoc-Lys(Boc)-OH, Fmoc-Lys(Boc)-OH, Fmoc-Met-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Met-OH and Fmoc-Met-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 55.2 mg of crude peptide was obtained, dissolved in an aqueous acetic acid solution and purified by HPLC using a reverse phase column to give 12.0 mg of Peptide 3.
Mass spectrometry (TOFMS); m/z = 4445.4 (monoisotopic mass, $M+H^+$)
Amino acid analysis; Asx 2.8 (3), Glx 3.1 (3), Ser 3.0 (3), Gly 3.1 (3), His 1.1 (1), Arg 1.1 (1), Thr 2.9 (3), Pro 1.0 (1), Tyr 1.0 (1), Val 4.0 (4), Met 5.7 (6), Ile 2.1 (2), Leu 3.2 (3), Phe 1.0 (1), Lys 4.0 (4)

(4)Synthesis of Peptide IS (SEQ ID NO:7: H-Leu-Ser-Pro-His-Thr-Lys-Leu-Ser-Tyr-Gly-Glu-Gly-Val-Phe-Leu-Arg-Leu-Ser-Lys-Ser-Glu-Gly-Leu-Met-Glu-Ile-Lys-Leu-Met-Glu-Asp-Leu-Met-Val-Phe-Pro-Lys-Thr-Gly-$NH_2$)

Using 60 mg of a carrier resin (NovaSyn TGR resin, manufactured by Nova Biochem) to which 12.6 μmol of Fmoc-NH was linked, as the starting material, the carrier resin was condensed with Fmoc-Gly-OH, Fmoc-Thr(tBu)-OH, Fmoc-Lys(Boc)-OH, Fmoc-Pro-OH, Fmoc-Phe-OH, Fmoc-Val-OH, Fmoc-Met-OH, Fmoc-Leu-OH, Fmoc-Asp(OtBu) -OH, Fmoc-Glu(OtBu)-OH, Fmoc-Met-OH, Fmoc-Leu-OH, Fmoc-Lys(Boc)-OH, Fmoc-Ile-OH, Fmoc-Glu(OtBu)-OH, Fmoc-Met-OH, Fmoc-Leu-OH, Fmoc-Gly-OH, Fmoc-Glu(OtBu)-OH, Fmoc-Ser(tBu)-OH, Fmoc-Lys(Boc)-OH, Fmoc-Ser(tBu)-OH, Fmoc-Leu-OH, Fmoc-Arg(Pmc)-OH, Fmoc-Leu-OH, Fmoc-Phe-OH, Fmoc-Val-OH, Fmoc-Gly-OH, Fmoc-Glu(OtBu)-OH, Fmoc-Gly-OH, Fmoc-Tyr(tBu)-OH, Fmoc-Ser(tBu)-OH, Fmoc-Leu-OH, Fmoc-Lys(Boc)-OH, Fmoc-Thr(tBu)-OH, Fmoc-His(Trt)-OH, Fmoc-Pro-OH, Fmoc-Ser(tBu)-OH and Fmoc-Leu-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 59.3 mg of crude peptide was obtained, dissolved in an aqueous acetic acid solution and purified by HPLC using a reverse phase column to give 10.3 mg of Peptide 1S
Mass spectrometry (TOFMS); m/z = 4380.9 (monoisotopic mass, $M+H^+$)
Amino acid analysis; Asx 0.9 (1), Ser 3.9 (4), Glx 4.1 (4), Gly 4.4 (4), His 1.0 (1), Arg 1.1 (1), Thr 2.2 (2), Pro 2.1 (2), Tyr 1.0 (1), Val 1.8 (2), Met 2.7 (3), Lys 4.0 (4), Ile 0.8 (1), Leu 7.2 (7), Phe 1.8 (2)

(5) Synthesis of Peptide 2S (SEQ ID NO:8: H-Leu-Ser-Pro-His-Thr-Lys-Leu-Ser-Tyr-Arg-Glu-Glu-Val-Phe-Leu-Arg-Leu-Ser-Lys-Ser-Glu-Gly-Leu-Met-Glu-Ile-Lys-Leu-Met-Glu-Asp-Leu-Met-Val-Phe-Ser-Arg-Thr-Gly-$NH_2$)

Using 60 mg of a carrier resin (NovaSyn TGR resin, manufactured by Nova Biochem) to which 12.6 μmol of Fmoc-NH was linked, as the starting material, the carrier resin was condensed with Fmoc-Gly-OH, Fmoc-Thr(tBu)-OH, Fmoc-Arg(Pmc)-OH, Fmoc-Ser(tBu)-OH, Fmoc-Phe-OH, Fmoc-Val-OH, Fmoc-Met-OH, Fmoc-Leu-OH, Fmoc-Asp(Ot-Bu)-OH, Fmoc-Glu(OtBu)-OH, Fmoc-Met-OH, Fmoc-Leu-OH, Fmoc-Lys(Boc)-OH, Fmoc-Ile-OH, Fmoc-Glu(OtBu)-OH, Fmoc-Met-OH, Fmoc-Leu-OH, Fmoc-Gly-OH, Fmoc-Glu(OtBu)-OH, Fmoc-Ser(tBu)-OH, Fmoc-Lys(Boc)-OH, Fmoc-Ser(tBu)-OH, Fmoc-Leu-OH, Fmoc-Arg(Pmc)-OH, Fmoc-Leu-OH, Fmoc-Phe-OH, Fmoc-Val-OH, Fmoc-Glu(Ot-Bu)-OH, Fmoc-Glu(OtBu)-OH, Fmoc-Arg(Pmc)-OH, Fmoc-Tyr(tBu)-OH, Fmoc-Ser(tBu)-OH, Fmoc-Leu-OH, Fmoc-Lys(Boc)-OH, Fmoc-Thr(tBu)-OH, Fmoc-His(Trt)-OH, Fmoc-Pro-OH, Fmoc-Ser(tBu)-OH and Fmoc-Leu-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 69.6 mg of crude peptide was obtained, dissolved in an aqueous acetic acid solution and purified by HPLC using a reverse phase column to obtain 13.2 mg of the Peptide 2S.
Mass spectrometry (TOFMS); m/z = 4569.8 (monoisotopic mass, $M+H^+$)
Amino acid analysis; Asx 0.9 (1), Glx 5.2 (5), Ser 5.0 (5), Gly 2.2 (2), His 1.0 (1), Arg 3.1 (3), Thr 2.1 (2), Pro 1.0 (1), Tyr 1.0 (1), Val 1.8 (2), Met 2.9 (3), Ile 0.8 (1), Leu 7.3 (7), Phe 1.8 (2), Lys 2.8 (3)

(6)Synthesis of Peptide 3S (SEQ ID NO:9: H-Val-Thr-Pro-His-Thr-Lys-Leu-Val-Tyr-Ser-Glu-Ser-Val-Ile-Met-Asn-Met-Ser-Lys-Gly-Glu-Gly-Leu-Met-Glu-Ile-Lys-Leu-Met-Asp-Asp-Met-Met-Thr-Phe-Arg-Lys-Val-Gly-$NH_2$)

Using 60 mg of a carrier resin (NovaSyn TGR resin, manufactured by Nova Biochem) to which 12.6 μmol of Fmoc-NH had been linked, as the starting material, the carrier resin was condensed with Fmoc-Gly-OH, Fmoc-Val-OH, Fmoc-Lys(Boc)-OH, Fmoc-Arg(Pmc)-OH, Fmoc-Phe-OH, Fmoc-Thr(tBu)-OH, Fmoc-Met-OH, Fmoc-Met-OH, Fmoc-Asp(OtBu)-OH, Fmoc-Asp(OtBu)-OH, Fmoc-Met-OH, Fmoc-Leu-OH, Fmoc-Lys(Boc)-OH, Fmoc-Ile-OH, Fmoc-Glu (OtBu)-OH, Fmoc-Met-OH, Fmoc-Leu-OH, Fmoc-Gly-OH, Fmoc-Glu(OtBu)-OH, Fmoc-Gly-OH, Fmoc-Lys(Boc)-OH, Fmoc-Ser(tBu)-OH, Fmoc-Met-OH, Fmoc-Asn(Trt)-OH, Fmoc-Met-OH, Fmoc-Ile-OH, Fmoc-Val-OH, Fmoc-Ser(tBu) -OH, Fmoc-Glu(OtBu)-OH, Fmoc-Ser(tBu)-OH, Fmoc-Tyr(tBu)-OH, Fmoc-Val-OH, Fmoc-Leu-OH, Fmoc-Lys(Boc) -OH, Fmoc-Thr(tBu)-OH, Fmoc-His(Trt)-OH, Fmoc-Pro-OH, Fmoc-Thr(tBu)-OH and Fmoc-Val-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 60.3 mg of crude peptide was obtained, dissolved in an aqueous acetic acid solution and purified by HPLC using a reverse phase column to give 8.7 mg of Peptide 3S.
Mass spectrometry (FABMS); m/z = 4445.6 (monoisotopic mass, $M+H^+$)
Amino acid analysis; Asx 3.1 (3), Glx 3.3 (3), Ser 3.0 (3), Gly 3.3 (3), His 1.0 (1), Arg 1.1 (1), Thr 2.8 (3), Pro 1.0 (1), Tyr 0.9 (1), Val 3.4 (4), Met 6.0 (6), Ile 1.6 (2), Leu 3.2 (3), Phe 1.1 (1), Lys 4.0 (4)

(7)Synthesis of Peptide 2 short (SEQ ID NO:10: H-Leu-Ile-Arg-Glu-Val-Ser-Ser-Gly-Ser-Glu-Glu-Thr-Phe-Ser-Tyr-Ser-Asp-Phe-Leu-Arg-Met-Met-Leu-$NH_2$)

Using 60 mg of a carrier resin (NovaSyn TGR resin, manufactured by Nova Biochem) to which 12.6 μmol of Fmoc-NH was linked, as the starting material, the carrier resin was condensed with Fmoc-Leu-OH, Fmoc-Met-OH, Fmoc-Met-OH, Fmoc-Arg(Pmc)-OH, Fmoc-Leu-OH, Fmoc-Phe-OH, Fmoc-Asp(OtBu)-OH, Fmoc-Ser(tBu)-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Glu(OtBu)-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Arg(Pmc)-OH, Fmoc-Ile-OH and Fmoc-Leu-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. In the same manner as in (1), 38.3 mg of crude peptide was obtained, dissolved in an aqueous acetic acid solution and purified by HPLC using a reverse phase column to obtain 11.7 mg of Peptide 2 short.
Mass spectrometry (FABMS); m/z = 2697.4 (monoisotopic mass, $M+H^+$)

Amino acid analysis; Asx 0.9 (1), Ser 4.9 (5), Glx 3.1 (3), Gly 1.1 (1), Arg 2.0 (2), Thr 0.9 (1), Tyr 1.0 (1), Val 1.1 (1), Met 2.1 (2), Ile 0.8 (1), Leu 3.0 (3), Phe 2.1 (2)

Example 2

Measurement of physiological activities of prepared peptides (specificity for organ):

In order to evaluate physiological activities of the peptides prepared in Example 1, the prepared peptides were allowed to act upon organs excised from the living body, and changes in the concentration of an intracellular signal transduction substance in cells constituting the organs were measured.

In order to measure changes in the concentration of an intracellular signal transduction substance in cells constituting the organs, a transgenic mouse prepared by integrated an apoaequorin gene expression vector into a chromosome was used. Specifically, a female mouse derived from the transgenic mouse disclosed in Reference Example 4 as an individual number 98075-04-6 was used in the test. The transgenic mouse was prepared and reared as an SPF animal in a clean room, and was not contaminated with pathogens including mouse hepatitis virus, Sendai virus, mycoplasma and Tyzzer bacterium.

Aequorin is a bioluminescent protein complex which is present in the umbrella region of luminescent *Aequorea victoria.* Aequorin is constituted from its protein moiety apoaequorin, its luminescence substrate coelenterazine and molecular oxygen, and emits light by its binding with calcium ion. Since expression of the apoaequorin protein has been confirmed in at least thymus, spleen, abdominal muscle, diaphragm, kidney, crystalline lens, pancreas, fat tissue, liver, skull, blood vessel, ovary, womb and adrenal gland of the transgenic mouse used in the test, it was able to monitor changes in the intracellular calcium ion concentration in these organs with a high sensitivity.

Organs were prepared from offsprings borne from the female of individual number 98075-04-6 to examine the activities of the peptides produced in Example 1. After the offsprings were killed, the thymus, spleen, abdominal muscle, diaphragm, kidney, crystalline lens, pancreas, fat tissue, liver, skull, blood vessel, ovary, womb and adrenal gland were excised. Each of the excised organs was cut into small cubes of about 1 to 2 mm square in PBS containing 10 units/ml of heparin sodium and thoroughly washed by changing the solution. Next, three pieces of each of the organ samples thus prepared were put into a 5 ml capacity tube (Rohren-Tubes; No. 55.476, manufactured by Sarstedt) which had been charged with RPMI 1640 medium containing 200 μl of 10 μmol/l coelenterazine (manufactured by Molecular Probes), and cultured at 37°C for 5 hours. Five hours after, various substances shown below dissolved in RPMI 1640 medium were added at 200 μl, and the luminescence was measured just after the addition at 1 second intervals using a luminometer AutoLumat LB953 (manufactured by BERTHOLD).

Fig. 1 shows a result of adding RPMI 1640, angiotensin II (final concentration: 1 μmol/l), bradykinin (final concentration: 10 μmol/l), A23187 (final concentration: 1 μmol/l) and Triton X-100 (final concentration: 2%) to each tissue of the adrenal grand, womb, blood vessel and spleen.

Fig. 2 shows a result of adding RPMI 1640, endothelin (final concentration: 1 nmol/l), calcitonin (final concentration: 10 nmol/l), A23187 (final concentration: 1 μmol/l) and Triton X-100 (final concentration: 2%) to each tissue of the blood vessel and heart.

Fig. 3 shows a result of adding RPMI 1640, carbachol (final concentration: 20 μmol/l), α-methylserotonin (final concentration: 20 μmol/l), ATP (final concentration: 100 μmol/l), phenylephrine (final concentration: 20 μmol/l), A23187 (final concentration: 1 μmol/l) and Triton X-100 (final concentration: 2%) to each tissue of the thymus, blood vessel and spleen.

Fig. 4 to Fig. 9 and Fig. 10 to Fig. 18 show respective results of adding RPMI 1640, peptides produced in Example 1 (final concentration: 10 μmol/l), ATP (final concentration: 100 μmol/l), A23187 (final concentration: 1 μmol/l) and Triton X-100 (final concentration: 2%) to the spleen, skull and thymus.

Also, the angiotensin II (product code: A-151), bradykinin (product code: B-120), ATP (product code: A-141), A23187 (product code: C-161), endothelin (product code: E-134), calcitonin (product code: C-210), carbachol (product code: C-107), α-methylserotonin (product code: M-110) and phenylephrine (product code: A-133) used in the experiment were purchased from Research Biochemicals International.

As a result, strong luminescence was observed in the blood vessel, womb and adrenal gland when angiotensin II was added to give a final concentration of 1 μmol/l (Fig. 1). Strong luminescence was observed in the blood vessel, womb and adrenal grand when bradykinin was added to give a final concentration of 10 μmol/l (Fig. 1). Strong luminescence was observed in the blood vessel when endothelin was added to give a final concentration of 10 nmol/l (Fig. 2). Strong luminescence was observed in the thymus and blood vessel when phenylephrine which was an agonist of adrenaline α1 receptor was added to give a final concentration of 20 μmol/l (Fig. 3). Strong luminescence was observed in the blood vessel when carbachol which was a muscarine receptor agonist was added to give a final concentration of 10 μmol/l (Fig. 3). Strong luminescence was observed in the thymus, blood vessel and spleen when ATP which was a P2Y receptor agonist was added to give a final concentration of 100 μmol/l (Fig. 3). Strong luminescence was observed in the spleen and skull when Peptide 1, Peptide 2 or Peptide 3 was added to give a final concentration of 10 μmol/l (Fig. 4, Fig. 5, Fig. 6, Fig. 10, Fig. 11, Fig. 12). On the other hand, such an activity was not detected by Peptides 1S, 2S and 3S produced as control peptides of the peptides (Fig. 7, Fig. 8, Fig. 9, Fig. 13, Fig. 14, Fig. 15). In organs other than the spleen and skull, namely the thymus, abdominal muscle, diaphragm, kidney, crystalline lens, pancreas, fat tissue, liver, blood vessel, ovary, womb and adrenal gland, the luminescence was not observed when any one of the seven peptides produced in Example 1 was added, and a phenomenon such as increase in the concentration of intracellular calcium ion was not observed. However, the luminescence was observed even in the organs when ATP, A23187 or Triton X-100 was added. As a typical example of the measured results, the results measured using thymus are shown in Fig. 16, Fig. 17 and Fig. 18.

Based on the above, it was found that Peptide 1, Peptide 2 and Peptide 3 have the activity to organ-specifically increase the concentration of an intracellular calcium ion.

Example 3

Measurement of physiological activities of prepared peptides (concentration-dependency):

Organs were prepared from offsprings borne from the female of individual number 98075-04-6 to examine the activities of the peptides produced in Example 1. After the offsprings were killed, the spleen was excised. The excised spleen was cut into small cubes of about 1 to 2 mm square in PBS containing 10 units/ml of heparin sodium and thoroughly washed by changing the solution. Next, three pieces of the prepared spleen sample were put into a 5 ml capacity tube (Rohren-Tubes; No. 55.476, manufactured by Sarstedt) which had been charged with RPMI 1640 medium containing 200 μl of 10 μmol/l coelenterazine (manufactured by Molecular Probes), and cultured at 37°C for 5 hours. Five hours after, various substances shown below dissolved in RPMI 1640 medium were added at 200 μl, and the luminescence was measured just after the addition at 1 second intervals using a luminometer AutoLumat LB953 (manufactured by BERTHOLD).

RPMI 1640, a peptide prepared in Example 1 (final concentration: 10 nmol/l), a peptide prepared in Example 1 (final concentration: 100 nmol/l), a peptide prepared in Example 1 (final concentration: 1 μmol/l), a peptide prepared in Example 1 (final concentration: 10 μmol/l), ATP (final concentration: 100 μmol/l), A23187 (final concentration: 1 μmol/l) and Triton X-100 (final concentration: 2%) were added in order. Fig. 19, Fig. 20, Fig. 21 and Fig. 22 show results of the measurement on Peptides 1, 2 and 3 and Peptide 2 short, respectively.

Concentration-dependent luminescence was observed from a final concentration of 100 nmol/l in Peptide 2, and from a final concentration of 1 μmol/l in Peptide 3 and Peptide 2 short (Fig. 20, Fig. 21 and Fig. 22). In Peptide 1, luminescence accompanied by the increased intracellular calcium was observed at 10 μmol/l (Fig. 19). Peptide 2 short consisting of 23 amino acids as a C-terminal partial sequence of Peptide 2 maintained about 1/10 of the activity of Peptide 2 consisting of 39 amino acids by the C-terminal moiety alone.

Example 4

Analysis of genes encoding the prepared physiologically active peptides:

A protein amino acid sequence data base SWISSPROP or a nucleotide sequence data base GenBank were searched for the amino acid sequences of produced Peptides 1, 2 and 3. As a result, it was found that the genes registered in NCBI (National Center for Biotechnology Information), USA, as CAA75060 (Y14768) (gi: 3805801), U95213 (gi: 2735480), Hs.169196, U19713 (gi: 1122908) and HS.76364 contained the same amino acid sequence of Peptide 1, the gene registered as AAC82481 (AF109719) (gi: 3941738) contained the same amino acid sequence of Peptide 2, and the genes registered as HS.32807 and AA016714 (gi: 1478945) contained the same amino acid sequence of Peptide 3. By a homology analysis using BLAST2, it was further found that P55009 (gi: 1703218), C88427 (gi: 2924709) and P81076 (gi: 3023279) exist as genes which show significant homology with the genes which were found as a result of the above search. Proteins encoded by these genes were human AIF-1 [U19713 (gi: 1122908), HS.76364], G1 as another splice form of AIF-1 [CAA75060 (Y14768) (gi: 3805801)], IRT-1 as still another splice form of AIF-1 [U95213 (gi: 2735480), Hs.169196], mouse AIF-1 [AAC82481 (AF109719) (gi: 3941738)], human and muse AIF-1 homologue [HS.32807, AAO16714 (gi: 1478945)], rat AIF [P55009 (gi: 1703218)], carp AIF [C88427 (gi: 2924709)] and swine AIF [P81076 (gi: 3023279)], and all of them belonged to the same family. When amino acid sequences of the proteins encoded by these genes are analyzed, basic amino acids such as lysine and arginine are present in the N-terminal moiety of a region corresponding to Peptide 1, 2 or 3, and amino acids such as glycine, lysine and arginine are present in the C-terminal moiety. Such a sequence is a sequence frequently found in nuclear proteins such as a transcription factor, and precursors of physiologically active peptides. Actually, 10,337 sequences of genes containing a sequence in which amino acids are aligned in 2 basis amino acids, amino acids of 10 to 40 residues, glycine, 2 basic amino acids in that order have been registered in NCBI, USA, at the time of December, 1999, so that it can be understood that such a sequence is contained in proteins having really various functions. However, since the present invention has revealed in Examples 2 and 3 that Peptides 1, 2 and 3 have a physiological activity to change the intracellular calcium concentration, it was found that each of the genes of CAA75060 (Y14768) (gi: 3805801), U95213 (gi: 2735480), Hs.169196, U19713 (gi: 1122908), HS.76364, AAC82481 (AF109719) (gi: 3941738), HS.32807, AA016714 (gi: 1478945), P55009 (gi: 1703218) and C88427 (gi: 2924709) obtained by the data base analysis functions as a precursor protein of the physiologically active peptide of the present invention.

Example 5

Preparation of antigen peptides for preparing antibodies against the produced physiologically active peptides:

A peptide consisting of the amino acid sequence represented by SEQ ID NO:23, 24, 25, 26 or 27 was produced. Herein, the produced peptides represented by SEQ ID NOs:23, 25, 26 and 27 are called Peptide 3C, Peptide 1C23, Peptide 1C and Peptide 2C, respectively. Also, the peptide consisting of the amino acid sequence represented by SEQ ID NO:24 is called Peptide 3C15. The peptides were used as antigen peptides for producing antibodies in experiments after Example 6.

Peptide 3C is a peptide in which a cysteine residue was added to the N-terminus of Peptide 3 produced in Example 1, and Peptide 1C is a peptide in which a cysteine residue was added to the N-terminus of Peptide 1 produced in Example 1. Peptide 1C23 is a peptide in which a cysteine residue was added to the N-terminus of a partial peptide consisting of 23 amino acid residues at the C-terminal of Peptide 1 produced in Example 1. Peptide 3C15 is a peptide in which a cysteine residue was added to the C-terminus of a partial peptide consisting of 15 amino acid residues at the N-terminal of Peptide 3 produced in Example

1. Peptide 2C is a peptide in which a cysteine residue was added to the N-terminus of Peptide 2 produced in Example 1.

The abbreviations of the amino acids and their protecting groups used in the synthesis were based on the recommendation of IUPAC-IUB Joint Commission on Biochemical Nomenclature [*European Journal of Biochemistry*, 138, 9 (1984)]. Specific abbreviations were used according to those described in Example 1. Also, the physicochemical properties of the produced peptides ware determined in a manner similar to the methods in Example 1.

1)Synthesis of Peptide 3C (SEQ ID NO: 23; H-Cys-Met-Met-Glu-Lys-Leu-Gly-Val-Pro-Lys-Thr-His-Leu-Glu-Met-Lys-Lys-Met-Ile-Ser-Glu-Val-Thr-Gly-Gly-Val-Ser-Asp-Thr-Ile-Ser-Tyr-Arg-Asp-Phe-Val-Asn-Met-Met-Leu-$NH_2$)

Into a reaction vessel of an automatic synthesizer (manufactured by Shimadzu Corporation), 60 mg of a carrier resin (NovaSyn® TGR resin, manufactured by Nova Biochem) to which 13 μmol of $NH_2$ was linked was put, 600 μl of DMF was added thereto, followed by stirring for 3 minutes, the solvent was removed, and the following steps were carried out according to the synthesis program of Shimadzu Corporation.

(a) To the carrier resin, 500 μl of 30% piperidine-DMF solution was added, the mixture was stirred for 4 minutes, the solvent was removed, and this step was repeated once again.
(b) The carrier resin was washed with 600 μl of DMF for 1 minute, the solvent was removed, and this step was repeated 5 times.
(c) Fmoc-Leu-OH (126 μmol), HBTU (126 μmol), HOBt monohydrate (126 μmol) and DIEA (252 μmol) were mixed in DMF (660 μl), the resulting solution was added to the resin, which was stirred for 60 minutes, and the solvent was removed.
(d) The carrier resin was washed with 600 μl of DMF for 1 minute, the solvent was removed, and this step was repeated 5 times.

In this manner, Fmoc-Leu-NH was synthesized on the carrier.
Next, after the steps of (a) and (b), condensation reaction was carried out using Fmoc-Met-OH in the step of (c), and Fmoc-Met-Leu-NH was synthesized on the carrier via the washing step of (d).
Subsequently, (a) to (d) were repeated using Fmoc-Met-OH, Fmoc-Asn(Trt)-OH, Fmoc-Val-OH, Fmoc-Phe-OH, Fmoc-Asp(OtBu)-OH, Fmoc-Arg(Pmc)-OH, Fmoc-Tyr(tBu)-OH, Fmoc-Ser(tBu)-OH, Fmoc-Ile-OH, Fmoc-Thr(tBu)-OH, Fmoc-Asp(OtBu)-OH, Fmoc-Ser(tBu)-OH, Fmoc-Val-OH, Fmoc-Gly-OH, Fmoc-Gly-OH, Fmoc-Thr(tBu)-OH, Fmoc-Val-OH, Fmoc-Glu(OtBu)-OH, Fmoc-Ser(tBu)-OH, Fmoc-Ile-OH, Fmoc-Met-OH, Fmoc-Lys(Boc)-OH, Fmoc-Lys(Boc)-OH, Fmoc-Met-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Met-OH, Fmoc-Met-OH and Fmoc-Cys(Trt)-OH in this order in the step (c), the deprotection and washing steps of (a) and (b) were carried out, followed by drying for 12 hours under a reduced pressure to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. The resin was filtered, about 10 ml of ether was added to the resulting solution, and the resulting precipitate was recovered by centrifugation and decantation to give 57.3 mg of the product as a crude peptide. A total amount of the crude product was dissolved in an aqueous acetic acid solution and purified by HPLC using a reverse phase column (manufactured by SHISEIDO, CAPCELL PAK C18, 30 mm I.D. × 250 mm). The elution was carried out by a linear density gradient method in which an aqueous solution of 90% acetonitrile containing 0.1% TFA was added to an aqueous solution of 0.1% TFA, and detected at 220 nm to give a fraction containing Peptide 3C. The fraction was freeze-dried to give 8.4 mg of Peptide 3C.
Mass spectrometry (FABMS); m/z = 4548.8 $(M+H^+)$
Amino acid analysis; Asx 3.1 (3), Glx 3.1 (3), Ser 2.9 (3), Gly 3.0 (3), His 1.1 (1), Arg 1.0 (1), Thr 2.9 (3), Pro 1.0 (1), Tyr 1.1 (1), Val 3.9 (4), Met 6.0 (6), Ile 2.0 (2), Leu 3.0 (3), Phe 1.0 (1), Lys 4.0 (4), Cys 1.2 (1)

(2)Synthesis of Peptide 3C15 (SEQ ID NO:24: H-Met-Met-Glu-Lys-Leu-Gly-Val-Pro-Lys-Thr-His-Leu-Glu-Met-Lys-Cys-OH)

Using 60 mg of a carrier resin (H-Cys(Trt)-2-ClTrt resin, manufactured by Nova Biochem) to which 34.2 μmol of H-Cys(Trt) was linked, as the starting material, the carrier resin was condensed with Fmoc-Lys(Boc)-OH, Fmoc-Met-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Pmoc-Leu-OH, Fmoc-Lys(Boc)-OH, Fmoc-Glu(OtBu)-OH, Fmoc-Met-OH and Fmoc-Met-OH in this order in the same manner as in (1), and the Fmoc group was removed, followed by washing and drying to give the carrier resin to which a side chain protected peptide was linked. To the carrier resin, 1 ml of a mixed solution consisting of TFA (90%), thioanisole (5%) and 1,2-ethanedithiol (5%) was added and allowed to stand at room temperature for 2 hours to remove the side chain protecting group and cut out the peptide from the resin. The resin was filtered, about 10 ml of ether was added to the resulting solution, and the resulting precipitate was recovered by centrifugation and decantation to give 38.2 mg of the product as a crude peptide. The crude product was dissolved in an aqueous acetic acid solution, applied to a reverse phase silica gel packed cartridge (YMC Dispo SPE C18) to adsorb the peptide onto, washed with an aqueous solution of 0.1% TFA and 10% acetonitrile and then eluted with an aqueous solution of 0.1% TFA and 10% acetonitrile to obtain a fraction containing Peptide 3C15. The fraction was freeze-dried to give 27.5 mg of Peptide 3C15 was obtained.
Mass spectrometry (TOFMS); m/z = 1874.6 $(M+H^+)$
Amino acid analysis; Glx 2.1 (2), Gly 0.9 (1), His 1.2 (1), Thr 0.9 (1), Pro 0.9 (1), Val 0.9 (1), Met 3.1 (3), Leu 2.1 (2), Lys 2.9 (3), Cys 1.8 (1)

(3)Synthesis of Peptide 1C23 (SEQ ID NO:25: H-Cys-Leu-Ile-Gly-Glu-Val-Ser-Ser-Gly-Ser-Gly-Glu-Thr-Phe-Ser-Tyr-Pro-Asp-Phe-Leu-Arg-Met-Met-Leu-$NH_2$)

Using 30 mg of a carrier resin (Rink amide MBHA resin, manufactured by Nova Biochem) to which 16.5 μmol of Fmoc-NH was linked, as the starting material, the carrier resin was condensed with Fmoc-Leu-OH, Fmoc-Met-OH, Fmoc-Met-OH, Fmoc-Arg(Pmc)-OH, Fmoc-Leu-OH, Fmoc-Phe-OH, Fmoc-Asp(OtBu)-OH, Fmoc-Pro-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Gly-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Gly-OH, Fmoc-Ile-OH, Fmoc-Leu-OH and Fmoc-Cys(Trt)-OH in this order in the same manner as in (1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. The resin was filtered, about 10 ml of ether was added to the resulting solution, and the resulting precipitate was recovered by centrifugation and decantation to give 48.9 mg of the product as a crude peptide. A total amount of the crude product was dissolved in an aqueous acetic acid solution and purified by HPLC using a reverse phase column (manufactured by SHISEIDO, CAPCELL PAK C18, 30 mm I.D. × 250 mm). Elution was carried out by a linear density gradient method in which an aqueous solution of 90% acetonitrile containing 0.1% TFA was added to an aqueous solution of 0.1% TFA, and detected at 220 nm to give a fraction containing Peptide 1C23. The fraction was freeze-dried to give 11.6 mg of Peptide 1C23.
Mass spectrometry (TOFMS); m/z = 2639.4 $(M+H^+)$

Amino acid analysis; Asx 0.9 (1), Glx 1.8 (2), Ser 3.7 (4), Gly 3.2 (3), Arg 1.0 (1), Thr 1.0 (1), Pro 1.1 (1), Tyr 1.0 (1), Val 1.0 (1), Met 2.1 (2), Ile 1.0 (1), Leu 3.1 (3), Phe 2.0 (2), Cys 1.2 (1)

(4)Synthesis of Peptide 1C (SEQ ID NO:26: H-Cys-Met-Leu-Glu-Lys-Leu-Gly-Val-Pro-Lys-Thr-His-Leu-Glu-Leu-Lys-Lys-Leu-Ile-Gly-Glu-Val-Ser-Ser-Gly-Ser-Gly-Glu-Thr-Phe-Ser-Tyr-Pro-Asp-Phe-Leu-Arg-Met-Met-Leu-$NH_2$)

Using 59.5 mg of a carrier resin (NovaSyn® TGR resin, manufactured by Nova Biochem) to which 12.5 μmol of $NH_2$ was linked, as the starting material, the carrier resin was condensed with Fmoc-Leu-OH, Fmoc-Met-OH, Fmoc-Met-OH, Fmoc-Arg(Pmc)-OH, Fmoc-Leu-OH, Fmoc-Phe-OH, Fmoc-Asp(OtBu)-OH, Fmoc-Pro-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Gly-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Gly-OH, Fmoc-Ile-OH, Fmoc-Leu-OH, Fmoc-Lys(Boc)-OH, Fmoc-Lys(Boc)-OH, Fmoc-Leu-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Leu-OH, Fmoc-Met-OH and Fmoc-Cys(Trt)-OH in this order in the same manner as in Example 5(1), and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added and allowed to stand at room temperature for 8 hours to remove the side chain protecting group and cut out the peptide from the resin. The resin was filtered, about 10 ml of ether was added to the resulting solution, and the resulting precipitate was recovered by centrifugation and decantation to give 54.2 mg of the product as a crude peptide. A total amount of the crude product was dissolved in an aqueous acetic acid solution and purified by HPLC using a reverse phase column (manufactured by SHISEIDO, CAPCELL PAK C18, 30 mm I.D. × 250 mm). Elution was carried out by a linear density gradient method in which an aqueous solution of 90% acetonitrile containing 0.1% TFA was added to an aqueous solution of 0.1% TFA, and detected at 220 nm to give a fraction containing Peptide 1C. The fraction was freeze-dried to give 8.7 mg of Peptide 1C.
Mass spectrometry (TOFMS); m/z = 4483.7 ($M+H^+$)
Amino acid analysis; Asx 1.0 (1), Glx 4.0 (4), Ser 3.8 (4), Gly 4.1 (4), His 1.0 (1), Arg 1.1 (1), Thr 2.0 (2), Pro 2.1 (2), Tyr 1.1 (1), Val 2.0 (2), Met 3.1 (3), Ile 0.9 (1), Leu 6.9 (7), Phe 2.1 (2), Lys 3.8 (4), Cys 1.2 (1)

(5)Synthesis of Peptide 2C (SEQ ID NO:27: H-Cys-Met-Leu-Glu-Lys-Leu-Gly-Val-Pro-Lys-Thr-His-Leu-Glu-Leu-Lys-Lys-Leu-Ile-Arg-Glu-Val-Ser-Ser-Gly-Ser-Glu-Glu-Thr-Phe-Ser-Tyr-Ser-Asp-Phe-Leu-Arg-Met-Met-Leu-$NH_2$)

Using 60 mg of a carrier resin (NovaSyn® TGR resin, manufactured by Nova Biochem) to which 12.6 μmol of $NH_2$ had been linked, as the starting material, the carrier resin was condensed with Fmoc-Leu-OH, Fmoc-Met-OH, Fmoc-Met-OH, Fmoc-Arg(Pmc)-OH, Fmoc-Leu-OH, Fmoc-Phe-OH, Fmoc-Asp(OtBu)-OH, Fmoc-Ser(tBu)-OH, Fmoc-Tyr(tBu)-OH, Fmoc-Ser(tBu)-OH, Fmoc-Phe-OH, Fmoc-Thr(tBu)-OH, Fmoc-Glu(OtBu)-OH, Fmoc-Glu(OtBu)-OH, Fmoc-Ser(tBu)-OH, Fmoc-Gly-OH, Fmoc-Ser(tBu)-OH, Fmoc-Ser(tBu)-OH, Fmoc-Val-OH, Fmoc-Glu(OtBu)-OH, Fmoc-Arg(Pmc)-OH, Fmoc-Ile-OH, Fmoc-Leu-OH, Fmoc-Lys(Boc)-OH, Fmoc-Lys(Boc)-OH, Fmoc-Leu-OH, Fmoc-Glu(OtBu)-OH, Fmoc-Leu-OH, Fmoc-His(Trt)-OH, Fmoc-Thr(tBu)-OH, Fmoc-Lys(Boc)-OH, Fmoc-Pro-OH, Fmoc-Val-OH, Fmoc-Gly-OH, Fmoc-Leu-OH, Fmoc-Lys(Boc)-OH, Fmoc-Glu(OtBu)-OH, Fmoc-Leu-OH, Fmoc-Met-OH and Fmoc-Cys(Trt)-OH in this order in the same manner as in I-1, and the Fmoc group was removed, followed by washing and drying to give the carrier resin to which a side chain protected peptide was linked. To the carrier resin, 1 ml of a mixed solution consisting of TFA (82.5%), thioanisole (5%), water (5%), ethylmethyl sulfide (3%), 1,2-ethanedithiol (2.5%) and thiophenol (2%) was added, and allowed to stand at room temperature for 8 hours to remove the side chain protecting group and cut out the peptide from the resin. The resin was filtered, about 10 ml of ether was added to the resulting solution, and the resulting precipitate was recovered by centrifugation and decantation to give 58.6 mg of the product as a crude peptide. A total amount of the crude product was dissolved in an aqueous acetic acid solution and purified by HPLC using a reverse phase column (manufactured by SHISEIDO, CAPCELL PAK C18, 30 mm I.D. × 250 mm). Elution was carried out by a linear density gradient method in which an aqueous solution of 90% acetonitrile containing 0.1% TFA was added to an aqueous solution of 0.1% TFA, and detected at 220 nm to give a fraction containing Peptide 2C. The fraction was freeze-dried to give 5.9 mg of Peptide 2C.
Mass spectrometry (TOFMS); m/z = 4644.4 ($M+H^+$)
Amino acid analysis; Asx 1.0 (1), Glx 5.3 (5), Ser 4.9 (5), Gly 2.0 (2), His 1.1 (1), Arg 1.9 (1), Thr 1.9 (2), Pro 1.0 (1), Tyr 1.1 (1), Val 2.0 (2), Met 3.1 (3), Ile 0.9 (1), Leu 6.8 (7), Phe 2.0 (2), Lys 3.9 (4), Cys 1.2 (1)

Example 6

Production of antibodies for the produced physiologically active peptides:

(1) Preparation of immunogens

In order to increase immunogenicity of Peptide 3C, Peptide 3C15, Peptide 1C23 and Peptide 1C produced in Example 5, their conjugates with KLH (manufactured by Calbiochem) were produced by the following method and used as immunogens. Specifically, KLH was dissolved in PBS buffer and adjusted to 10 mg/ml, and 1/10 volume of 25 mg/ml MBS [N-(m-maleimidobenzoyl)succinimide; manufactured by Nakalai Tesque] was added dropwise thereto and allowed to react for 30 minutes under stirring. KLM-MBS (2.5 mg) obtained by removing free MBS using a Sephadex G-25 gel filtration column (manufactured by Amersham Pharmacia) which had been equilibrated in advance with PBS buffer was mixed with 1 mg of each peptide dissolved in a 0.1 M sodium phosphate buffer (pH 7.0) and allowed to react at room temperature for 3 hours under stirring. After the reaction, the mixture was dialyzed against PBS buffer and used as the immunogen.

(2) Immunization of animal and preparation of antibody-producing cells

To each of 5-week-old female BALB/c mice, 100 μg of each of the KLH conjugates of Peptide 3C, Peptide 3C15, Peptide 1C23 and Peptide 1C produced in the above (1) was administered together with 2 mg of aluminum hydroxide adjuvant (*Antibodies - A Laboratory Manual,* Cold Spring Harbor Laboratory, p. 99, 1988) and $1\times10^9$ cells of pertussis vaccine (manufactured by Chiba Serum Institute). After the administration for 2 weeks, 100 μg of each KLH conjugate was administered one a week and a total of 4 times. A blood sample was taken from the venous plexus of the fundus of the eyes of the mice, its serum antibody titer was examined by enzyme immunoassay shown below, and the spleen was excised from a mouse which showed a sufficient antibody titer 3 days after the final immunization.

The spleen was cut to pieces in MEM (minimum essential medium) (manufactured by Nissui Pharmaceutical), loosened with tweezers and then centrifuged (250 × g, 5 minutes). A tris-ammonium chloride buffer (pH 7.6) was added to the resulting precipitation fraction to treat erythrocytes for 1 to 2 minutes and remove them. The resulting precipitation fraction (cell fraction) was washed with MEM 3 times and used for cell fusion.

(3) Enzyme immunoassay (binding ELISA)

Each of the peptides obtained in Example 5 was conjugated with thyroglobulin (hereinafter referred to as "THY") and used as the antigen in the assay. The production method was as described in Example 6(1), except that SMCC [4-(maleimidomethyl)-cyclohexane-1-carboxylic acid N-hydroxysuccinimide ester; Sigma] was used as the crosslinking agent instead of MBS. The conjugate produced as described in the above (10 μg/ml) was dispensed at 50 μl/well into a 96 well EIA plate (manufactured by Greiner) and allowed to stand for adsorption at 4°C overnight. The plate was washed, and Dulbecco's phosphate buffer (phosphate buffered saline: PBS) containing 1% bovine serum albumin (hereinafter referred to as "PBS") was dispensed at 100 μl/well and allowed to stand at room temperature for 1 hour to block remaining active groups.

After standing for 1 hour, the PBS buffer containing 1% BSA (hereinafter referred to as "1% BSA/PBS") was discarded, an immunized mouse antiserum, a culture supernatant of a monoclonal antibody or a purified monoclonal antibody was dispensed into the plate at 50 μl/well and allowed to stand for 2 hours. The plate was washed with PBS buffer containing 0.05% polyoxyethylene (20) sorbitan monolaurate [(a product equivalent to Tween 20 available from ICI: manufactured by Wako Pure Chemical Industries)] (hereinafter referred to as "Tween-PBS"), and then a peroxidase-labeled rabbit anti-mouse immunoglobulin was dispensed at 50 μl/well and allowed to stand at room temperature for 1 hour. After the plate was washed with Tween-PBS, an ABTS substrate solution [2,2-azinobis(3-ethylbenzothiazole-6-sulfonic acid) ammonium salt, 1 mmol/l ABTS/0.1 mol/l citrate buffer (pH 4.2)] was added to develop color, and the absorbance at OD415 nm was measured using a plate reader (Emax, manufactured by Molecular Devices).

(4) Preparation of mouse myeloma cell

8-Azaguanine resistant mouse myeloma cell line P3X63Ag8U.1 (P3-U1: purchased from ATCC) was cultured in a normal medium (RPMI medium supplemented with 10% fetal calf serum (hereinafter referred to as "FCS")), $2\times10^7$ or more cells were ensured at the time of cell fusion and submitted as a parent cell line for use in the cell fusion.

(5) Production of hybridoma

The mouse spleen cells obtained in (2) and the myeloma cells obtained in (4) were mixed at a proportion of 10 : 1 and centrifuged (250 × g, 5 minutes). The cells of the resulting precipitation fraction were thoroughly disintegrated, a mixture of 2 g of polyethylene glycol-1000 (PEG-1000), 2 ml of MEM and 0.1 ml of dimethyl sulfoxide was added to the cells under stirring in an amount of 0.5 ml per $10^8$ mouse spleen cells at 37°C, 1 ml of MEM was added to the suspension several times at 1- to 2-minute intervals and then the total volume was adjusted to 50 ml by adding MEM thereto.

The suspension was centrifuged (900 rpm, 5 minutes), the cells of the resulting precipitation fraction were loosened gently, and the cells were suspended in 100 ml of HAT medium (prepared by adding HAT Media Supplement (manufactured by Boehringer Mannheim) to the 10% fetal bovine serum-added RPMI medium) gently by repeated drawing up into and discharging from a measuring pipette. The suspension was dispensed at 200 µl/well into a 96 well incubation plate and incubated in a 5% $CO_2$ incubator at 37°C for 10 to 14 days.

After the incubation, the culture supernatant was examined by the enzyme immunoassay described in (3), and a well which reacted with the antigen peptide but did not react with the control in which the antigen peptide was not coated was selected, and cloning by limiting dilution from the cells contained therein was repeated twice to establish hybridomas capable of producing antibodies for the physiologically active peptides of the present invention. As a result, hybridomas KM2947 and KM2946 capable of producing the monoclonal antibodies KM2947 and KM2946 were obtained using Peptide 3C as the antigen, a hybridoma KM2952 capable of producing the monoclonal antibody KM2952 was obtained using Peptide 3C15 as the antigen, a hybridoma KM3022 capable of producing the monoclonal antibody KM3022 was obtained using Peptide 1C23 as the antigen, and hybridomas KM3030 and KM3032 capable of producing the monoclonal antibodies KM3030 and KM3032 were obtained using Peptide 1C as the antigen.

Each of the antibodies showed specific reactivity for respective peptide used as the antigen. Fig. 23 shows a result of binding ELISA in a manner similar to the method described in (3) using KM2947, KM2952, KM3022 and KM3030 among the above antibodies.

Also, the hybridomas KM2947, KM2952, KM3022 and KM3030 were deposited on July 12, 2001 in International Patent Organism Depositary, National Institute of Advanced Industrial Science and Technology (Central 6, 1-1-1 Higashi, Tsukuba, Ibaraki, Japan: postal code 305-8566) as FERN BP-7656, FERM BP-7657, FERM BP-7658 and FERM BP-7659, respectively.

(6) Purification of monoclonal antibodies

Each of the hybridoma cell lines obtained in the above (5) was intraperitoneally injected into pristane-treated 8-week-old female nude mice (BALB/c) at a dose of 5 to $20 \times 10^6$ cells per mouse. Ten to twenty-one days after, the ascitic fluid was collected from each mouse filled with the ascitic fluid caused by ascites tumor by the hybridoma (1 to 8 ml/mouse).

The ascitic fluid was centrifuged (1,200 × g, 5 minutes) to remove a solid matter, and purified by the caprylic acid precipitation method according to a purified IgG monoclonal antibody (*Antibodies - A Laboratory Manual,* Cold Spring Harbor Laboratory, 1988). Subclasses of the monoclonal antibodies KM2947, KM2952, KM3022 and KM3030 were determined as IgG1, IgG3, IgG1 and IgG, respectively, by ELISA using a subclass typing kit.

Example 7

Detection of physiologically active peptides by Western blotting using the monoclonal antibody KM2952:

Each of Peptide 1, Peptide 3 and Peptide 3S produced in Example 1 was fractionated by electrophoresis using 15% SDS-polyacrylamide gel (manufactured by ATTO) in an amount of 100 ng/lane, and then blotted on a PVDF membrane (manufactured by Millipore) in a conventional way (*Antibodies - A Laboratory Manual,* Cold Spring Harbor, 1988). After the membrane was blocked with 1% BSA/PBS, the monoclonal antibody KM2952 produced in Example 6 was added at a concentration of 10 µg/ml and allowed to stand at room temperature for 1 hour. After standing for 1 hour, the membrane was washed with Tween-PBS and 2,000 folds-diluted peroxidase-labeled rabbit anti-mouse immunoglobulin (manufactured by DAKO) was added as the secondary antibody and allowed to stand at room temperature for 1 hour. After washing again with Tween-PBS, the peptides on the membrane were detected by exposing them to an X-ray film using an ECL Western detection reagent (manufactured by Amersham Pharmacia Biotech).

The results are shown in Fig. 24. Strong coloring was detected at positions of the molecules of interest on the lanes of electrophoresis of Peptide 1 and Peptide 3, but coloring was not observed on the lane of electrophoresis of Peptide 3S. Also, coloring was not observed when Peptide 1S was subjected to the electrophoresis in the same manner. Thus, it was found that KM2952 specifically recognizes Peptide 1 and Peptide 3.

Example 8

Detection of physiologically active peptides by binding ELISA using KM3022:

Each of Peptide 1C, Peptide 1C23, Peptide 2C, Peptide 3C and Peptide 3C15 prepared in Example 5 was dissolved in PBS buffer to give a concentration of 5 μg/ml. The peptide solution was dispensed at 50 μl/well into a 96 well EIA plate (manufactured by Greiner) and allowed to stand at 4°C overnight for adsorption onto the plate bottom. On the next day, the plate was washed with PBS, and 1% BSA/PBS was dispensed at 100 μl/well and allowed to stand at room temperature for 1 hour to block remaining active groups. After standing for 1 hour, 1% BSA/PBS was discarded, an antibody solution prepared by diluting the monoclonal antibody KM3022 prepared in Example 6 with PBS to give a concentration of 1 μg/ml was dispensed at 50 μl/well and allowed to stand for 2 hours. Two hours after, each well was washed with Tween-PBS, and a peroxidase-labeled rabbit anti-mouse immunoglobulin which had been diluted 400 folds with 1% BSA/PBS was dispensed at 50 μl/well and allowed to stand at room temperature for 1 hour. One hour after, the wells were subsequent washed with Tween-PBS, 1 mM ABTS substrate solution (ABTS [2,2'-azinobis(3-ethylbenzothiazoline-6-sulfonic acid) ammonium salt]/0.1 M citrate buffer (pH 4.2)) was added to analyze binding ability of the antigen peptide based on the coloring strength. The coloring as the absorbance at 415 nm was measured using a plate reader Emax (manufactured by Molecular Devices).

The results are shown in Fig. 25. Significant coloring was observed only in wells coated with Peptide 1C23 and Peptide 1C. Also, similar results were obtained when Peptide 1 prepared in Example 1 was used instead of Peptide 1C, and Peptide 3 prepared in Example 1 was used instead of Peptide 3C. That is, significant coloring was observed in the well coated with Peptide 1, but coloring was not observed in the well coated with Peptide 3. Based on the above, it was found that the monoclonal antibody KM3022 specifically recognizes Peptide 1.

Example 9

Influence of monoclonal antibodies on the activity of the peptide of the present invention:

Organs were prepared from offsprings borne from the female of individual number 98075-04-6 to examine effects of the monoclonal antibodies produced in Example 6 on the physiological actions of the peptides produced in Example 1.

After the offsprings borne from the female of individual number 98075-04-6, the spleen was excised. The excised spleen was cut into small cubes of about 1 to 2 mm square in PBS containing 10 units/ml of heparin sodium and thoroughly washed by changing the solution. Next, three pieces of the prepared spleen samples were put into a 5 ml capacity tube (Rohren-Tubes; No. 55.476, manufactured by Sarstedt) which had been charged with RPMI 1640 medium containing 200 μl of 10 μmol/l coelenterazine (manufactured by Molecular Probes), and cultured at 37°C for 5 hours. Five hours after, various substances shown below dissolved in RPMI 1640 medium were added at 200 μl, and the luminescence was measured just after the addition at 1 second intervals using a luminometer AutoLumat LB953 (manufactured by BERTHOLD).

RPMI 1640 medium, test samples containing the peptides produced in Example 1 and the antibodies produced in Example, and Triton X-100 (final concentration: 2%) were added in this order.

RPMI 1640 medium containing only each of the peptides produced in Example 1 was used as a positive control, and RPMI 1640 medium containing none of the peptides and antibodies was used as a negative control. Each of the monoclonal antibodies produced in Example 6 was added in an appropriate amount to the RPMI 1640 medium containing each of the peptides produced in Example 1 and allowed to react at room temperature for 30 minutes, and the resulting solutions were used as test samples.

Effect of the monoclonal antibody KM3030 on Peptide 1 is shown in Fig. 26. Strong luminescence was observed in the spleen when Peptide 1 was added to give a final concentration of 6 μmol/l, but significant luminescence was not observed in a test sample to which the KM3030 was added to give a final concentration of 1.8 mg/ml. Thus, it was found that the monoclonal antibody KM3030 is an antibody having the activity to neutralize physiological activity of Peptide 1.

Effect of the monoclonal antibody KM2947 on Peptide 3 is shown in Fig. 27. Strong luminescence was observed in the spleen when Peptide 3 was added to give a final concentration of 8 μmol/l, but significant luminescence was not observed in a test sample to which the KM2947 was added to give a final concentration of 2.0 mg/ml. Thus, it was found that the monoclonal antibody KM2947 is an antibody having the activity to neutralize physiological activity of Peptide 3.

Example 10

Measurement of physiological activities of the peptide of the present invention (measurement using spleen-derived cells):

Physiological activities of the peptides produced in Example 1 were measured using spleen cells.

After offsprings borne from the female of individual number 98075-04-6, were killed, the spleen was excised and washed with PBS containing 10 units/ml of heparin sodium, and the spleen was mashed by interposing and rubbing the spleen between the frost sides of two frost edge glasses (manufactured by Matsunami Glass) in RPMI 1640 medium. Unnecessary tissue fragments were removed from the cell suspension using a cell strainer (manufactured by Falcon, Catalogue No. 2350) and the number of cells was counted. The cells were suspended to give a cell density of $1 \times 10^7$ cells/100 μl in RPMI 1640 medium in which 10 μmol/l of coelenterazine (manufactured by Molecular Probes) had been dissolved, and cultured at 37°C for 5 hours in a 5 ml capacity tube (manufactured by Rohren-Tubes; manufactured by Sarstedt, No. 55.476). Five hours after, various substances dissolved in RPMI 1640 medium were added in 100 μl portions, and the luminescence was measured just after the addition at 1 second intervals using a luminometer AutoLumat LB953 (manufactured by BERTHOLD).

Fig. 28 shows results of the addition of RPMI 1640 medium, ATP (final concentration: 100 μmol/l), A23187 (final concentration: 1 μmol/l) or Triton X-100 (final concentration: 2%) to the splenocyte suspension prepared in the above.

Fig. 29 shows results of the addition of Peptide 1 (final concentration: 10 μmol/l), Peptide 2 (final concentration: 10 μmol/l) or Peptide 3 (final concentration: 10 μmol/l) to the splenocyte suspension prepared in the above.

When these peptides produced in Example 1 were added to the splenocyte suspension, significant luminescence was observed similar to the case of the test using tissue pieces. Thus it was found that these peptides have the activity to increase intracellular calcium concentration by acting upon individual cells constituting the spleen.

Example 11

Expression of precursor protein in *Escherichia coli*:

(1) Construction of plasmid expressing a fusion protein of AIF-1 or AIF-2 with maltose binding protein

AIF-1 is a precursor protein comprising the amino acid sequence represented by SEQ ID NO:1. Also, AIF-2 is a precursor protein comprising the amino acid sequence represented by SEQ ID NO:5. Amino acid sequences of AIF-1 and AIF-2 are shown in SEQ ID NOs:34 and 35, respectively.

A single-stranded cDNA was synthesized from human kidney-derived mRNA (manufactured by Clontech) using a kit (SUPERSCRIPT™ Preamplification System; manufactured by BRL). Oligo(dT) primers were used as the primers. The single-stranded cDNA was synthesized from 1 μg of the mRNA and diluted 250 folds with sterile distilled water. PCR was carried out using the single-stranded cDNA as the primer and using an oligonucleotide primer 5' A1 comprising the nucleotide sequence represented by SEQ ID NO:28 and an oligonucleotide primer 3' A1 comprising the nucleotide sequence represented by SEQ ID NO:29 to amplify a DNA fragment (hereinafter referred to as "A1 fragment") in which an *EcoRI* site was added to the 5'-terminal side of an AIF-1-encoding DNA (SEQ ID NO:32) and a HindIII site was added to the 3'-terminal side thereof. PCR was carried out by 30 cycles of a repeated reaction, each cycle consisting of denaturation at 94°C for 30 seconds, annealing at 55°C for 30 seconds and elongation at 72°C for 30 seconds, by using ExTaq buffer (manufactured by Takara Shuzo) containing the template DNA (final concentration: 1 ng/ml), primers (final concentration: 2.0 μmol/l for each), dNTP (final concentration: 0.25 mmol/l) and ExTaq polymerase (manufactured by Takara Shuzo) (final concentration: 125 mU/μl) as the reaction solution. The amplified A1 fragment was purified using GENECLEAN SPIN Kit (manufactured by Qbiogene).

Also, PCR was carried out using the single-stranded cDNA prepared in the above as the primer and using an oligonucleotide primer 5' A2 comprising the nucleotide sequence represented by SEQ ID NO:30 and an oligonucleotide primer 3' A2 comprising the nucleotide sequence represented by SEQ ID NO:31 to amplify a DNA fragment (hereinafter referred to as "A2 fragment") in which an *Eco*RI site was added to the 5'-terminal side of an AIF-2-encoding DNA (SEQ ID NO:33) and a *Hin*dIII site was added to the 3'-terminal side thereof. PCR was carried out by 30 cycles of a repeated reaction, one cycle consisting of denaturation at 94°C for 30 seconds, annealing at 55°C for 30 seconds and elongation at 72°C for 30 seconds, using ExTaq buffer (manufactured by Takara Shuzo) containing the template DNA (final concentration: 1 ng/ml), primers (final concentration: 2.0 μmol/l for each), dNTP (final concentration: 0.25 mmol/l) and ExTaq polymerase (manufactured by Takara Shuzo) (final concentration: 125 mU/μl) as the reaction solution. The amplified A2 fragment was purified using GENECLEAN SPIN Kit (manufactured by Qbiogene).

Plasmid pCR-A1 and plasmid pCR-A2 were prepared by ligating each of the purified A1 fragment and A2 fragment to pCR-TOPO vector using TOPO TA Cloning Kit (manufactured by Invitrogen). Nucleotide sequences of the A1 fragment and A2 fragment inserted into respective plasmids were determined using a DNA sequencer (ABI PRISM 377, manufactured by PE Biosystems) by carrying out the reaction using a DNA sequencing reagent (Dye Terminator Cycle Sequencing FS Ready Reaction Kit or BigDye Terminator Cycle Sequencing FS Ready Reaction Kit, manufactured by PE Biosystems) according to the manufacture's instructions to thereby confirm that mutation was not present.

A vector pMAL-p2X (manufactured by New England BioLabs) for expression of a fusion protein with a maltose binding protein (hereinafter referred to as "MBP") was digested with *Eco*RI and then 5'-terminal of the digestion site was dephosphorylated using a bacterial alkaline phosphatase. The resulting vector was ligated with 450 bp DNA fragments obtained by digesting each of pCR-A1 and pCR-A2 with *Eco*RI to thereby construct a vector pMALpAIF1 for expression of a fusion protein of MBP and AIF1 and to construct a vector pMALpAIF2 for expression of a fusion protein of MBP and AIF2 in *Escherichia coli.* Also, direction of the ligated DNA fragments was confirmed by digesting these prepared plasmids for expression with *Hin*dIII and comparing sizes of the digested DNA fragments.

The plasmid pMALpAIF1 and plasmid pMALpAIF2 was deposited on Jury 18, 2001, in International Patent Organism Depositary, National Instituted of Advanced Industrial Science and Technology (Central 6, 1-1-1 Higashi, Tsukuba, Ibaraki, Japan: postal code 305-8566) as FERM BP-7669 and FERM BP-7670, respectively.

The fusion protein of MBP and AIF1 and the fusion protein of MBP and AIF2 were expressed and purified as follows according to the instructions of New England BioLabs attached to the vector pMAL-p2X for fusion protein expression.

(2) Expression of fusion protein in *Escherichia coli*

Transformants JM109/pMALpAIF1 and JM109/pMALpAIF2 were obtained by introducing the plasmid pMALpAIF1 and plasmid pMALpAIF2, respectively, into *Escherichia coli* JM109 in a conventional way.

Each of JM109/pMALpAIF1 and JM109/pMALpAIF2 was inoculated into ampicillin-containing LB medium (1% Bacto-Tryptone, 0.5% yeast extract, 1% NaCl, 50 mg/l ampicillin, pH 7.2) and cultured at 37°C for overnight. Into 1 liter of the ampicillin-containing LB medium, 10 ml of the culture medium was inoculated, and cultured at 37°C while measuring the absorbance at 600 nm ($A_{600}$) of the culture medium with the lapse of time. When the culture medium reached $A_{600}$ = 0.5, isopropyl thiogalactoside (IPTG) was added to give a final concentration of 1 mmol/l for promoter induction, and the culturing was further continued at 37°C.

The cells (about 10 µl) cultured for 4 hours after the addition of IPTG was subjected to SDS-PAGE using 4-10% gradient gel (manufactured by Daiichi Pure Chemicals), and the MBP fusion protein in the cells was detected by Coomassie Brilliant Blue staining (Fig. 30, a). As a result, a band was detected at a position of about 55 kDa expected to be the molecular weight of the fusion protein of MBP and AIF-1, from two strains (clone Nos. 5 and 11) transformed with the plasmid pMALpAIF1. A band was detected at a position of about 55 kDa expected to be the molecular weight of the fusion protein of MBP and AIF-2, from three strains (clone Nos. 15, 16 and 24) transformed with the plasmid pMALpAIF2. The bands were not detected before the addition of IPTG but increased with the lapse of time until 3 hours after the addition of IPTG. Thus it was found that they are proteins formed and accumulated after the promoter induction by IPTG.

Also, SDS-PAGE samples of the transformant JM109/pMALpAIF1 and JM109/pMALpAIF2 prepared in the same manner were respectively subjected to Western blotting using an anti-MBP antibody according to the instructions of New England BioLabs (Fig. 30, b). As a result, a band at a position of about 55 kDa detected by the Coomassie Brilliant Blue staining was stained, and it was confirmed that the bands are MBP fusion proteins.

Furthermore, SDS-PAGE samples of the transformant JM109/pMALpAIF1 and JM109/pMALpAIF2 prepared in the same manner were respectively subjected to Western blotting using KM2946 in a manner similar to the Western blotting described in Example 7 (Fig. 30, c). As a result, a band at a position of about 55 kDa stained by the Coomassie Brilliant Blue staining and the Western blotting using the anti-MBP antibody was stained, and it was confirmed that the fusion protein of MBP and AIF-1 and the fusion protein of MBP and AIF-2 were expressed.

(3) Purification of the fusion protein of MBP and AIF-1 and the fusion protein of MBP and AIF-2 by affinity column.

After IPTG was added to each of the above JM109/pMALpAIF1 and JM109/pMALpAIF2, 250 ml of the culture medium obtained by further culturing at 37°C for 4 hours was centrifuged, and respective cells were recovered and preserved at -80°C.

The frozen cells were thawed, 30 ml of buffer A (20 mmol/l Tris-HCl pH 7.4, 0.2 mol/l NaCl and 1 mmol/l ethylenediaminetetraacetic acid) was added thereto, and 80% or more of the cells were disrupted. The cell disruption solution was centrifuged at 9,000 × g for 15 minutes, and the supernatant was separated and recovered. The supernatant was diluted 2-folds with buffer A and filtered through a filter of 0.45 µm, and the filtrate was subjected to the following affinity column chromatography.

A column packed with 5 ml of an amylose resin (manufactured by New England BioLabs) was equilibrated with 15 ml of buffer A and used as the affinity column. To the column, 30 ml of the above cell disrupt sample was applied to adhere the MBP fusion protein to the column. The column was washed by passing 40 ml of buffer A, and then 25 ml of buffer A containing 10 mmol/l maltose was passed to elute the MBP fusion protein. After collecting the eluate at 5 ml, each of the fractions, bypassed fractions obtained by passing the supernatant and a part of washed fractions (each 10 μl) were subjected to SDS-PAGE using a 4 to 10% gradient gel (manufactured by Daiichi Pure Chemicals), and the MBP fusion proteins in respective fractions were detected by carrying out Coomassie Brilliant Blue staining or Western blotting staining using an anti-MBP antibody. As a result, the fusion protein of MBP and AIF-1 (about 55 kDa) was detected in the first two fractions (equivalent to 10 ml elution) of the eluate. The fusion protein of MBP and AIF-2 (about 55 kDa) was also detected mostly in the first two fractions (equivalent to 10 ml elution) of the eluate.

Also, purity of crude purification sample of the fusion protein of MBP and AIF-1 and crude purification sample of the fusion protein of MBP and AIF-2 was estimated to be about 30% in both cases as the result of SDS-PAGE.

To 10 ml of the thawed crude purification sample was added 1.6 μg of blood coagulation factor $X_a$, which was subjected to reaction at 25°C according to the manual of New England BioLabs to cut a peptide between the MBP and AIF-1 or AIF-2 and isolate AIF-1 or AIF therefrom. Whether the peptide was cut or not was confirmed by subjecting a part of the reaction mixture (10 μl) to SDS-PAGE using 4 to 10% gradient gel (manufactured by Daiichi Pure Chemicals) and then staining the gel using KM2946 or KM3032 according to the Western blotting described in Example 7. As a result, it was observed that the peptide between MBP and the fusion protein was cut six hours after the reaction (Fig. 31).

Six hours after, AIF-1 and AIF-2 were purified by subjecting the above reacted samples to HPLC using a TSK-gel SW-3000 gel filtration column. Physiological activities of the purified samples were measured in a manner similar to the method described in Example 3, but the activity found in Peptide 1 or Peptide 3 was not observed in the purified AIF-1 and AIF-2.

Reference Example 1

Construction of apoaequorin expression plasmid:

(1) Construction of plasmid pAGE107-AEQ having apoaequorin gene in downstream of SV40 early promoter (cf. Fig. 32)

In 30 μl of Universal Buffer M (manufactured by Takara Shuzo), 2 μg of the plasmid pAGE107 (Japanese Published Unexamined Patent Application No. 22979/91, *Cytotechnology*, 3, 133 (1990)) was dissolved, and 10 units of *Xba*I and 10 units of *Sac*II were added thereto for the digestion at 37°C for 2 hours. After the reaction solution was subjected to agarose gel electrophoresis, a DNA fragment of about 4.6 Kb containing an ampicillin (Ap) resistance gene was recovered using QIAEX II Gel Extraction Kit (manufactured by QIAGEN) (hereinafter, this method is used for the recovery of DNA from agarose gel).

Also, 2 μg of the plasmid pAGE107 was dissolved in 30 μl of Universal Buffer H (manufactured by Takara Shuzo) and 10 units of *Pst*I and 10 units of *Sac*II were added thereto for the digestion at 37°C for 2 hours. After the reaction solution was subjected to agarose gel electrophoresis, a DNA fragment of about 1.7 Kb containing the poly (A) addition signal of rabbit β globulin was recovered.

Separately, 2 μg of a plasmid pMAQ2 (manufactured by Molecular Probes) was dissolved in 30 μl of Universal Buffer H (manufactured by Takara Shuzo), and 10 units of *Xba*I and 10 units of *PstI* were added thereto for the digestion at 37°C for 2 hours. After the reaction solution was subjected to agarose gel electrophoresis, a DNA fragment of about 0.63 Kb containing an apoaequorin gene was recovered.

In 30 μl of T4 ligase buffer, 0.1 μg of the *Xba*I-*Sac*II fragment (4.6 Kb) derived from the plasmid pAGE107, 0.1 μg of the *Pst*I-*Sac*II fragment (1.7 Kb) derived from the plasmid pAGE107 and 0.1 μg of the *Xba*I-*Pst*I fragment (0.63 Kb) derived from the plasmid pMAQ2, obtained in the above, were dissolved, and 100 units of T4 ligase were added therefor the ligation at 12°C for 16 hours.

*Escherichia coli* DH5 (manufactured by TOYOBO) was transformed using the reaction solution in a manner similar to the method of Cohen *et al. (Proc. Natl. Acad. Sci. USA,* 69, 2110, 1972) (hereinafter, this method is used for the transformation of *Escherichia coli)* to obtain an ampicillin resistant (Ampr) strain. A plasmid DNA was isolated from the transformant in a conventional way (*Nucleic Acids Res., 1*, 1513, 1979) (hereinafter, this method is used for the isolation of plasmid). Structure of the obtained plasmid was confirmed by restriction enzyme digestion. Hereinafter, the plasmid is called pAGE107-AEQ.

(2) Construction of plasmid pBSAEQpA having apoaequorin gene (cf. Fig. 33)

Primer 1 of 27 mer represented by SEQ ID NO:17 and Primer 2 of 28 mer represented by SEQ ID NO:18 were produced based on the nucleotide sequence of the apoaequorin gene cDNA described in Inoue *et al*. (*Proc. Natl. Acad. Sci. USA*, 82, 3154, 1985; Japanese Published Unexamined Patent Application No. 135586/86), a reaction solution was prepared in a conventional way using plasmid pAGE107-AEQ as the material, PCR was carried out by a reaction at 95°C for 5 minutes; subsequently 30 cycles, each cycle consisting of reactions at 95°C for 1 minute, 55°C for 2 minutes and 72°C for 4 minutes; and finally a reaction at 72°C for 10 minutes, and the reaction solution was preserved at 4°C overnight. The PCR reaction solution was subjected to phenol-chloroform extraction treatment and the amplified DNA fragment was recovered by ethanol precipitation. The recovered DNA fraction was dissolved in 30 µl of Universal Buffer H (manufactured by Takara Shuzo), and 10 units of *Xba*I and 10 units of *Eco*RI were added thereto for the digestion at 37°C for 2 hours. After the reaction solution was subjected to agarose gel electrophoresis, a DNA fragment of about 0.65 Kb containing the apoaequorin gene was recovered.

Next, 2 µg of a plasmid pRL-TK (manufactured by Promega) was dissolved in 30 µl of Universal Buffer H (manufactured by Takara Shuzo), and 10 units of *Xba*I and 10 units of *Bam*HI were added thereto for the digestion at 37°C for 2 hours. After the reaction solution was subjected to agarose gel electrophoresis, a DNA fragment of about 0.25 Kb containing the poly(A) addition signal of the SV40 late gene was recovered.

Furthermore, 2 µg of a plasmid pBluescript II SK(-) (manufactured by Stratagene) was dissolved in 30 µl of Universal Buffer H (manufactured by Takara Shuzo), and 10 units of *Eco*RI and 10 units of *Bam*HI were added thereto for the digestion at 37°C for 2 hours. After the reaction solution was subjected to agarose gel electrophoresis, a DNA fragment of about 2.9 Kb containing the ampicillin (Ap) resistance gene was recovered.

A 0.1 µg portion of the apoaequorin gene-containing DNA fragment (0.65 Kb), the *Xba*I-*Bam*HI fragment (0.25 Kb) derived from the plasmid pRL-TK and 0.1 µg of the *Eco*RI-*Bam*HI fragment (2.9 Kb) derived from the plasmid pBluescript II SK(-), obtained in the above, were dissolved in 30 µl of T4 ligase buffer, and 100 units of T4 DNA ligase were added thereto for the ligation at 12°C for 16 hours.

Using the reaction solution, *Escherichia coli* DH5 (manufactured by TOYOBO) was transformed in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Also, its nucleotide sequence was determined in a conventional way using DNS Sequencer 377 (manufactured by Perkin-Elmer) to confirm that the known apoaequorin cDNA sequence is contained in the plasmid. Hereinafter, the plasmid is called pBSAEQpA.

(3) Construction of plasmid pBSKS(+)CAG promoter having CAG promoter (cf. Fig. 34)

In 30 µl of Universal Buffer H (manufactured by Takara Shuzo), 2 µg of a cosmid pAxCAwt (Nucleic Acids Research, 23, 3818, 1995) was dissolved, and 10 units of *Sail* and 10 units of *Cal*I were added thereto for the digestion at 37°C for 2 hours. After the reaction solution was subjected to agarose gel electrophoresis, a DNA fragment of about 1.7 Kb containing the CAG promoter was recovered using QIAEX II Gel Extraction Kit (manufactured by QIAGEN).

Next, in 30 µl of Universal Buffer H (manufactured by Takara Shuzo), 2 µg of a plasmid pBluescript II KS(+) (manufactured by Stratagene) was dissolved, and 10 units of *Sal*I and 10 units of *Cal*I were added thereto for the digestion at 37°C for 2 hours. After the reaction solution was subjected to agarose gel electrophoresis, a DNA fragment of about 2.9 Kb containing the ampicillin (Ap) resistance gene was recovered using QIAEX II Gel Extraction Kit (manufactured by QIAGEN).

In 30 µl of T4 ligase buffer, 0.1 µg of the *Sal*I-*Cla*I fragment (1.7 Kb) derived from the cosmid pAxCAwt and 0.1 µg of the *Sal*I-*Cla*I fragment (2.9 Kb) derived from the plasmid pBluescript II SK(+), obtained in the above, were dissolved, and 100 units of T4 DNA ligase were added thereto for the ligation at 12°C for 16 hours.

*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called pBSKS(+)CAG promoter.

(4) Construction of plasmid pCAG-AEQ having apoaequorin gene in downstream of CAG promoter (cf. Fig. 35)

A 2 µg portion of plasmid pBSKS(+)CAG promoter was dissolved in 30 µl of Universal Buffer H (manufactured by Takara Shuzo) and mixed with 10 units of *Sail* and 10 units of *Eco*RI for the digestion at 37°C for 2 hours. After the reaction solution was subjected to agarose gel electrophoresis, a DNA fragment of about 1.7 Kb containing the CAG promoter was recovered using QIAEX II Gel Extraction Kit (manufactured by QIAGEN).

Next, in 30 µl of Universal Buffer H (manufactured by Takara Shuzo), 2 µg of pBSAEQpA was dissolved, and 10 units of *Sal*I and 10 units of *Eco*RI were added thereto for the digestion at 37°C for 2 hours. After the reaction solution was subjected to agarose gel electrophoresis, a DNA fragment of about 3.8 Kb containing the apoaequorin gene was recovered using QIAEX II Gel Extraction Kit (manufactured by QIAGEN).

In 30 μl of T4 ligase buffer, 0.1 μg of the *Sal*I-EcoRI fragment (1.7 Kb) derived from the plasmid pBSKS(+) CAG promoter and 0.1 μg of the *Sal*I-*Eco*RI fragment (3.8 Kb) derived from the plasmid pBSAEQpA, obtained in the above, were dissolved, and 100 units of T4 DNA ligase were added thereto for the ligation at 12°C for 16 hours.
*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called pCAG-AEQ.

(5) Construction of plasmid ploxp#1 (cf. Fig. 36)

In 30 μl of Universal Buffer M (manufactured by Takara Shuzo), 2 μg of a plasmid pKO (manufactured by Lexicon Genetics) was dissolved, and 10 units of *KpnI* and 10 units of *Xho*I were added thereto for the digestion at 37°C for 2 hours. After the reaction solution was subjected to agarose gel electrophoresis, a DNA fragment of about 1.97 Kb containing the ampicillin (Ap) resistance gene was recovered.
Next, a 44 mer single-stranded synthetic DNA loxPKpnI/XhoI-A represented by SEQ ID NO:19 containing the loxP sequence described in Araki *et al*. (*Nucleic Acids Res.,* 25, 868, 1997) and a 52 mer single-stranded synthetic DNA loxPKpnI/XhoI-B represented by SEQ ID NO:20 were produced and annealed to obtain a double-stranded DNA.
In 30 μl of T4 ligase buffer, 1.0 μg of the KpnI-*Xho*I fragment (1.97 Kb) derived from the plasmid pKO and 0.1 μg of the double-stranded DNA containing the loxP sequence comprising SEQ ID NOs:19 and 20, obtained in the above, were dissolved, and 100 units of T4 DNA ligase were added thereto for the ligation at 12°C for 16 hours.
*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called plox#1.

(6) Construction of plasmid ploxp (cf. Fig. 37)

In 30 μl of Universal Buffer H (manufactured by Takara Shuzo), 2 μg of the plasmid plox#1 was dissolved, and 10 units of *Bam*HI and 10 units of *Cla*I were added thereto for the digestion at 37°C for 2 hours. After the reaction solution was subjected to agarose gel electrophoresis, a DNA fragment of about 2.02 Kb containing the ampicillin (Ap) resistance gene was recovered.
Next, a 47 mer single-stranded synthetic DNA loxPBamHI/ClaI-A represented by SEQ ID NO:21 and a 45 mer single-stranded synthetic DNA loxPBamHI/ClaI-B represented by SEQ ID NO:22 were produced and annealed to obtain a double-stranded DNA.
In 30 μl of T4 ligase buffer, 1.0 μg of the *Bam*HI-*Cla*I fragment (2.02 Kb) derived from the plasmid ploxP#1 and 0.1 μg of the double-stranded DNA containing the loxP sequence comprising SEQ ID NOs:21 and 22, obtained in the above, were dissolved, and 100 units of T4 DNA ligase were added thereto for the ligation at 12°C for 16 hours.
*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called ploxp.

(7) Construction of plasmid ploxpHPRT (cf. Fig. 38)

In 30 μl of NEB Buffer 4 (manufactured by New England Biolabs), 2 μg of the plasmid ploxp was dissolved, and 10 units of *Asc*I were added thereto for the digestion at 37°C for 2 hours. After the reaction solution was subjected to agarose gel electrophoresis, a DNA fragment of about 2.07 Kb containing the ampicillin (Ap) resistance gene was recovered. The recovered DNA fragment was dissolved in 30 μl of an alkaline phosphatase buffer (manufactured by Takara Shuzo), and 44 units of bovine small intestine alkaline phosphatase (manufactured by Takara Shuzo) were added thereto for the reaction at 37°C for 30 minutes and, after phenol-chloroform extraction, about 2.07 Kb of dephosphorylated DNA fragment was recovered by ethanol precipitation.
Next, 2 μg of a plasmid pKOSelectHPRT (manufactured by Lexicon Genetics Inc.) was dissolved in 30 μl of NEB Buffer 4 (manufactured by New England Biolabs), and 10 units of *Asc*I were added thereto for the digestion at 37°C for 2 hours. After the reaction solution was subjected to agarose gel electrophoresis, a DNA fragment of about 3.8 Kb containing the hypoxanthine phosphoribosyltransferase (hprt) gene was recovered.
In 30 μl of T4 ligase buffer, 0.1 μg of the *Asc*I fragment (2.07 Kb) derived from the plasmid ploxp and 0.1 μg of the *AscI* fragment (3.8 Kb) derived from the plasmid pKOSelectHPRT, obtained in the above, were dissolved, and 100 units of T4 DNA ligase were added thereto for the ligation at 12°C for 16 hours.

*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called ploxpHPRT.

(8) Construction of plasmid ploxpHPRTnew (cf. Fig. 39)

In 30 μl of Universal Buffer K (manufactured by Takara Shuzo), 2 μg of the plasmid was dissolved, and 10 units of *HpaI* were added thereto for the digestion at 37°C for 2 hours. After the reaction solution was subjected to agarose gel electrophoresis, a DNA fragment of about 2.07 Kb containing the ampicillin (Ap) resistance gene was recovered. The recovered DNA fragment was dissolved in 30 μl of an alkaline phosphatase buffer (manufactured by Takara Shuzo), and 44 units of bovine small intestine alkaline phosphatase (manufactured by Takara Shuzo) was added thereto for the reaction at 37°C for 30 minutes and, after phenol-chloroform extraction, about 5.9 Kb of dephosphorylated DNA fragment was recovered by ethanol precipitation.

In 30 μl of T4 ligase buffer, 0.1 μg of the *Hpa*I fragment (5.9 Kb) derived from the plasmid ploxpHPRT, obtained in the above, and 0.1 μg of a synthetic linker pBamHI (8 mer) (5'-pd(CGGATCCG)-3'; manufactured by Takara Shuzo) were dissolved, and 100 units of T4 DNA ligase were added thereto for the ligation at 12°C for 16 hours.

*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called ploxpHPRTnew.

(9) Construction of plasmid pCAG-AEQ-pHPRTp having hprt gene in downstream of PGK promoter and also having apoaequorin gene connected to CAG promoter in downstream of CAG promoter (cf. Fig. 40)

In 30 μl of Universal Buffer L (manufactured by Takara Shuzo), 2 μg of the plasmid ploxpHPRTnew was dissolved, and 10 units of *Kpn*I were added thereto for the digestion at 37°C for 2 hours. Thereafter, 4 μl of 10-folds concentration Universal Buffer H (manufactured by Takara Shuzo) and 10 units of *Sal*I were added thereto to give a total volume of 40 μl, and the digestion was further carried out at 37°C for 2 hours. After the reaction solution was subjected to agarose gel electrophoresis, a DNA fragment of about 3.8 Kb containing the hprt gene was recovered.

Next, 2 μg of the plasmid pCAG-AEQ was dissolved in 30 μl of Universal Buffer L (manufactured by Takara Shuzo), and 10 units of *Kpn*I were added thereto for the digestion at 37°C for 2 hours. Thereafter, 4 μl of 10-folds concentration Universal Buffer H (manufactured by Takara Shuzo) and 10 units of *Sal*I were added thereto to give a total volume of 40 μl, and the digestion was further carried out at 37°C for 2 hours. After the reaction solution was subjected to agarose gel electrophoresis, a DNA fragment of about 5.5 Kb containing the ampicillin (Ap) resistance gene was recovered.

In 30 μl of T4 ligase buffer, 0.1 μg of the *Kpn*I-SalII fragment (3.8 Kb) derived from the plasmid ploxpHPRTnew and 0.1 μg of the *Kpn*I-*Sal*II fragment (5.5 Kb) derived from the plasmid pCAG-AEQ, obtained in the above, were dissolved, and 100 units of T4 DNA ligase were added thereto for the ligation at 12°C for 16 hours.

*Escherichia coli* DH5 (manufactured by TOYOBO) was transformed using the reaction solution in a manner similar to the method of Cohen *et al*. to obtain an ampicillin resistant (Ampr) strain. A plasmid DNA was isolated from the transformant in a conventional way. Structure of the obtained plasmid was confirmed by restriction enzyme digestion. Hereinafter, the plasmid is called pCAG-AEQ-pHPRTp.

Reference Example 2

Introduction of constructed apoaequorin expression plasmids into mammal cell and examination of transient expression quantity of apoaequorin:

The apoaequorin gene was transiently expressed by introducing the constructed various apoaequorin expression plasmids into a dihydrofolate reductase (dhfr) gene-defective Chinese hamster ovary cell CHO dhfr(-) DG44 (A.S. Kolhekar *et al., Biochemistry,* 36, 10901 (1997); G. Urlaub *et al., Cell,* 33, 405 (1983)), and comparison of the expressed amounts was carried out.

Apoaequorin expression plasmids were introduced into the CHO dhfr(-) cell DG44 according to the lipofection method. Specifically, using 2 ml of IMDM medium (manufactured by GIBCO BRL) containing 10% fetal bovine serum, the cell line was inoculated into a 6 well tissue culture microplate (manufactured by Iwaki Glass; code 3810-006) to give a density of $3\times10^5$ cells/well, cultured at 37°C in 5% $CO_2$ for 24 hours and washed several times with OPTI-MEM medium (manufactured by GIBCO BRL). OPTI-MEM medium (100 μl) containing 2 μg of each plasmid was gently

mixed with 100 μl of OPTI-MEM medium containing 6 μg of Lipofectoamine reagent (manufactured by GIBCO BRL) and allowed to react at room temperature for 30 minutes, and the total volume was adjusted to 1 ml using OPTI-MEM medium and added to the washed cells, followed by culturing for 4 hours. Four hours after, the gene was introduced by adding 1 ml of IMDM containing 20% fetal bovine serum, followed by further culturing for 24 hours.

Next, the gene-introduced cells were washed once with IMDM medium, and 1 ml of IMDM medium containing 10 μM of coelenterazine (manufactured by Molecular Probes) was added thereto, followed by culturing for 4 hours. Four hours after, the cells were peeled off using 0.05% trypsin solution, suspended in modified Krebs-Ringer buffer (20 mM HEPES pH 7.4 solution containing 125 mM NaCl, 5 mM KCl, 1 mM $MgSO_4$, 1 mM $CaCl_2$, 1 mM $Na_2HPO_4$ and 5.5 mM glucose) to give a density of $1\times10^6$/ml and then inoculated at 100 μl/well into a 96 well cell culture white plate (manufactured by Sumitomo Bakelite; product number MS-8096W). Using a microplate luminometer LB96P (manufactured by EG & G BERTHOLD), 100 μl of the modified Krebs-Ringer buffer containing 2 μM of a calcium ionophore A23187 (manufactured by Research Biochemical International) was added to each of the cell-inoculated wells and the luminescence was immediately measured at intervals of 3 seconds.

By the above method, plasmid pAGE107-AEQ, plasmid pCAG-AEQ and plasmid pBSAEQpA were introduced into the CHO dhfr(-) cell DG44, and comparison of promoter activities of respective plasmids was carried out using the luminescence of aequorin by A23187 stimulus as the index. The results are shown in Fig. 41. It was shown that the CAG promoter has the transcription activity higher than that of the promoter of SV40 early gene which is generally used for the expression in mammal cells.

Reference Example 3

Preparation of stable transformant by introducing the constructed apoaequorin expression plasmid into mammal cell and examination of apoaequorin expression quantity:

A stable transformant in which the plasmid pCAG-AEQ-pHPRTp was introduced into mouse myeloma cell P3.X63/Ag8.U1 (hereinafter referred to as "P3U1") (D.E. Yelton *et al., Curr. Top. Microbiol. Immunol.*, 81, 1, 1978) was obtained and a mutant cell line in which expression unit of the hprt gene was removed was obtained by transiently expressing Cre recombinase in the cell. Expressed amounts of apoaequorin before and after removal of the hprt expression unit were compared.

The plasmid pCAG-AEQ-pHPRTp was introduced into P3U1 cell according to electroporation. That is, the cell line was suspended in K-PBS buffer (mM KCl, 2.7 mM NaCl, 8.1 mM $Na_2HPO_4$, 1.5 mM $KH_2PO_4$, 4 mM $MgCl_2$) to give a density of $8\times10^6$ cells/ml, and 200 μl of the cell suspension and 4 μl of the plasmid pCAG-AEQ-pHPRTp adjusted to 1 μg/μl were mixed in a fusion chamber Gene Pulser Cuvette (inter-electrode distance 2 mm) (manufactured by BIORAD; catalog number 165-2086). Gene transfer was carried out using a cell fusion apparatus Gene Pulser (manufactured by BIORAD), under conditions of a pulse voltage of 0.25 KV and an electric capacity of 125 μF, and the cells were suspended in 10 ml of RPMI 1640 medium (manufactured by GIBCO BRL) containing 10% fetal bovine serum and cultured at 37°C for 24 hours in 5% $CO_2$. After the culturing for 24 hours, a drug-resistant cell line was obtained by adding 200 μl of HAT-Media Supplement (50 ×) (manufactured by Boehringer Mannheim Biochemica) and further culturing for 6 days. The number of viable cells of the obtained drug-resistant cell line was counted, and the cells were diluted to 5 cells/ml using RPMI 1640 medium containing 2% HT-Media Supplement (50 x) (manufactured by Boehringer Mannheim Biochemica) and 10% fetal bovine serum and then inoculated at 100 μl/well into a 96 well plate. One week after, cells were obtained from a well where a single colony was formed.

Using the Southern hybridization method described in Reference Example 4(3), copy numbers of the introduced gene were measured in a manner similar to the method described in *Embryo Engineering Experimentation Manual (Hassei Kogaku Jikken Manual)*, "Method for Preparing Transgenic Mouse (Transgenic Mouse No TsukuriKata)", Kodansha (1987), and a cell line into which one copy of the introduced gene had been inserted was selected. Hereinafter, this cell is called clone 21.

Next, cells of the clone 21 suspended in PBS buffer to give a density of $1.1\times10^7$ cells /ml and 25 μl of a plasmid pBS185 (manufactured by GIBCO BRL) adjusted to 1 μg/μl were mixed in a fusion chamber Gene Pulser Cuvette (inter-electrode distance 4 mm) (manufactured by BIORAD; catalog number 165-2088). Gene transfer was carried out using a cell fusion apparatus Gene Pulser (manufactured by BIORAD) under conditions of a pulse voltage of 0.23 KV and an electric capacity of 500 μF, and the cells were suspended in 10 ml of RPMI 1640 medium (manufactured by GIBCO BRL) containing 10% fetal bovine serum and cultured at 37°C for 24 hours in 5% $CO_2$. After the culturing for 24 hours, a drug-resistant cell line was obtained by adding 6-thioguanine (2-amino-6-mercaptopurine; manufactured by Sigma) to a concentration of 10 μM and culturing for 6 days. Hereinafter, this drug-resistant cell line is called clone 21Δhprt.

The obtained cell line was suspended in RPMI 1640 medium containing 10 μM of coelenterazine to give a density of $1\times10^6$ cells/ml and cultured for 4 hours. Four hours after, the cells were recovered by centrifugation at 1,000 × g for 5 minutes, washed twice using modified Krebs-Ringer buffer, suspended in the modified Krebs-Ringer buffer to give a density of $1\times10^6$ cells/ml and then inoculated at 100 μl/well into a 96 well cell culture white plate (manufactured by Sumitomo Bakelite; product number MS-8096W). Using a microplate luminometer LB96P (manufactured by EG & G BERTHOLD), 100 μl of the modified Krebs-Ringer buffer containing 2 μM of a calcium ionophore A23187 (manufactured by Research Biochemical International) was added to each of the cell-inoculated wells and the luminescence was immediately measured at intervals of 3 seconds.

By the above method, the expressed amounts of apoaequorin in the cell line clone 21 transformed with plasmid pCAG-AEQ-pHPRTp and the mutant clone 21Δhprt obtained by removing expression unit of the hprt gene from the clone 21 were compared. The results are shown in Fig. 42. It was shown that the clone 21 containing an hprt gene expression unit having the hprt gene in the downstream of the PKG promoter expresses the apoaequorin gene in higher level than the clone 21Δhprt.

Reference Example 4

Preparation of transgenic mouse having hprt gene in the downstream of PKG promoter and containing apoaequorin gene connected to CAG promoter in further downstream:

(1) Injection of DNA into fertilized egg and transplantation of the fertilized egg

Eight-week-old mice of BDF1 line were purchased for egg collection and reared for 1 week under light period conditions for 12 hours from 8:00 to 20:00, a follicle-stimulating hormone (pregnant mare serum gonadotropin, generally referred to as "PMSG") was intraperitoneally injected at 17:00 on the 1st day (51 U/individual), a luteinizing hormone (human chorionic gonadotropin, generally referred to as "hCG") was intraperitoneally injected at 17:00 on the 3rd day (51 U/individual), and at 17:00, each individual was allowed to live 1 : 1 with other individual of male mouse of BDF1 line of in or after 8 weeks of age for crossbreeding. Vaginal plug confirmation of crossed female rats was carried out at 9:00 on the 4th day, and individuals after the vaginal plug confirmation were sacrificed starting at 13:30 to start egg collection. A DNA fragment of about 6.6 Kb containing the hprt gene connected to the PGK promoter and the apoaequorin gene connected to the CAD promoter (hereinafter referred to as "trans-gene") was prepared by digesting the plasmid pCAG-AEQ-pHPRTp obtained in Reference Example 1(9) with restriction enzymes *Pvu*I and *Sac*I, recovering the fragment after agarose gel electrophoresis in 1 mM Tris (pH 8.0) solution using QIAEX II Gel Extraction Kit (manufactured by QUIAGEN) and adjusting the solution to a concentration of 10 μg to 100 μg/ml using PBS. A pronucleus-formed egg was selected from the fertilized eggs and starting at 14:30, 1 to 2 μl of the prepared DNA solution of the trans-gene was injected into male pronucleus of the fertilized single cell stage BDF1 line mouse egg while observing under a microscope. Next, the egg cell was cultured using the well known modified Witten medium and, after confirmation of the two cell stage embryo at 13:30 on the 5th day, transplanted into the oviduct of a pseudopregnancy female MCH line mouse and implanted in a manner similar to the method described in *Manipulating the Mouse Embryo, A* Laboratory Manual, Second Edition, Cold Spring Harbor Laboratory Press (1994). Regarding the pseudopregnancy female MCH line mouse (in or after 10 weeks of age), a female MCH line mouse of in or after 10 weeks of age at proestrus to estrus stage was allowed to live 1 : 1 with an individual of spermatic ductligated male mouse of MCH line of in or after 10 weeks of age, at 17:00 on the 4th day to effect crossbreeding. The crossed female mouse was checked for vaginal plug confirmation at 9:00 on the 5th day and used for the above object.

(1)Detection of trans-gene from offspring

An experimentation was carried out by PCR using genome DNA collected from the tail of an offspring reached 2-week-old from the birth. Specifically, PCR was carried out using Primer 1 of 27 mer shown in SEQ ID NO:17 and Primer 2 of 28 mer shown in SEQ ID NO:18 containing the cDNA nucleotide sequence of the apoaequorin gene. The genome DNA used in the analysis was extracted from about 0.5 cm fragment of the tail by the method described in *Manipulating the Mouse Embryo - A Laboratory Manual,* Second Edition, Cold Spring Harbor Laboratory Press (1994). The obtained nucleic acid pellet was washed once in 70% ethanol, dried and then re-suspended in 10 mM Tris (pH 8.0) containing 300 μl of 200 μg/ml RNase A and 1 mM EDTA and used as a tail genome DNA preparation. The tail genome DNA preparation (1 μl) was diluted 50 folds with sterilized water, PCR was carried out using Primer 1 and Primer 2 by a reaction at 94°C for 5 minutes, subsequently 30 repeating cycles, each cycle consisting of reactions at 94°C for 1 minute, 55°C for 2 minutes and 72°C for 3 minutes, and finally a reaction at 72°C for 10 minutes, and the reaction solution was preserved at 4°C overnight. The reaction product was subjected to an electrophoresis by passing it through a 1.0% agarose GTG (manufactured by FMC Bioproduct) gel to thereby select a mouse from which a DNA band having a size of about 600 bp was found. The DNA band having a size of about 600 bp was found in 5 mice among a total of 21 born offsprings. Based on the above results, it was confirmed that the mice containing the trance gene in at least tail cells were 5 individuals of individual numbers 98075-02-2 (female), 98075-04-5 (male), 98075-04-6 (male), 98075-04-7 (female) and 98075-04-9 (female).

(3)Identification of germ line transgenic mice

The 5 individuals in which the presence of trans-gene was confirmed from the tail genome DNA were reared until the age of 8 weeks and crossed with mouse C57BL/6J line individuals of in or after the age of 8 weeks in 1:1 combination of respective male and female or female with male to obtain offsprings, and the experimentation was carried out using genome DNA samples collected from tails of the obtained offsprings. Genome DNA was prepared by the method described in Reference Example 4(2). PCR was carried out using Primer 1 of 27 mer and Primer 2 of 28 mer containing the apoaequorin gene cDNA nucleotide sequence. The tail genome DNA preparation (1 μl) was diluted 50 times with sterilized water, PCR was carried out using Primer 1 and Primer 2 by a reaction at 94°C for 5 minutes, subsequently 30 repeating cycles, each cycle consisting of reactions at 94°C for 1 minute, 64°C for 2 minutes and 72°C for 3 minutes, and finally a reaction at 72°C for 10 minutes, and then the reaction solution was preserved at 4°C overnight. The reaction product was subjected to an electrophoresis by passing it through a 1.0% agarose GTG (manufactured by FMC Bioproduct) gel to thereby select mice ' from which a DNA band having a size of about 600 bp was found. The DNA band having a size of about 600 bp was found in 9 mice among a total of 17 mice of offsprings derived from the individual of individual number 98075-02-2, in 9 mice among a total of 15 mice of offsprings derived from the individual of individual number 98075-04-5 and in 13 mice among a total of 18 mice of offsprings derived from the individual of individual number 98075-04-6. The band was not observed in the offsprings derived from the individual of individual number 98075-04-7 and derived from the individual of individual number 98075-04-9.

Next, one mouse among the offsprings of each of the PCR-positive three individuals was analyzed by the well known Southern hybridization using a radioisotope-labeled DNA probe containing the apoaequorin gene sequence. In each case, the experimentation was carried out by digesting the tail DNA with EcoRI and using, as a probe, a fragment prepared by labeling an *Eco*RI-*Xba*I fragment (0.6 Kb) containing the apoaequorin gene derived from the plasmid pBSAEQpA with $^{32}$P-dCTP (deoxycytidine 5'-triphosphate (dCTP), [α-$^{32}$P]; manufactured by NEN). A commercially available T7 QuickPrime Kit (manufactured by Pharmacia Biotech) was used in the labeling. Each DNA sample (10 μg) of the 3 PCR-positive individuals was completely digested with the restriction enzyme *Eco*RI, subjected to 0.8% agarose gel electrophoresis and transferred onto a nylon filter (Hybond™-N+; manufactured by Amersham) in a manner similar to the method described in Southern *(J. Mol. Biol.*, 98, 503 (1975)). The filter was allowed to hybridize overnight with the labeled probe, washed at 65°C twice for 5 minutes and once for 10 minutes with 2× SSC and 0.1% SDS, and superposed on an X-ray film (Kodak Scientific Imaging Film X-OMAT™ AR; manufactured by Kodak) for exposure at -80°C overnight, and development was carried out on the next day. As a result of the Southern hybridization, a signal was found at a position of 4.8 Kbp in 3 mice of the tested 3 mice, so that it was found that these 3 mice maintained the injected trans-gene. When Southern hybridization was carried out on offsprings derived from the PCR-positive three individuals, introduction of the trans-gene was verified in one mouse of offsprings derived from each individual, a total of 3 individuals. Based on the above results, it was confirmed that the germ line transgenic mice are three individuals of individual numbers 98075-02-2 (female), 98075-04-5 (male) and 98075-04-6 (male).

## INDUSTRIAL APPLICABILITY

The present invention provides a physiologically active peptide, a DNA encoding the peptide, an antibody which recognizes the peptide, and application methods thereof useful in screening and developing a therapeutic agent for diseases which accompany infection and inflammation such as microbial infection, HIV infection, chronic hepatitis B, rheumatoid arthritis, sepsis, graft versus host diseases, insulin-dependent diabetes mellitus, glomerulonephritis, Crohn's disease, traumatic brain damage, inflammatory bowel diseases, *etc.;* diseases which accompany abnormal differentiation and proliferation of smooth muscle cells such as arteriosclerosis, re-stricture, *etc.*; diseases which accompany abnormal activation of fibroblasts such as psoriasis, *etc.*; diseases which accompany abnormal activation of a synovial tissue such as rheumatic arthritis, *etc.*; diseases which accompany disorder of pancreatic β cells such as diabetes mellitus, *etc.*; diseases which accompany abnormality of osteoblasts or osteoclasts such as osteoporosis, *etc.*; diseases which accompany abnormal activation of immunocytes such as allergy, atopy, asthma, pollinosis, airway oversensitivity, autoimmune diseases, *etc.*; diseases which accompany disorder of a blood vessel such as myocardial infarction, cerebral infarction, peripheral obstruction, angina pectoris, hypertension, diabetes mellitus, arteriosclerosis, SLE, *etc.*; diseases of an eye based on angiogenesis such as diabetic retinopathy, retinopathy of prematurity, senile macular degeneration, neovascular glaucoma, *etc.*; diseases which accompany neopla such as acute myelomonocytic leukemia, malignant tumor, *etc.*; diseases in which linkage to gene regions of a major histocompatibility antigen is confirmed such as insulin-dependent diabetes mellitus, rheumatoid arthritis, tetanic spondylitis, myasthenia gravis, IgA deficiency, Hashimoto's disease, Basedow's disease, Behcet's disease, *etc.*; and the like.

Free Text of Sequence Listing

SEQ ID NO:7-Description of artificial sequence: Sequence in which the amino acid in the sequence represented by SEQ ID NO:1 were rearranged
SEQ ID NO:8-Description of artificial sequence: Sequence in which the amino acid in the sequence represented by SEQ ID NO:2 were rearranged
SEQ ID NO:9-Description of artificial sequence: Sequence in which the amino acid in the sequence represented by SEQ ID NO:5 were rearranged
SEQ ID NO:17-Description of artificial sequence: Synthetic DNA
SEQ ID NO:18-Description of artificial sequence: Synthetic DNA
SEQ ID NO:19-Description of artificial sequence: Synthetic DNA
SEQ ID NO:20-Description of artificial sequence: Synthetic DNA
SEQ ID NO:21-Description of artificial sequence: Synthetic DNA
SEQ ID NO:22-Description of artificial sequence: Synthetic DNA
SEQ ID NO:28-Description of artificial sequence: Synthetic DNA
SEQ ID NO:29-Description of artificial sequence: Synthetic DNA
SEQ ID NO:30-Description of artificial sequence: Synthetic DNA
SEQ ID NO:31-Description of artificial sequence: Synthetic DNA

## Claims

**1.** A peptide represented by the following formula (I);

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence selected from the group consisting of the following (a) to (l)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;
(h) an amino acid sequence comprising amino acids of positions 17 to 39 in the amino acid sequence represented by SEQ ID NO:2;
(i) an amino acid sequence comprising amino acids of positions 17 to 39 in the amino acid sequence represented by SEQ ID NO:3;
(j) an amino acid sequence comprising amino acids of positions 17 to 39 in the amino acid sequence represented by SEQ ID NO:4;
(k) an amino acid sequence comprising amino acids of positions 17 to 39 in the amino acid sequence represented by SEQ ID NO:5;
(l) an amino acid sequence comprising amino acids of positions 17 to 39 in the amino acid sequence represented by SEQ ID NO:6.

**2.** A peptide, which has an activity to change a concentration of an intercellular calcium ion, represented by the following formula (I):

$$R^1\text{-A-}R^2 \quad \text{(I)}$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence in which one or more amino acid are substituted, deleted or added in the amino acid sequence selected from the group consisting of the following (a) to (l)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6;
(g) an amino acid sequence comprising amino acids of positions 17 to 39 in the amino acid sequence represented by SEQ ID NO:1;
(h) an amino acid sequence comprising amino acids of positions 17 to 39 in the amino acid sequence represented by SEQ ID NO:2;
(i) an amino acid sequence comprising amino acids of positions 17 to 39 in the amino acid sequence represented by SEQ ID NO:3;
(j) an amino acid sequence comprising amino acids of positions 17 to 39 in the amino acid sequence represented by SEQ ID NO:4;
(k) an amino acid sequence comprising amino acids of positions 17 to 39 in the amino acid sequence represented by SEQ ID NO:5;
(l) an amino acid sequence comprising amino acids of positions 17 to 39 in the amino acid sequence represented by SEQ ID NO:6.

**3.** A peptide, which has an activity to change a concentration of an intracellular calcium ion, represented by the following formula (I):

$$R^1\text{-A-}R^2 \qquad (I)$$

(wherein $R^1$ represents a hydrogen atom, substituted or unsubstituted alkanoyl, substituted or unsubstituted aroyl, substituted or unsubstituted heteroarylcarbonyl, substituted or unsubstituted alkoxycarbonyl, substituted or unsubstituted aryloxycarbonyl, or substituted or unsubstituted heteroaryloxycarbonyl; $R^2$ represents hydroxy, substituted or unsubstituted alkoxy, or substituted or unsubstituted amino; and A represents a peptide residue comprising an amino acid sequence having a homology of 60% or more with the amino acid sequence selected from the group consisting of the following (a) to (f)), or a pharmaceutically acceptable salt thereof:

(a) the amino acid sequence represented by SEQ ID NO:1;
(b) the amino acid sequence represented by SEQ ID NO:2;
(c) the amino acid sequence represented by SEQ ID NO:3;
(d) the amino acid sequence represented by SEQ ID NO:4;
(e) the amino acid sequence represented by SEQ ID NO:5;
(f) the amino acid sequence represented by SEQ ID NO:6.

**4.** The peptide according to claim 2 or 3, wherein the activity to change a concentration of an intracellular calcium ion is an activity to increase a concentration of an intracellular calcium ion, or a pharmaceutically acceptable salt thereof.

**5.** The peptide according to claim 2 or 3, wherein the cell is derived from a bone or a spleen, or a pharmaceutically acceptable salt thereof.

**6.** A peptide comprising an amino acid sequence selected from the group consisting of the amino acid sequences represented by SEQ ID NOs:36, 37, 38 and 10.

**7.** A DNA which encodes the peptide according to any one of claims 1 to 6.

**8.** A DNA which comprises the nucleotide sequence represented by SEQ ID NO:11, 12, 13, 14, 15 or 16.

**9.** A DNA which comprises a nucleotide sequence comprising nucleotides of positions 49 to 117 in the nucleotide sequence represented by SEQ ID NO:11, 12, 13, 14, 15 or 16.

**10.** A DNA which hybridizes with the DNA according to any one of claims 7 to 9 under stringent conditions and has an activity to change a concentration of an intracellular calcium ion.

**11.** The DNA according to claim 10, wherein the activity to change a concentration of an intracellular calcium ion is an activity to increase a concentration of an intracellular calcium ion.

**12.** The DNA according to claim 10, wherein the cell is derived from a bone or a spleen.

**13.** A recombinant DNA which is obtainable by inserting the DNA according to any one of claims 7 to 12 into a vector.

**14.** A transformant which is obtainable by introducing the recombinant DNA according to claim 13 into a host cell.

**15.** A process for producing a peptide, which comprises culturing the transformant according to claim 14 in a medium to thereby form and accumulate the peptide according to claim 1 to 6 in the culture, and recovering the peptide from the culture.

**16.** An antibody which recognizes the peptide according to any one of claims 1 to 6.

**17.** The antibody according to claim 16, which recognizes the amino acid sequence represented by SEQ ID NO:1 and recognizes the amino acid sequence represented by SEQ ID NO:5.

**18.** The antibody according to claim 16 or 17, which recognizes the peptide represented by SEQ ID NO:36 and recognizes the peptide represented by SEQ ID NO:38.

**19.** The antibody according to claim 16, which recognizes the amino acid sequence represented by SEQ ID NO:1 but does not recognize the amino acid sequence represented by SEQ ID NO:5.

**20.** The antibody according to claim 16 or 19, which recognizes the peptide represented by SEQ ID NO:36 but does not recognize the peptide represented by SEQ ID NO:38.

**21.** The antibody according to claim 16, which recognizes the amino acid sequence represented by SEQ ID NO:5 but does not recognize the amino acid sequence represented by SEQ ID NO:1.

**22.** The antibody according to claim 16 or 21, which recognizes the peptide represented by SEQ ID NO:38 but does not recognize the peptide represented by SEQ ID NO:36.

**23.** The antibody according to claim 16, which is an antibody having an activity to inhibit the peptide according to any one of claims 1 to 7.

**24.** The antibody according to claim 23, wherein the peptide is represented by SEQ ID NO:36 or 38.

**25.** A monoclonal antibody which is produced by a hybridoma KM2952 (FERM BP-7657).

**26.** A monoclonal antibody which is produced by a hybridoma KM3022 (FERM BP-7658).

**27.** A monoclonal antibody which is produced by a hybridoma KM3030 (FERM BP-7659).

**28.** A monoclonal antibody which is produced by a hybridoma KM2947 (FERM BP-7656).

**29.** An oligonucleotide which corresponds to a sequence comprising continuous 5 to 60 nucleotides in the nucleotide sequence contained in the DNA according to any one of claims 7 to 12, or an oligonucleotide which corresponds to a sequence complementary to the oligonucleotide.

**30.** A method for detecting expression of a gene encoding the peptide according to any one of claims 1 to 6 by hybridization, which comprises using the DNA according to any one of claims 7 to 12.

**31.** A method for detecting mutation of a gene encoding the peptide according to any one of claims 1 to 6 by hybridization, which comprises using the DNA according to any one of claims 7 to 12.

**32.** An immunological detection method of the peptide according to any one of claims 1 to 6, which comprises using the antibody according to any one of claims 16 to 28.

**33.** A tissue immunostaining method, which detecting the peptide according to any one of claims 1 to 6 by using the antibody according to any one of claims 16 to 28.

**34.** A method for detecting expression of a gene encoding the peptide according to any one of claims 1 to 6 by polymerase chain reaction, which comprises using the oligonucleotide according to claim 29.

**35.** A method for detecting expression of a gene encoding the peptide according to any one of claims 1 to 6 by polymerase chain reaction, which comprises using the oligonucleotide according to claim 29.

**36.** A method for inhibiting transcription of a gene or translation of a mRNA encoding the peptide according to any one of claims 1 to 6, which comprises using the DNA according to any one of claims 7 to 12 or the oligonucleotide according to claim 29.

**37.** A method for obtaining a promoter region of a gene encoding the peptide according to any one of claims 1 to 6, which comprise using the DNA according to any one of claims 7 to 12 or the oligonucleotide according to claim 29.

**38.** A method for diagnosing diseases which accompany infection and inflammation, diseases which accompany abnormal differentiation proliferation of a smooth muscle cell, diseases which accompany abnormal activation of a fibroblast, diseases which accompany abnormal activation of a synovial tissue, diseases which accompany disorder of a pancreatic β cell, diseases which accompany abnormality of an osteoblast or an osteoclast, diseases which accompany abnormal activation of an immunocyte, diseases which accompany disorder of blood vessel, diseases of an eye based on angiogenesis, diseases which accompany neopla or diseases in which linkage relation to a gene region of a major histocompatibility antigen is confirmed, which comprises using the method according to any one of claims 30 to 35.

**39.** A medicament which comprises the peptide according to any one of claims 1 to 6 as an active ingredient.

**40.** A medicament which comprises the DNA according to any one of claims 7 to 12 as an active ingredient.

**41.** A medicament which comprises the antibody according to any one of claims 16 to 28 as an active ingredient.

**42.** A medicament which comprises the oligonucleotide according to claim 29 as an active ingredient.

**43.** The medicament according to any one of claims 39 to 42, which is a medicament for diagnosing, treating or preventing diseases which accompany infection and inflammation, diseases which accompany abnormal differentiation proliferation of a smooth muscle cell, diseases which accompany abnormal activation of a fibroblast, diseases which accompany abnormal activation of a synovial tissue, diseases which accompany disorder of a pancreatic β cell, diseases which accompany abnormality of an osteoblast or an osteoclast, diseases which accompany abnormal activation of an immunocyte, diseases which accompany disorder of blood vessel, diseases of an eye based on angiogenesis, diseases which accompany neopla or diseases in which linkage relation to a gene region of a major histocompatibility antigen is confirmed.

**44.** A method for screening a receptor which specifically interacts with the peptide according to any one of claims 1 to 6, which comprises using the peptide.

**45.** A receptor which specifically interacts with the peptide according to any one of claims 1 to 6 which is obtainable by the screening method according to claim 44.

**46.** A knockout non-human animal in which expression of a gene encoding the peptide according to any one of claims 1 to 6 is partially or completely suspended.

**47.** A knockout non-human animal in which the activity of the peptide according to any one of claims 1 to 6 is partially or completely suspended.

**48.** A method for screening an agonist for the peptide according to any one of claims 1 to 6, which comprises using the peptide.

**49.** An agonist for the peptide according to any one of claims 1 to 6, which is identified by the method according to claim 48.

**50.** A method for screening an antagonist for the peptide according to any one of claims 1 to 6, which comprises using the peptide.

**51.** An antagonist for the peptide according to any one of claims 1 to 6, which is identified by the method according to claim 50.

**52.** A method for analyzing expression of a gene encoding the peptide according to any one of claims 1 to 6, which comprises using the transformant according to claim 14.

FIG. 1

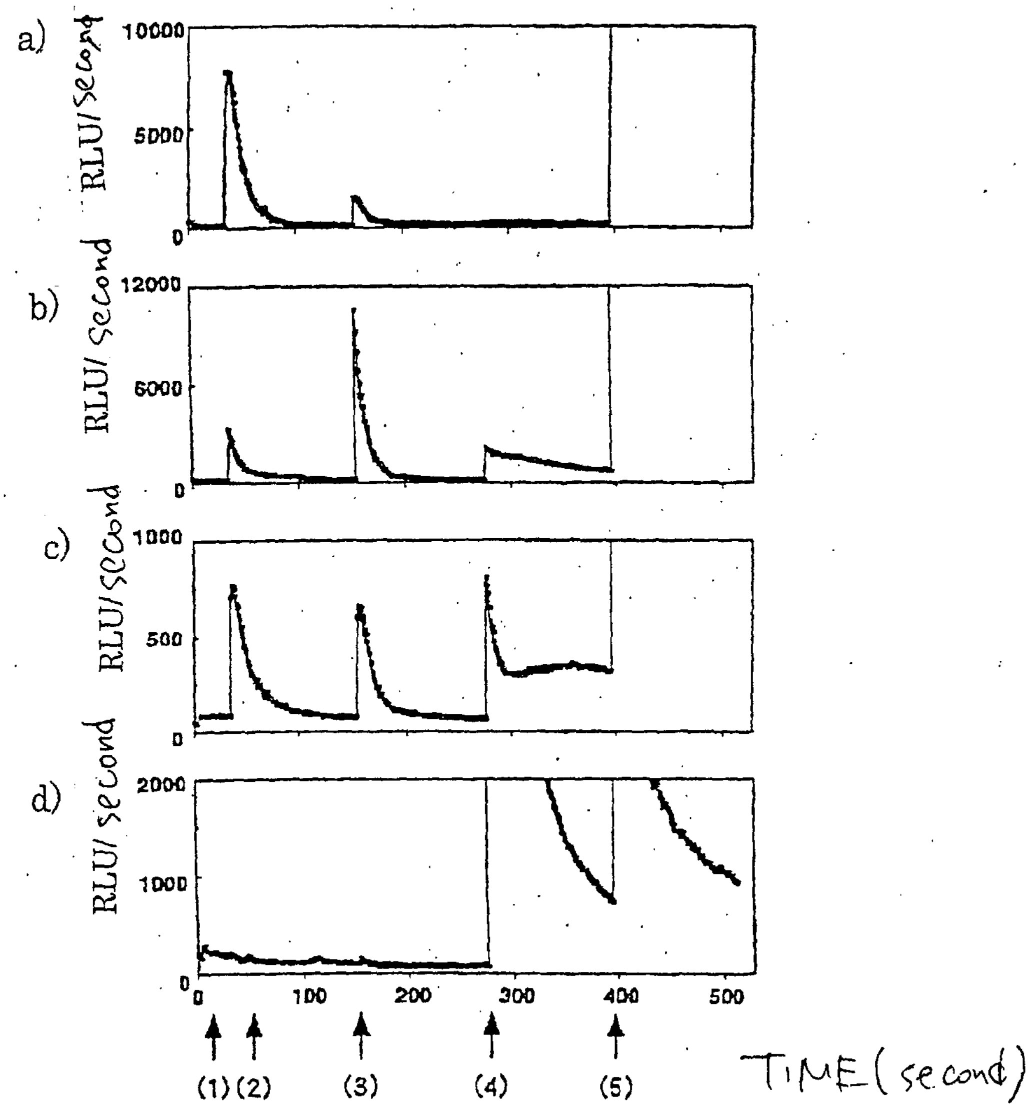
a)
RLU/second
10000
5000
0
b)
RLU/second
12000
6000
0
c)
RLU/second
1000
500
0
d)
RLU/second
2000
1000
0
0
100
200
300
400
500
(1)
(2)
(3)
(4)
(5)
TIME(second)

FIG. 2

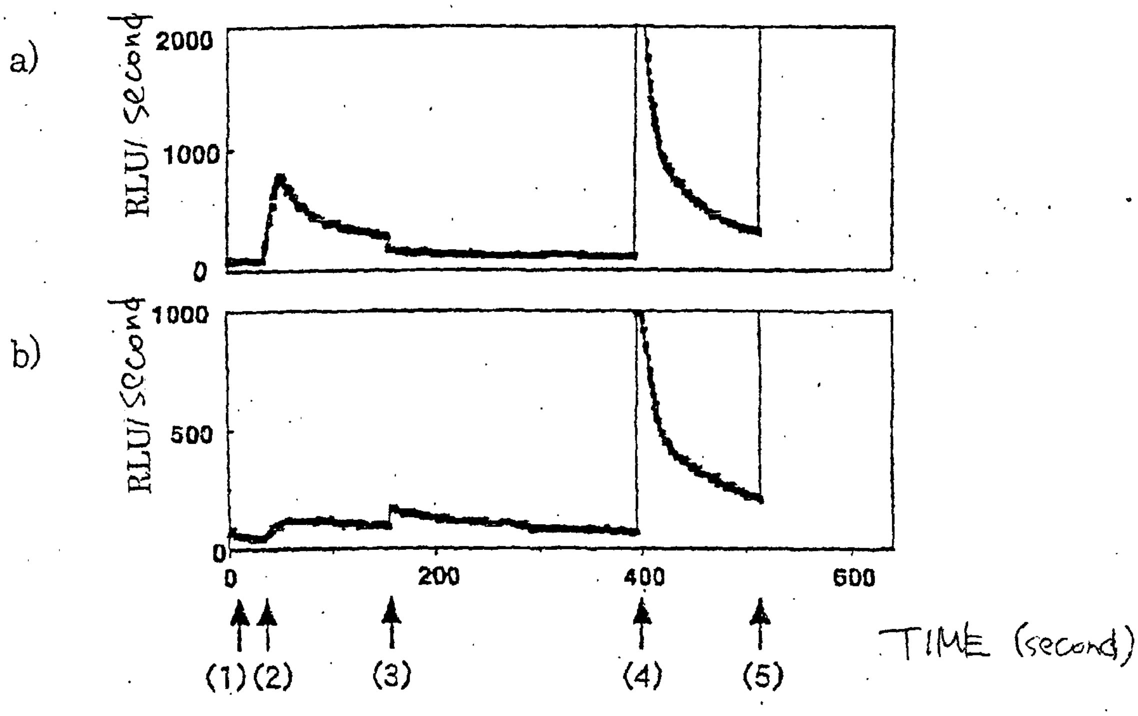
a)
RLU/ second
2000
1000
0
b)
RLU/ second
1000
500
0
0
200
400
600
(1)
(2)
(3)
(4)
(5)
TIME (second)

FIG.3

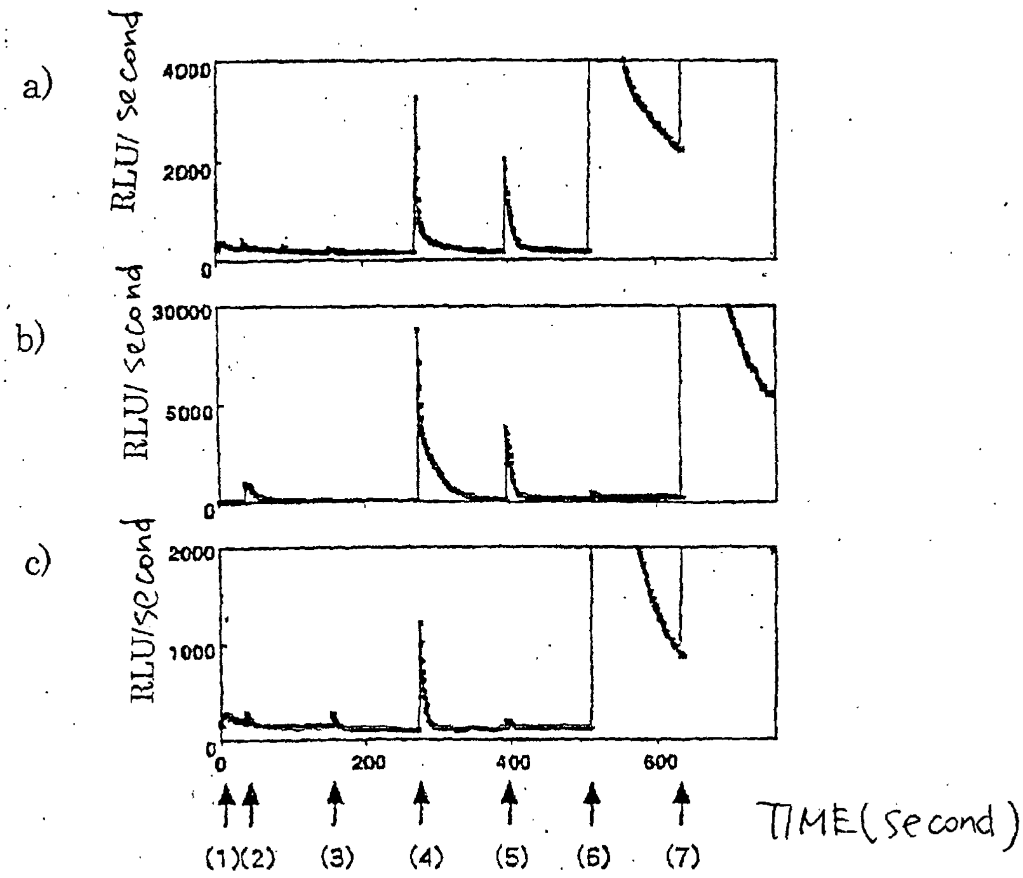
a)
RLU/ second
4000
2000
0
b)
RLU/ second
30000
5000
0
c)
RLU/second
2000
1000
0
0
200
400
600
(1)(2)
(3)
(4)
(5)
(6)
(7)
TIME(second)

FIG.4

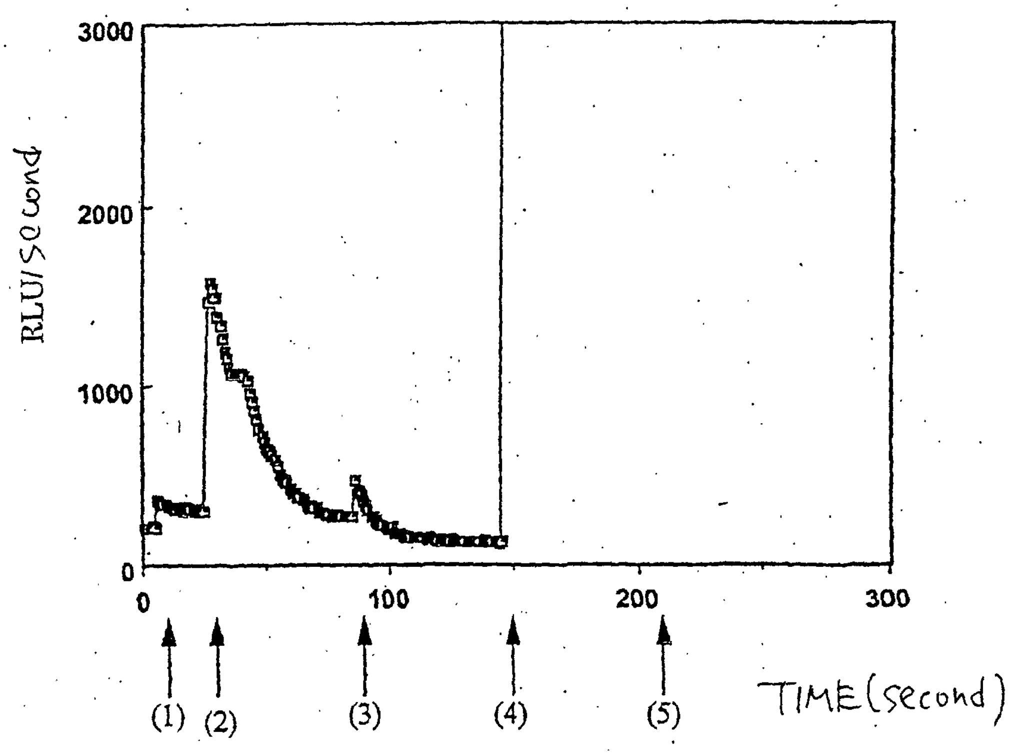
3000
2000
1000
0
RLU/second
0
100
200
300
(1)
(2)
(3)
(4)
(5)
TIME(second)

FIG.5

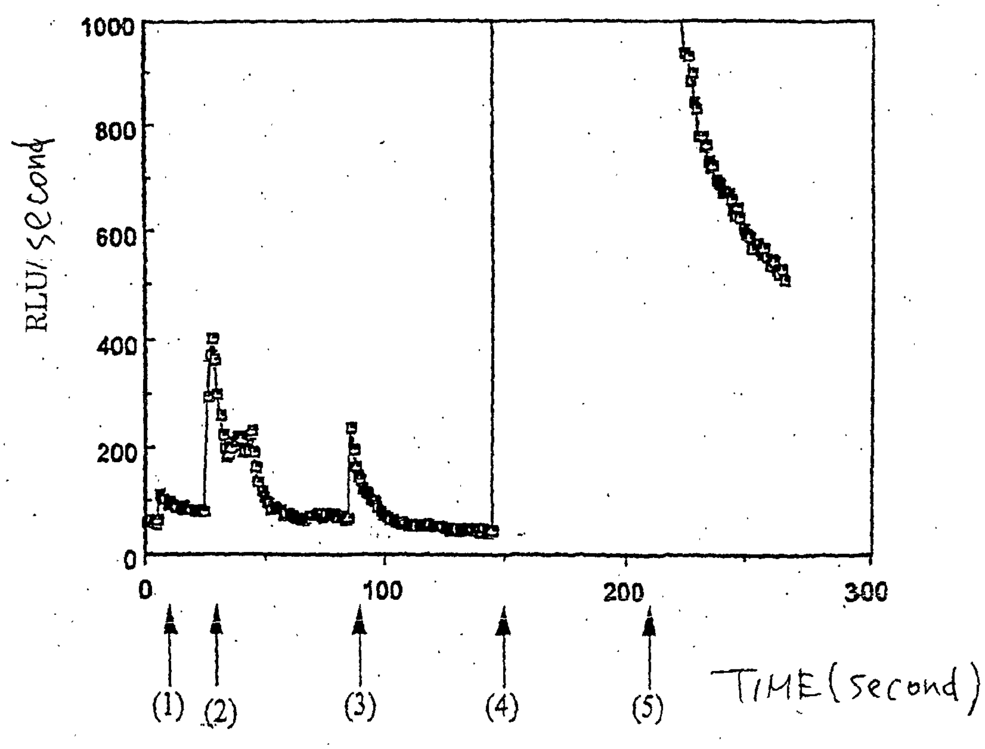
1000
800
600
400
200
0
RLU/second
0
100
200
300
(1)
(2)
(3)
(4)
(5)
TIME(second)

FIG.6

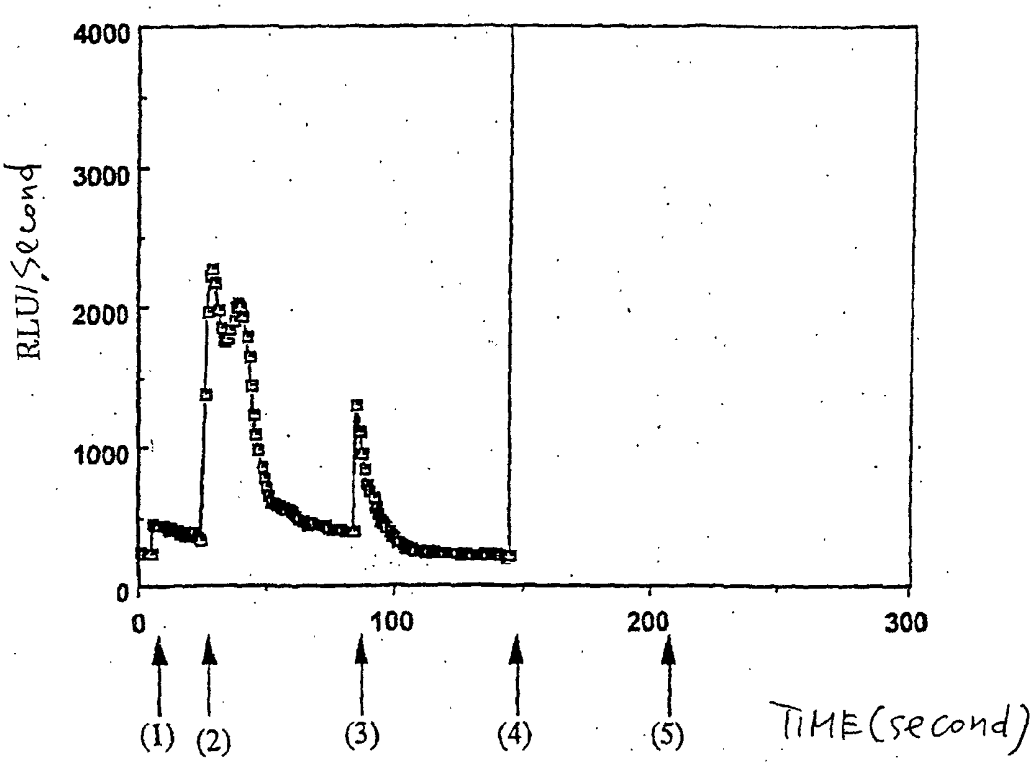
4000
3000
2000
1000
0
RLU/second
0
100
200
300
(1)
(2)
(3)
(4)
(5)
TIME(second)

FIG.7

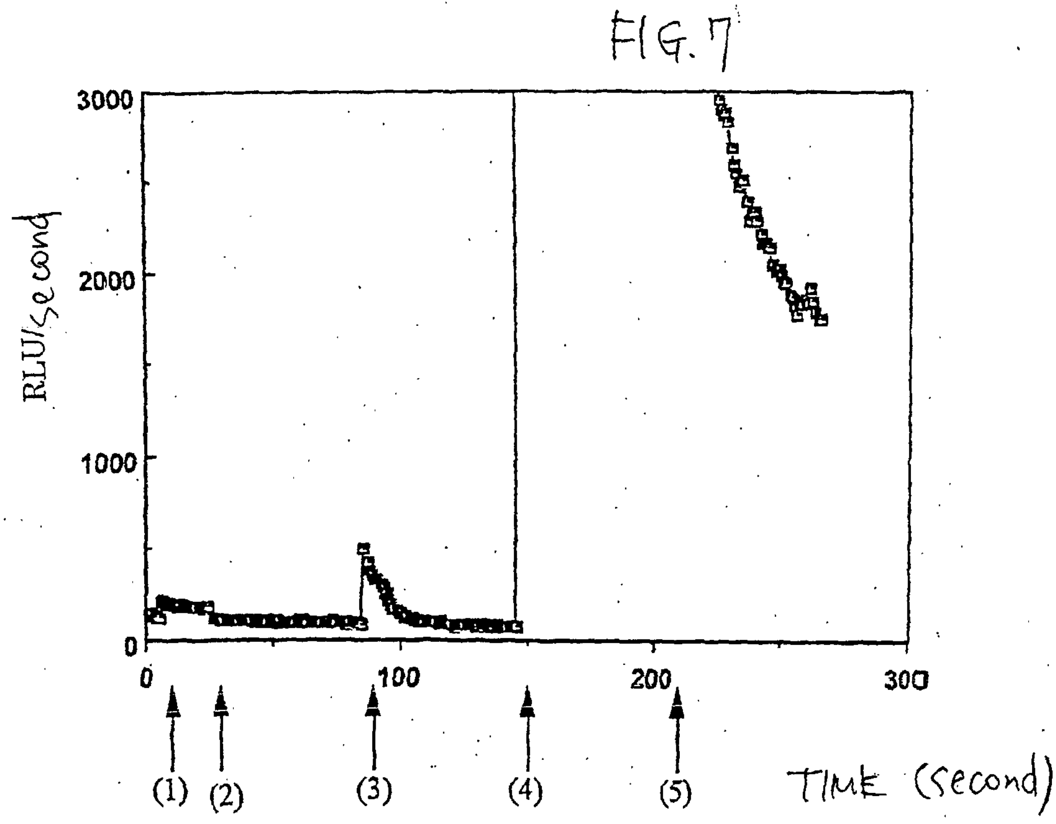
3000
2000
1000
0
RLU/second
0
100
200
300
(1)
(2)
(3)
(4)
(5)
TIME (second)

FIG. 8

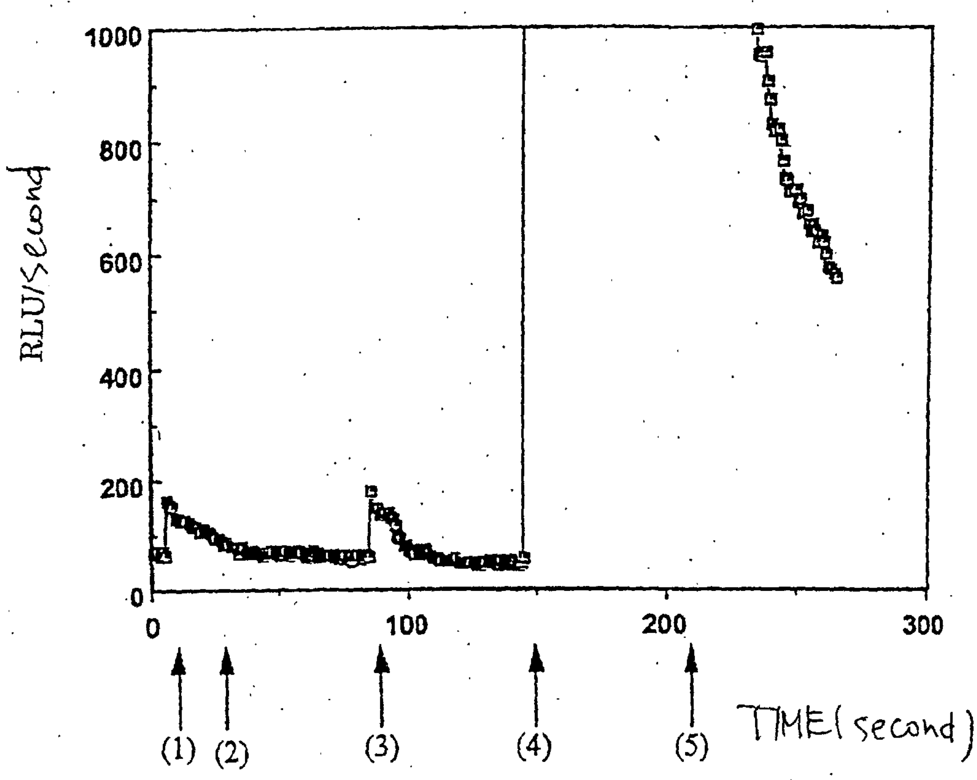
RLU/second
1000
800
600
400
200
0
0
100
200
300
(1)
(2)
(3)
(4)
(5)
TIME (second)

FIG. 9

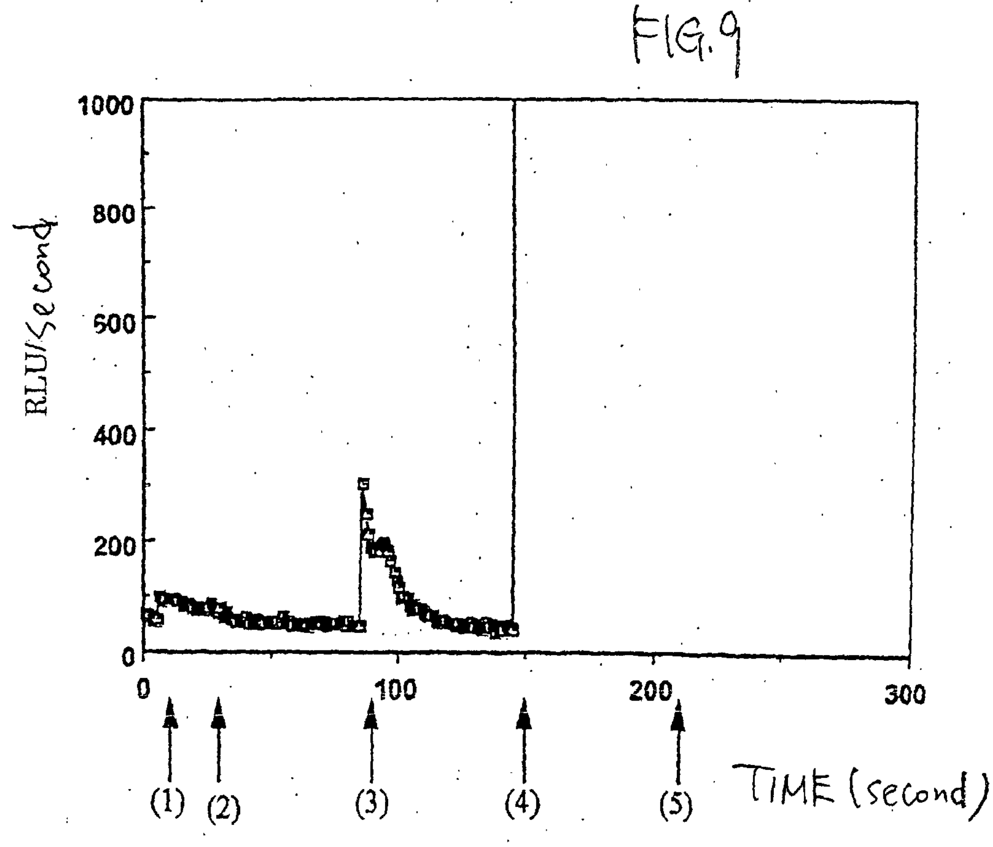
RLU/second
1000
800
600
400
200
0
0
100
200
300
(1)
(2)
(3)
(4)
(5)
TIME (second)

FIG.10

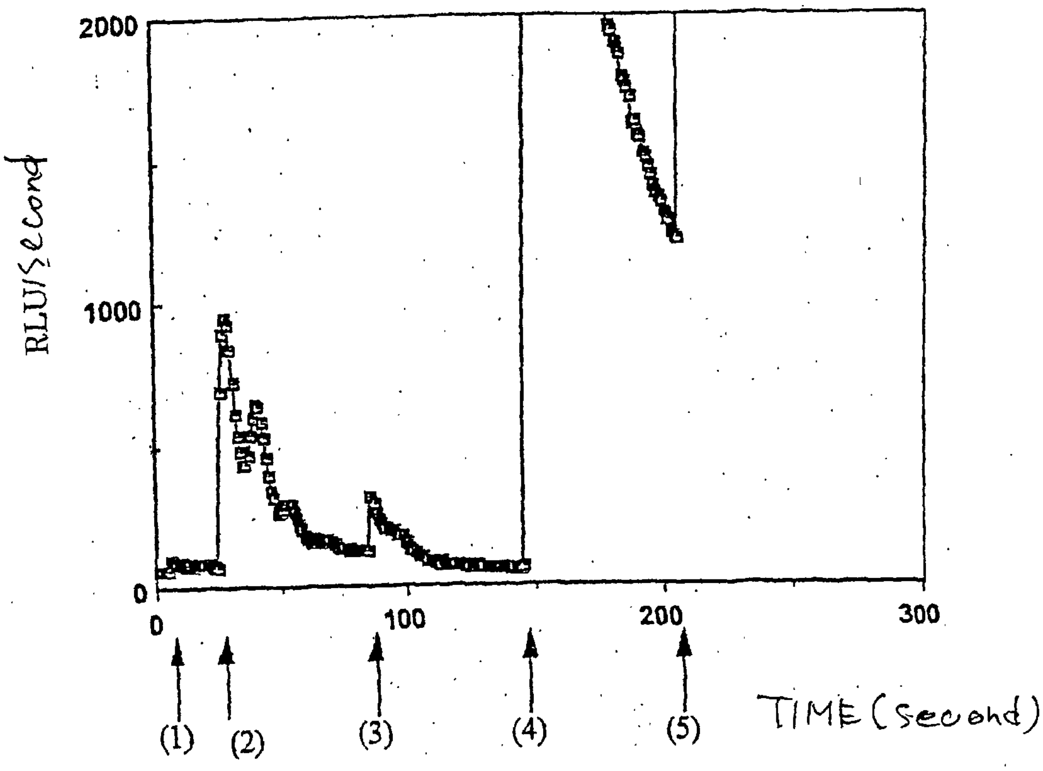
2000
1000
0
RLU/second
0
100
200
300
(1)
(2)
(3)
(4)
(5)
TIME(second)

FIG.11

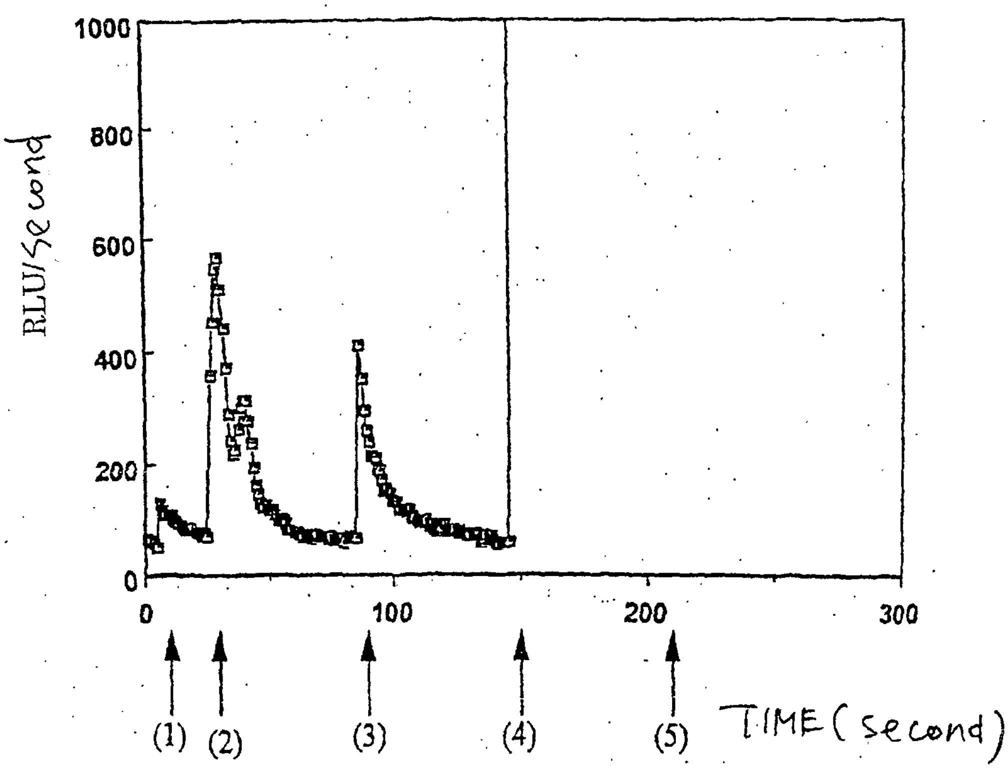
1000
800
600
400
200
0
RLU/second
0
100
200
300
(1)
(2)
(3)
(4)
(5)
TIME(second)

FIG. 12

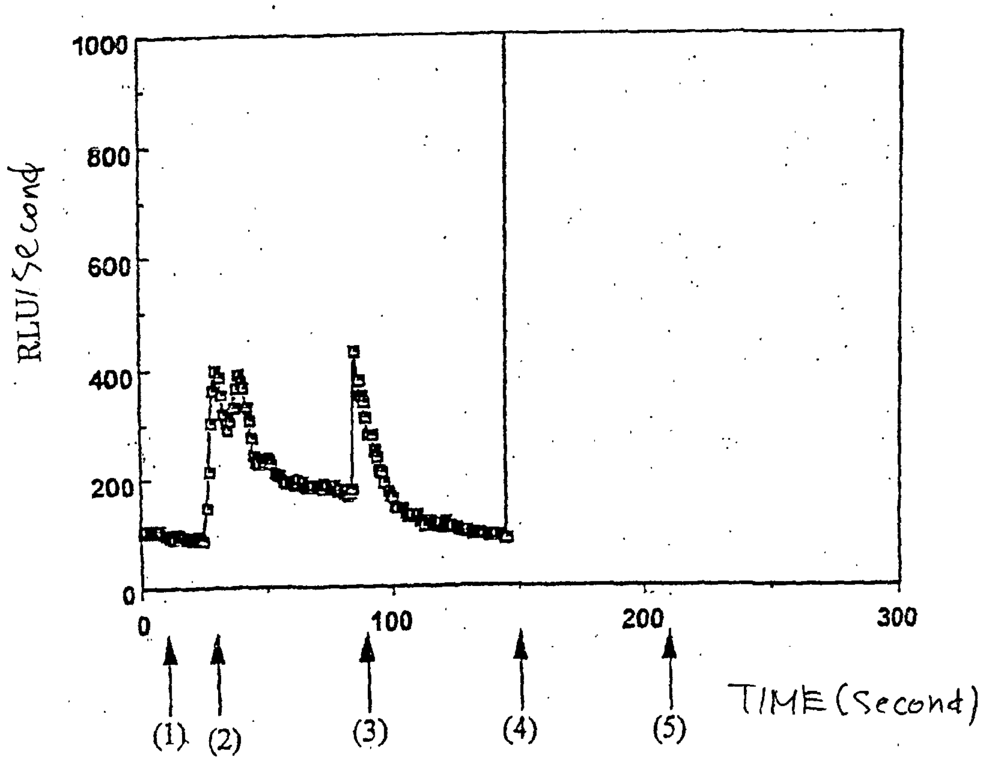
RLU/second
1000
800
600
400
200
0
0
100
200
300
(1)
(2)
(3)
(4)
(5)
TIME (second)

FIG. 13

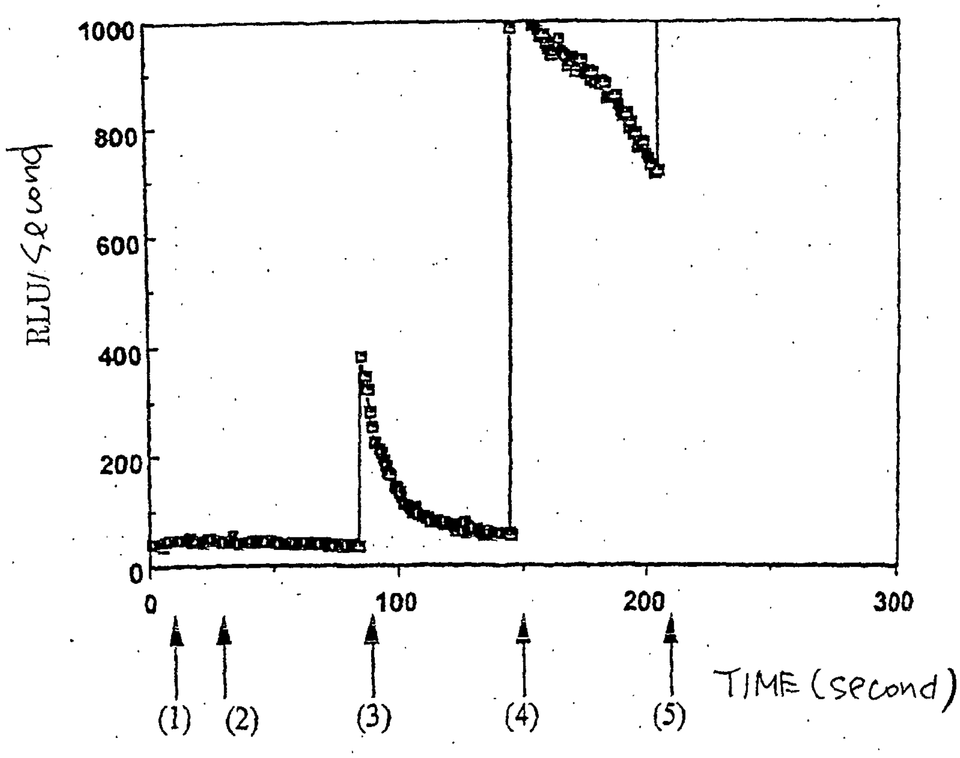
RLU/second
1000
800
600
400
200
0
0
100
200
300
(1)
(2)
(3)
(4)
(5)
TIME (second)

FIG. 14

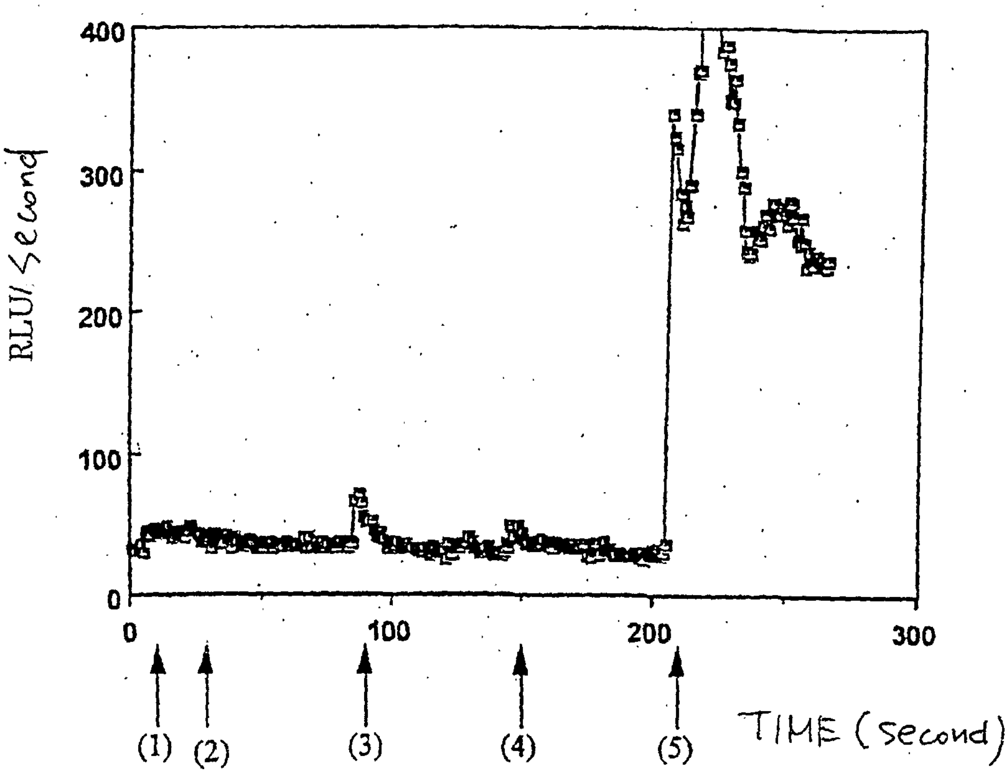
400
300
200
100
0
RLU/ Second
0
100
200
300
(1)
(2)
(3)
(4)
(5)
TIME (second)

FIG. 15

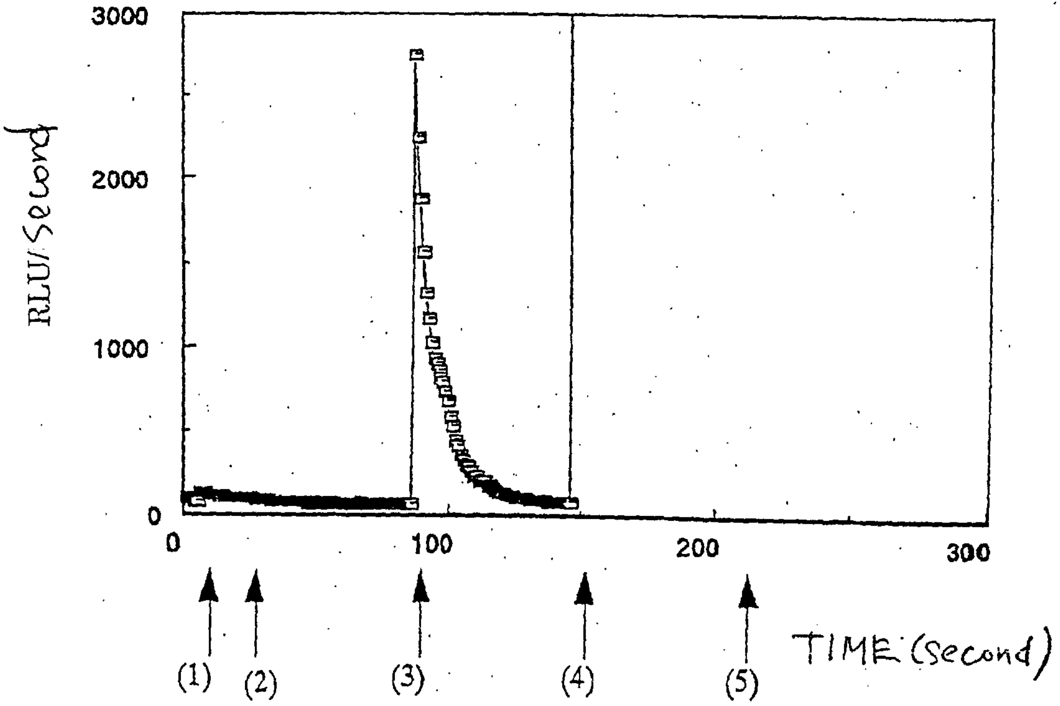
3000
2000
1000
0
RLU/ Second
0
100
200
300
(1)
(2)
(3)
(4)
(5)
TIME (second)

FIG. 16

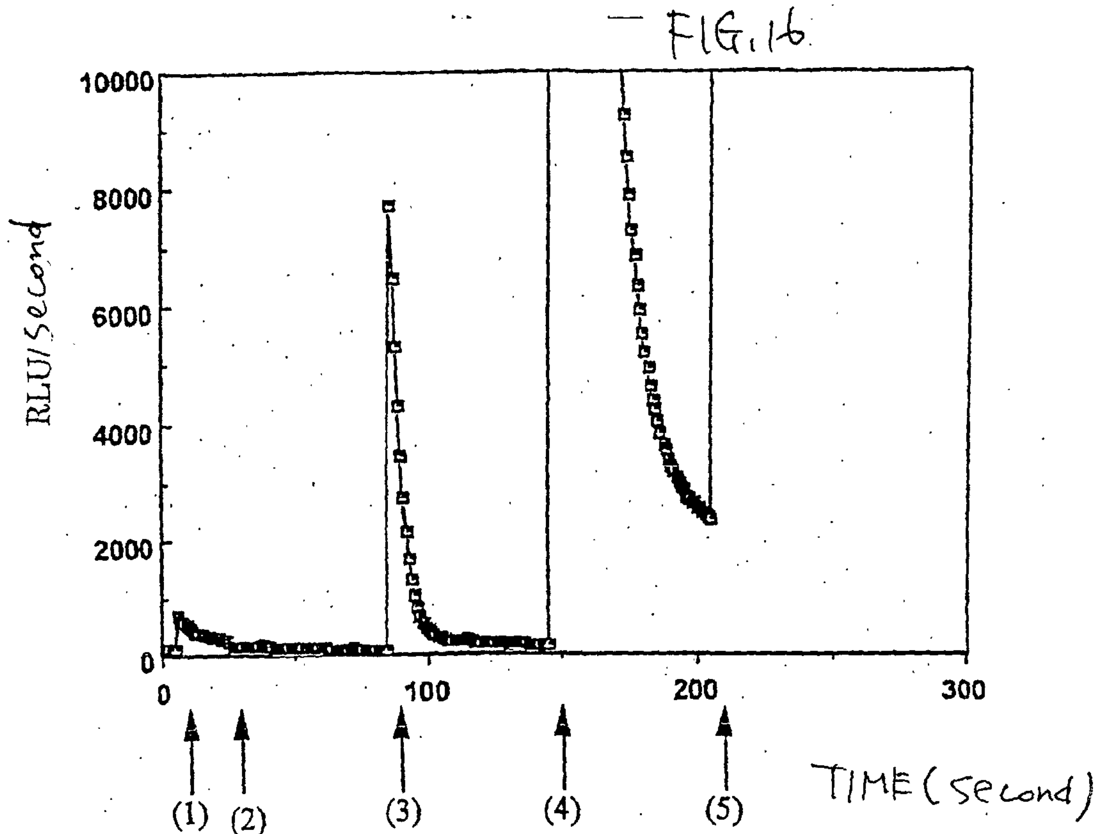
10000
8000
6000
4000
2000
0
RLU/Second
0
100
200
300
(1)
(2)
(3)
(4)
(5)
TIME (second)

FIG. 17

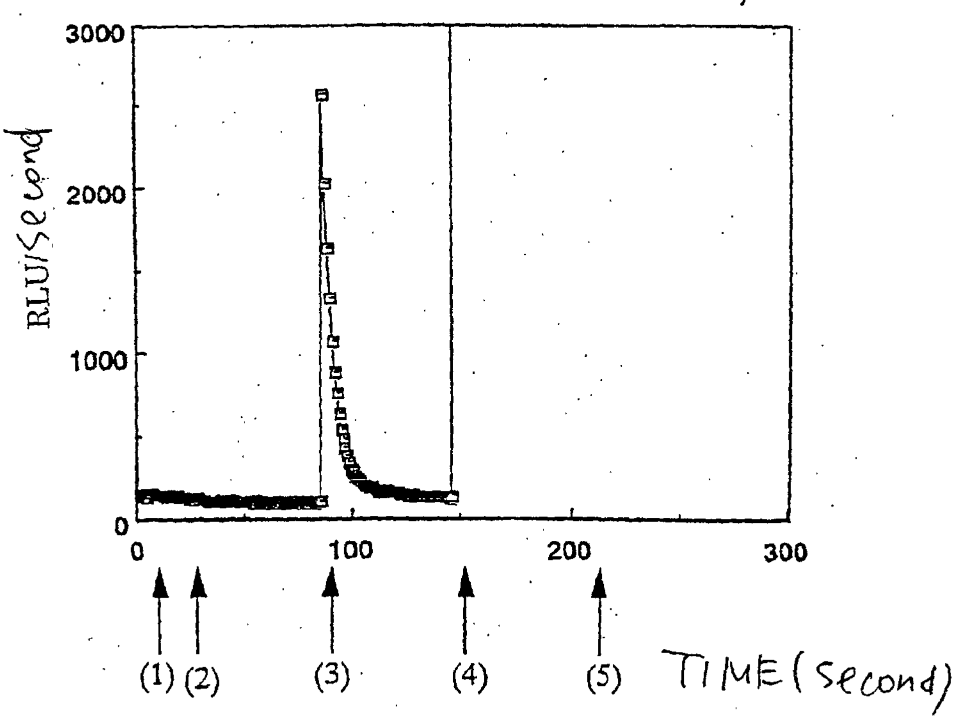
3000
2000
1000
0
RLU/Second
0
100
200
300
(1)
(2)
(3)
(4)
(5)
TIME (second)

FIG. 18

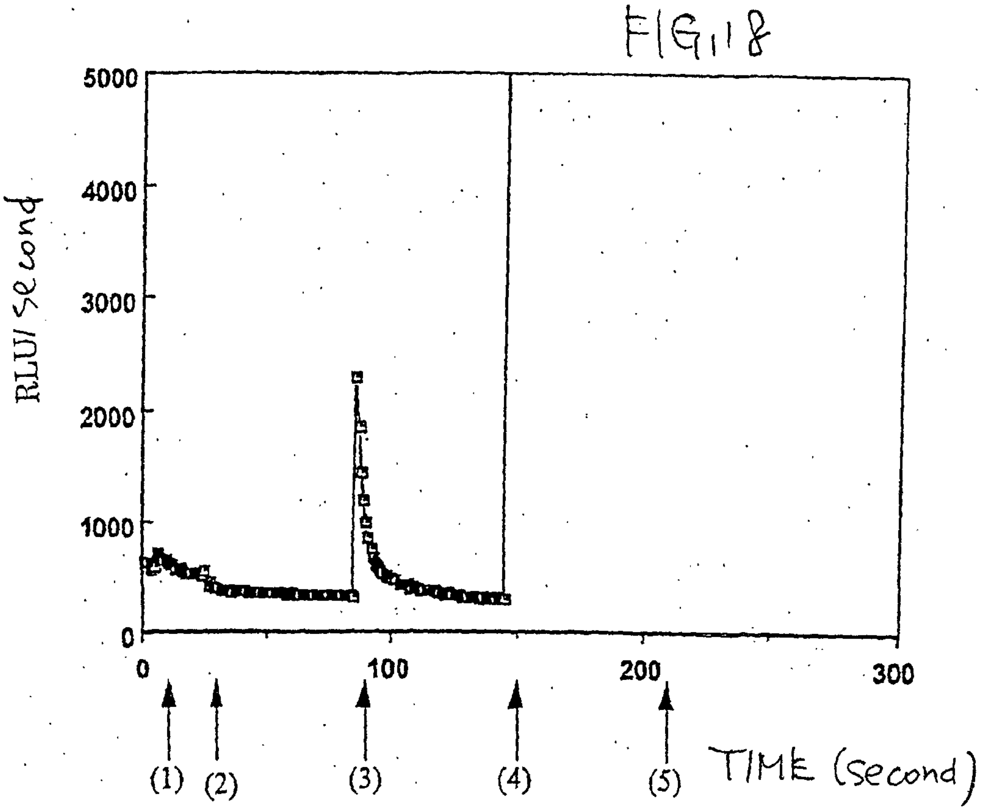
5000
4000
3000
2000
1000
0
RLU/second
0
100
200
300
(1)
(2)
(3)
(4)
(5)
TIME (second)

FIG. 19

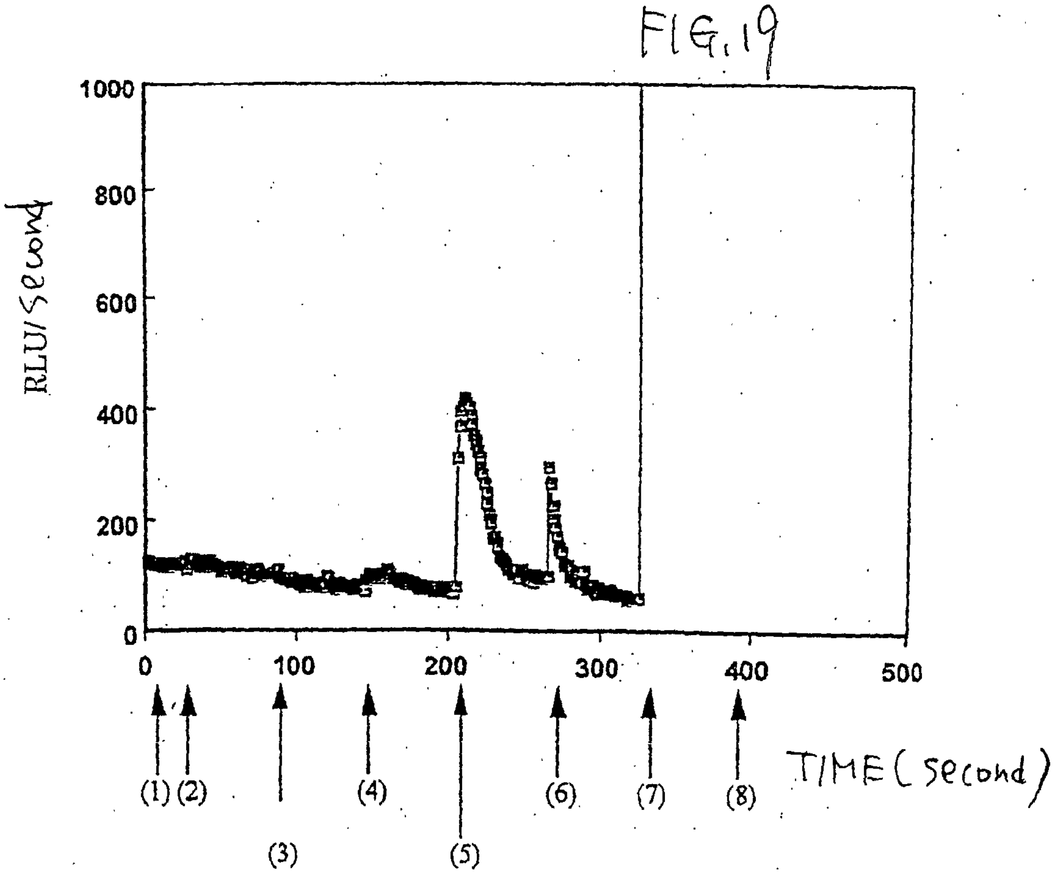
1000
800
600
400
200
0
RLU/second
0
100
200
300
400
500
(1)
(2)
(3)
(4)
(5)
(6)
(7)
(8)
TIME (second)

FIG. 20

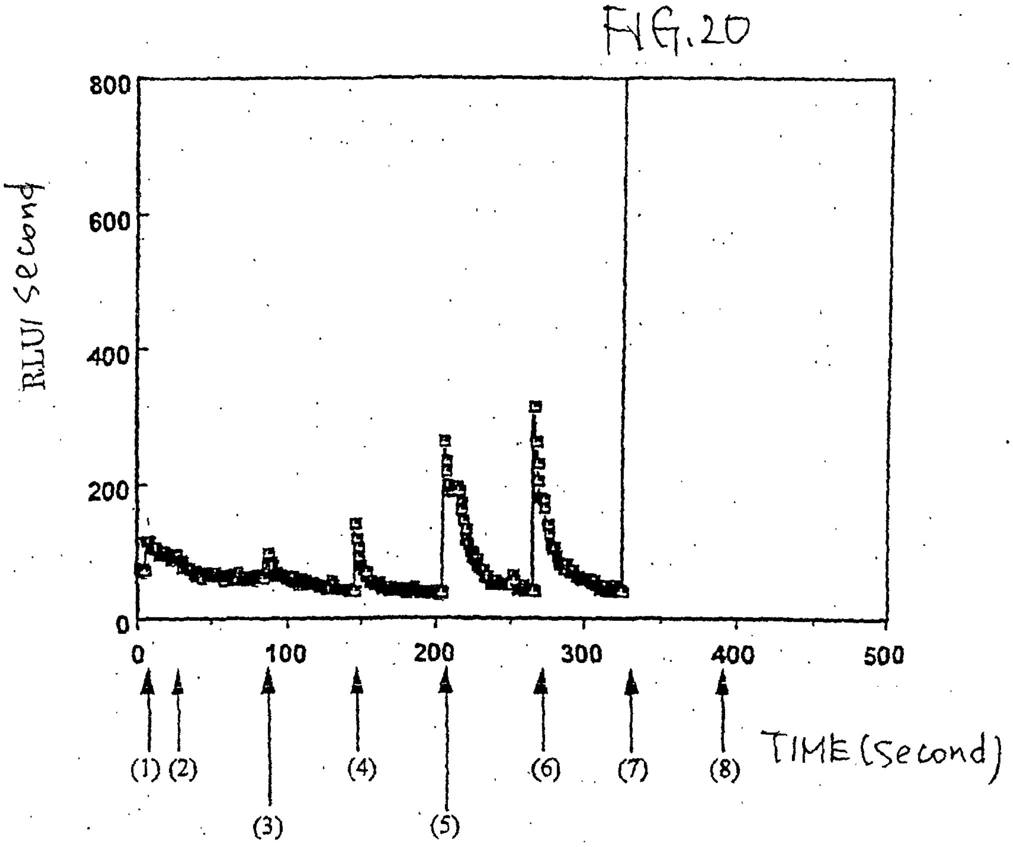
RLU/ second
800
600
400
200
0
0
100
200
300
400
500
(1) (2)
(3)
(4)
(5)
(6)
(7)
(8)
TIME (second)

FIG. 21

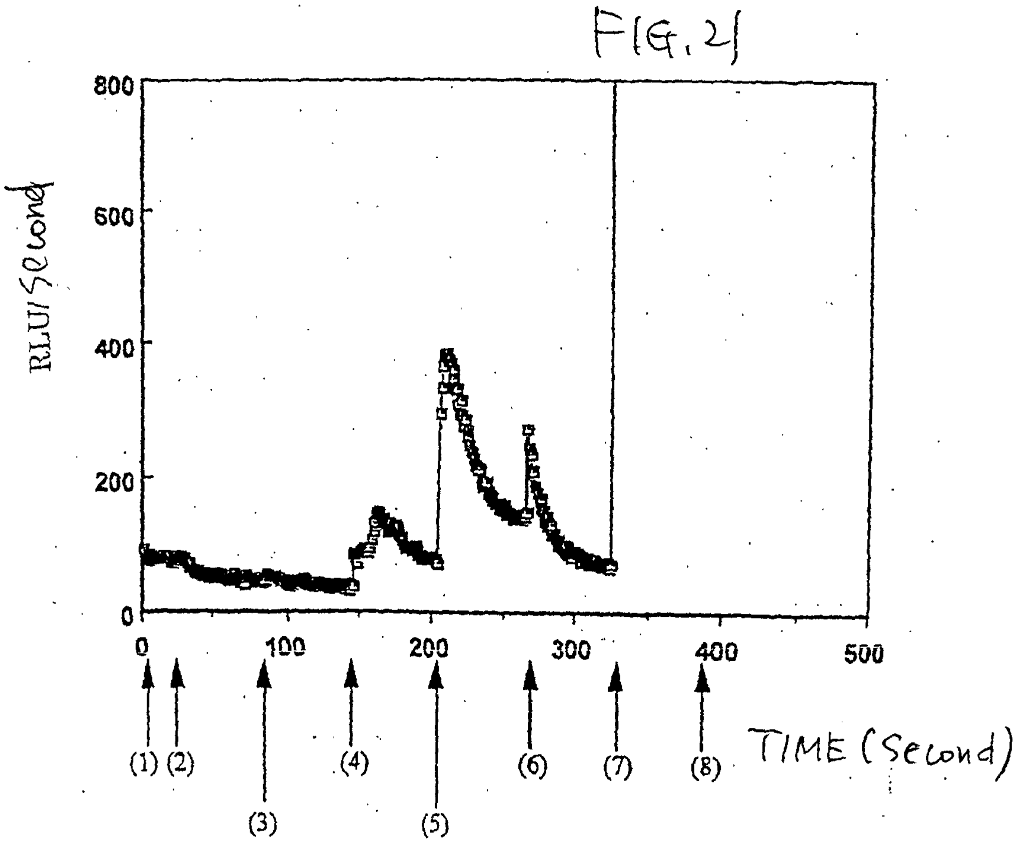
RLU/ second
800
600
400
200
0
0
100
200
300
400
500
(1) (2)
(3)
(4)
(5)
(6)
(7)
(8)
TIME (second)

FIG. 22

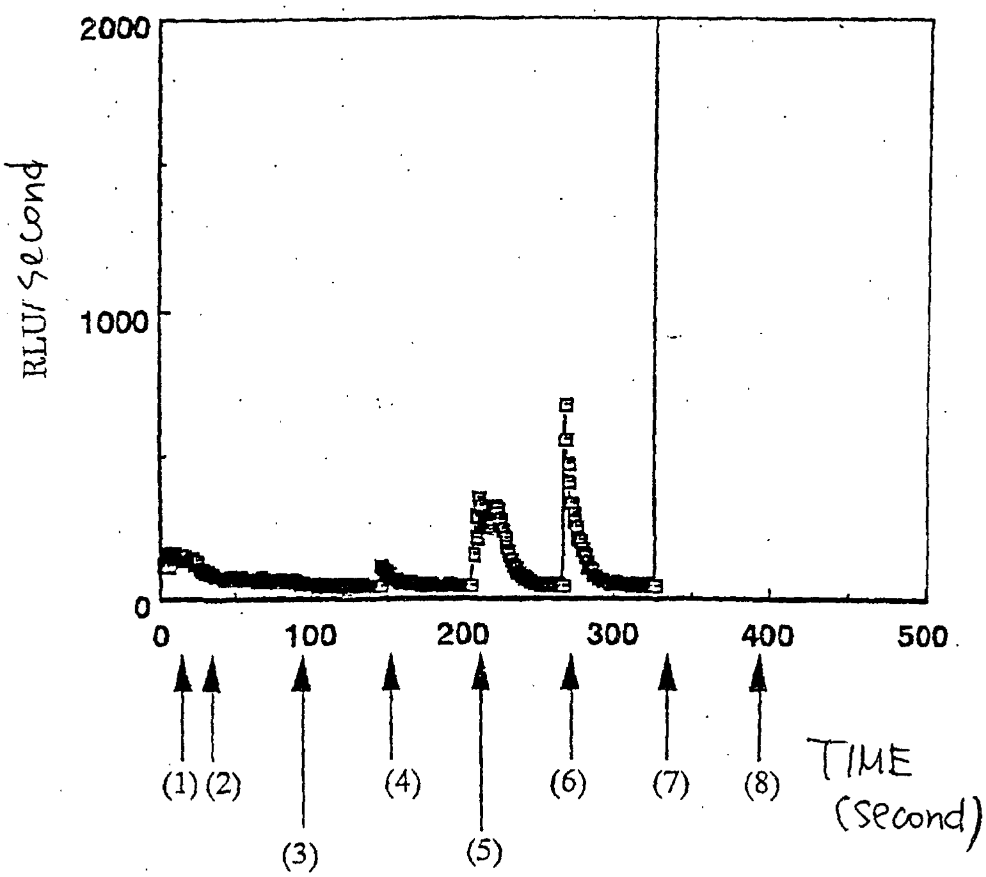
2000
1000
0
RLU/second
0
100
200
300
400
500
(1)
(2)
(3)
(4)
(5)
(6)
(7)
(8)
TIME (second)

FIG. 23

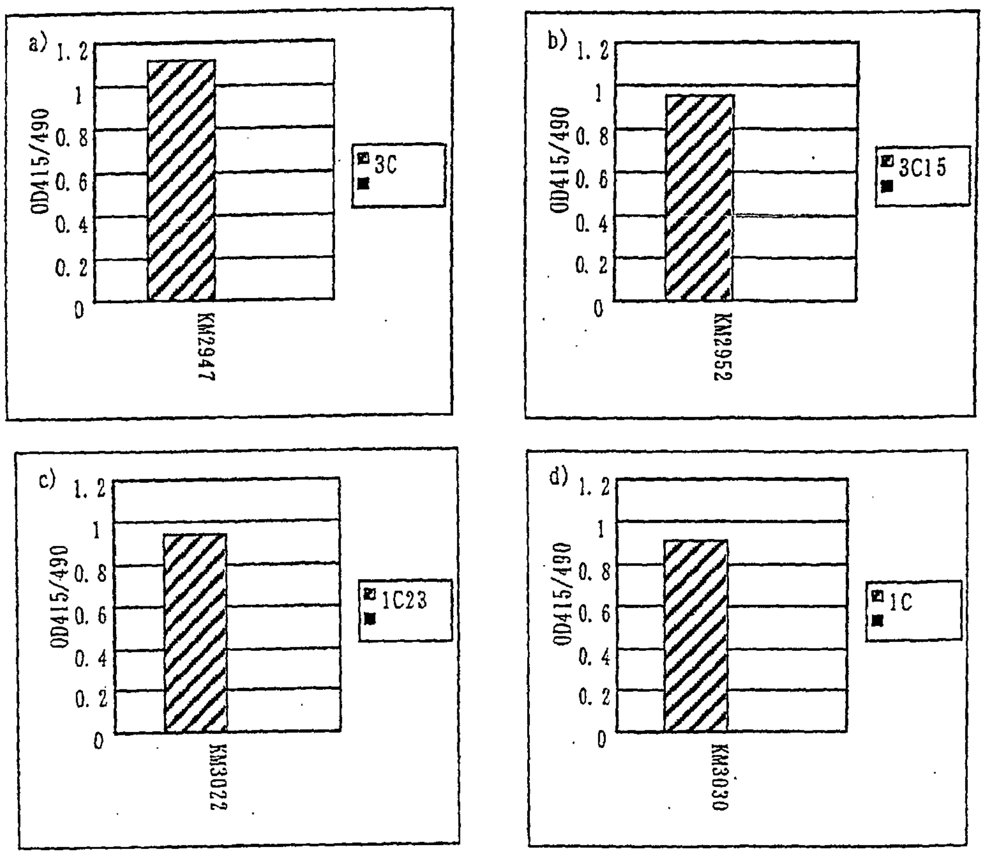
a)
OD415/490
1.2
1
0.8
0.6
0.4
0.2
0
KM2947
3C
b)
OD415/490
1.2
1
0.8
0.6
0.4
0.2
0
KM2952
3C15
c)
OD415/490
1.2
1
0.8
0.6
0.4
0.2
0
KM3022
1C23
d)
OD415/490
1.2
1
0.8
0.6
0.4
0.2
0
KM3030
1C

FIG. 24

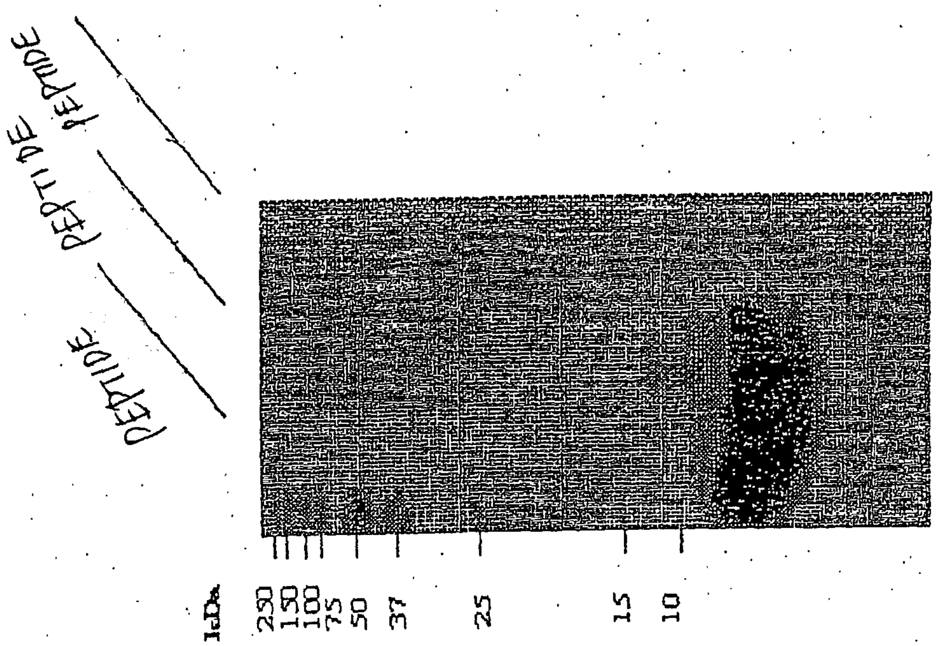
PEPTIDE
PEPTIDE
PEPTIDE
kDa
230
150
100
75
50
37
25
15
10

FIG. 25

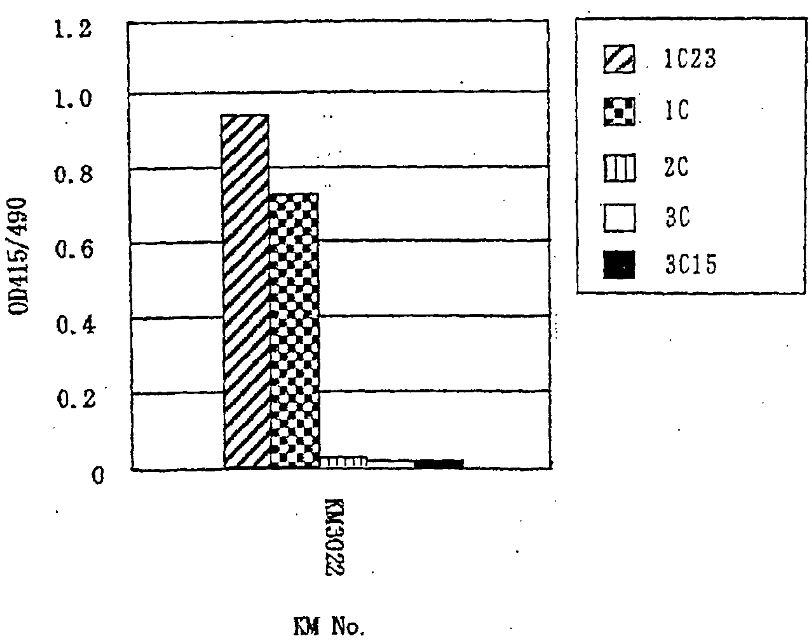
1.2
1.0
0.8
0.6
0.4
0.2
0
OD415/490
KM3022
KM No.
1C23
1C
2C
3C
3C15

FIG. 26

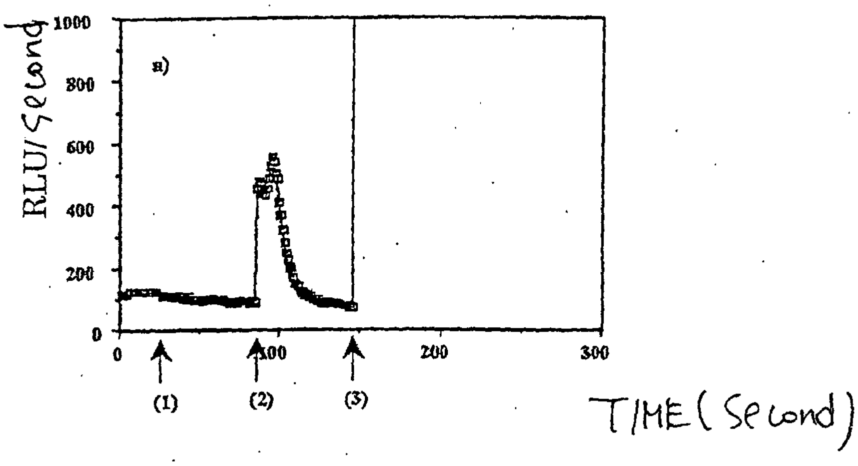
RLU/Second
a)
1000
800
600
400
200
0
0
100
200
300
(1)
(2)
(3)
TIME (Second)

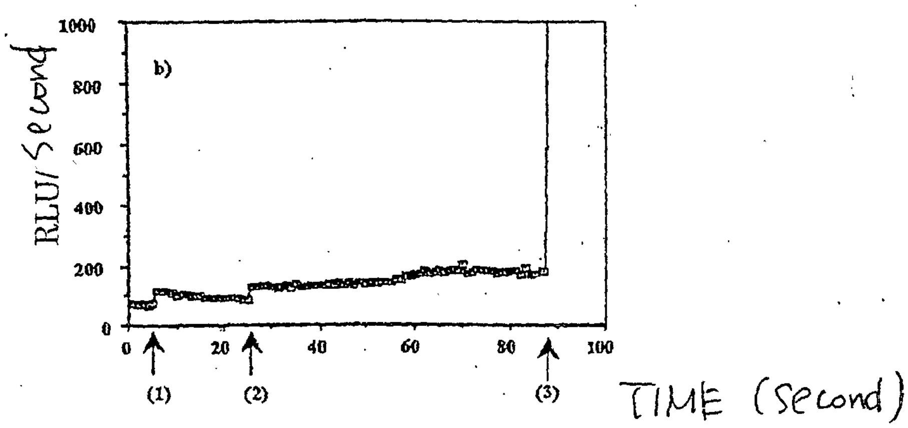
RLU/Second
b)
1000
800
600
400
200
0
0
20
40
60
80
100
(1)
(2)
(3)
TIME (Second)

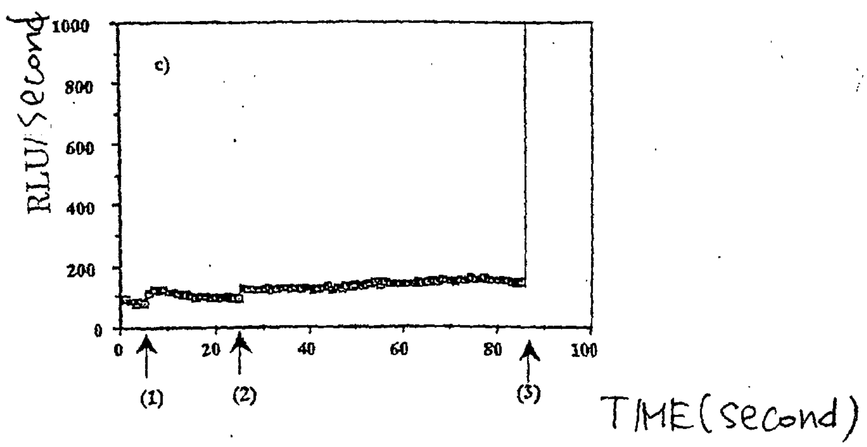
RLU/Second
c)
1000
800
600
400
200
0
0
20
40
60
80
100
(1)
(2)
(3)
TIME (Second)

FIG. 27

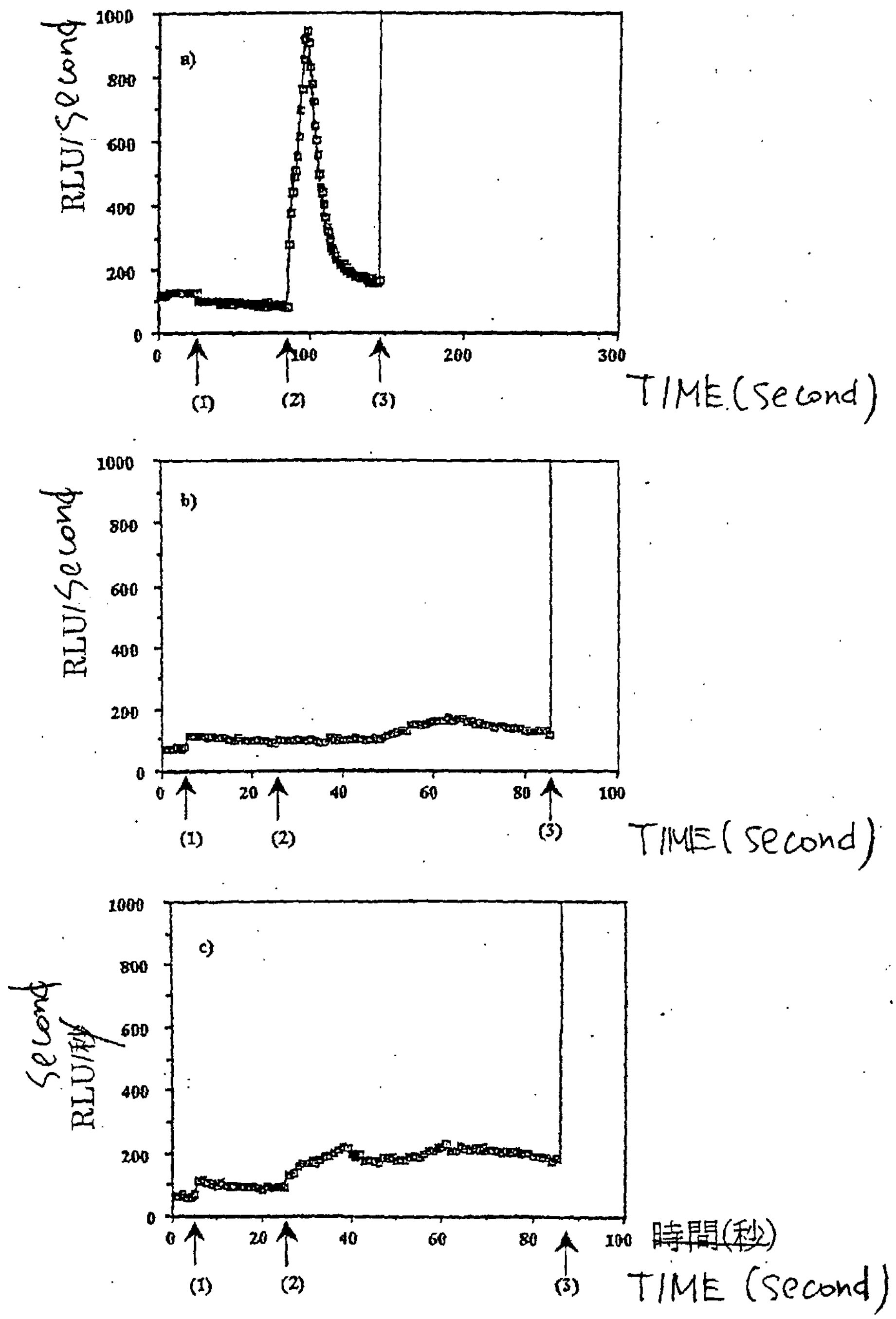
a)
RLU/Second
1000
800
600
400
200
0
0
100
200
300
(1)
(2)
(3)
TIME (Second)
b)
RLU/Second
1000
800
600
400
200
0
0
20
40
60
80
100
(1)
(2)
(3)
TIME (Second)
c)
RLU/秒 Second
1000
800
600
400
200
0
0
20
40
60
80
100
時間(秒)
(1)
(2)
(3)
TIME (Second)

FIG. 28

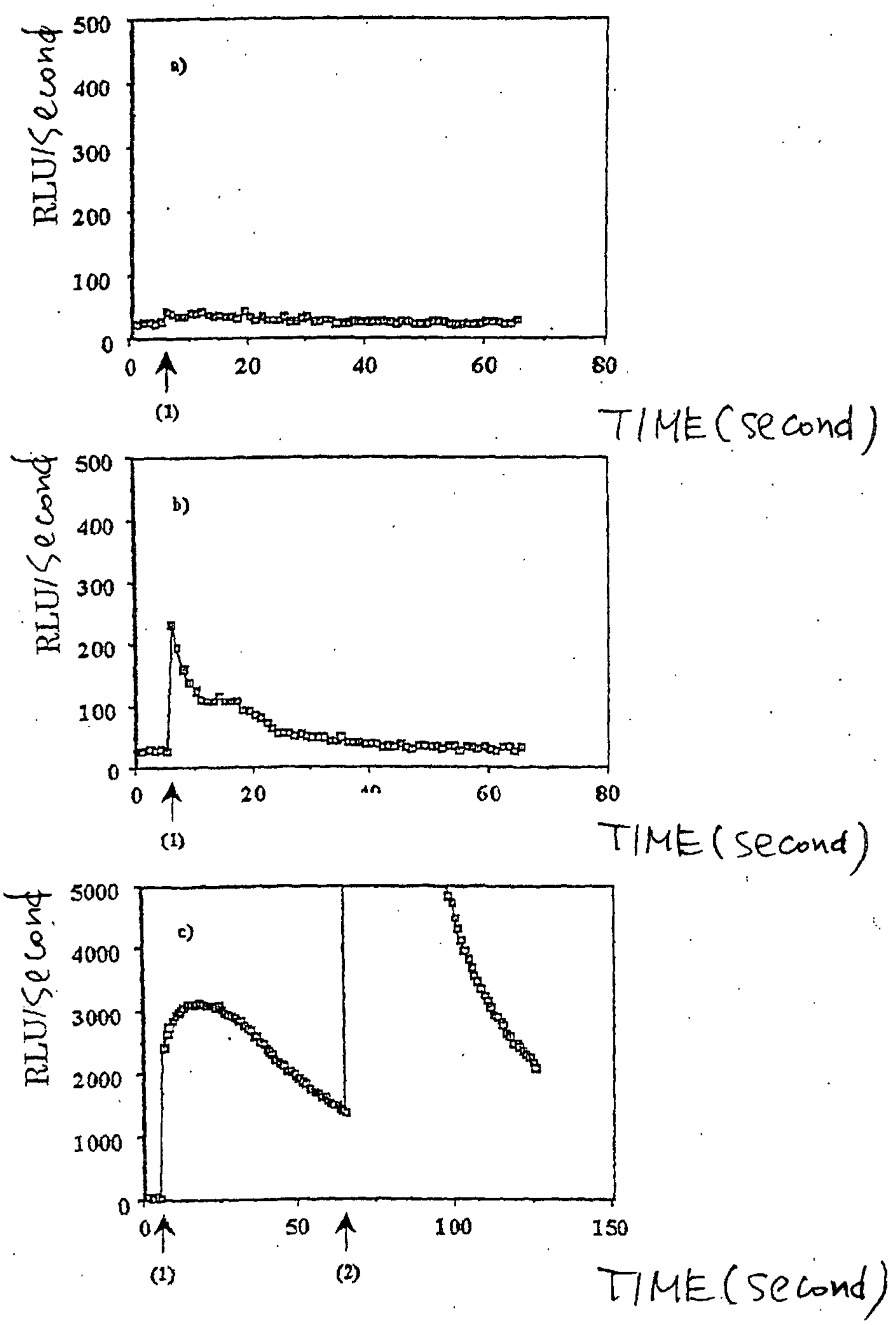
RLU/Second
500
400
300
200
100
0
a)
0
20
40
60
80
(1)
TIME (second)
RLU/Second
500
400
300
200
100
0
b)
0
20
40
60
80
(1)
TIME (second)
RLU/Second
5000
4000
3000
2000
1000
0
c)
0
50
100
150
(1)
(2)
TIME (second)

FIG. 29

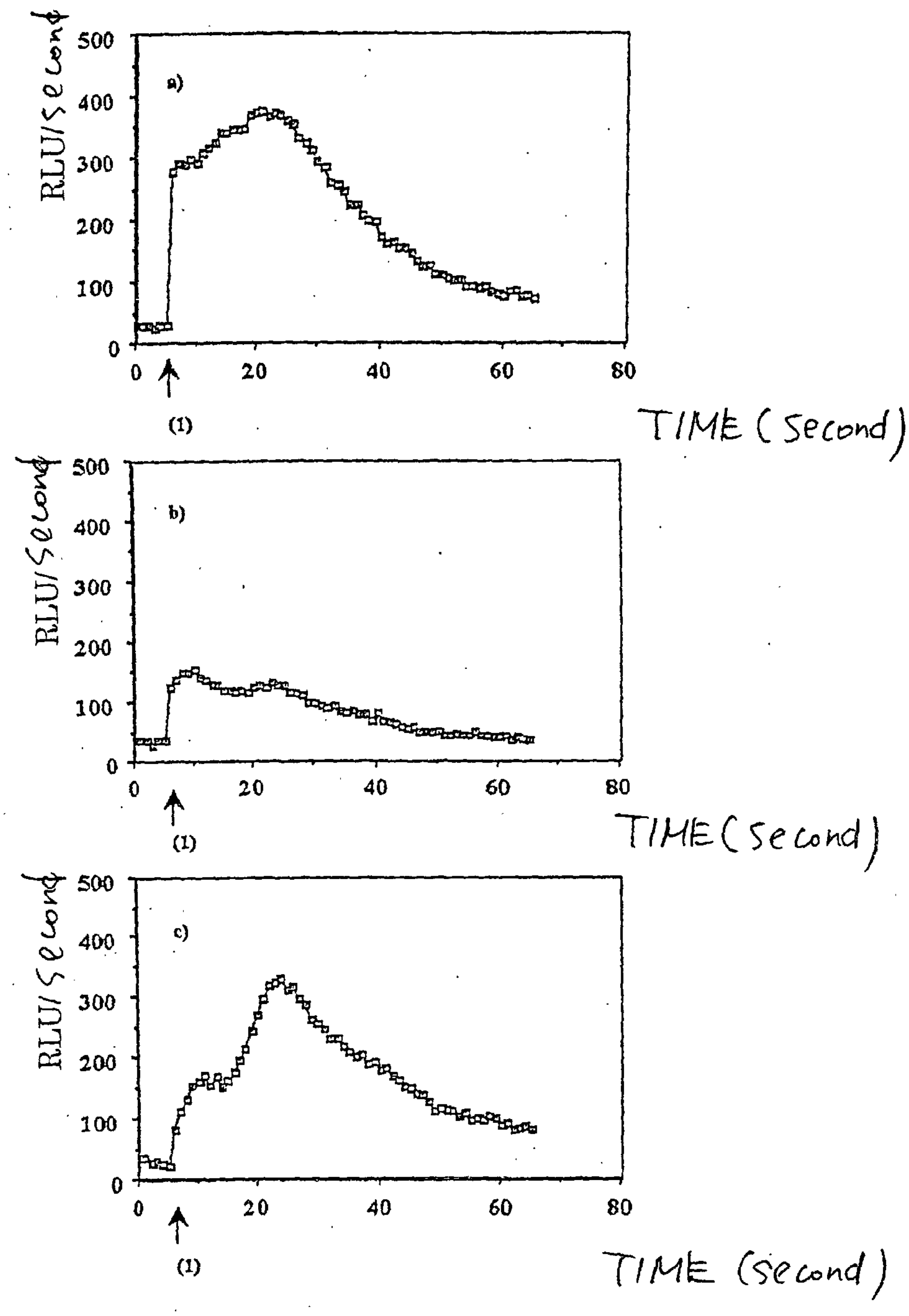
RLU/second
500
400
300
200
100
0
a)
0
20
40
60
80
(1)
TIME (second)
RLU/second
500
400
300
200
100
0
b)
0
20
40
60
80
(1)
TIME (second)
RLU/second
500
400
300
200
100
0
c)
0
20
40
60
80
(1)
TIME (second)

FIG.30

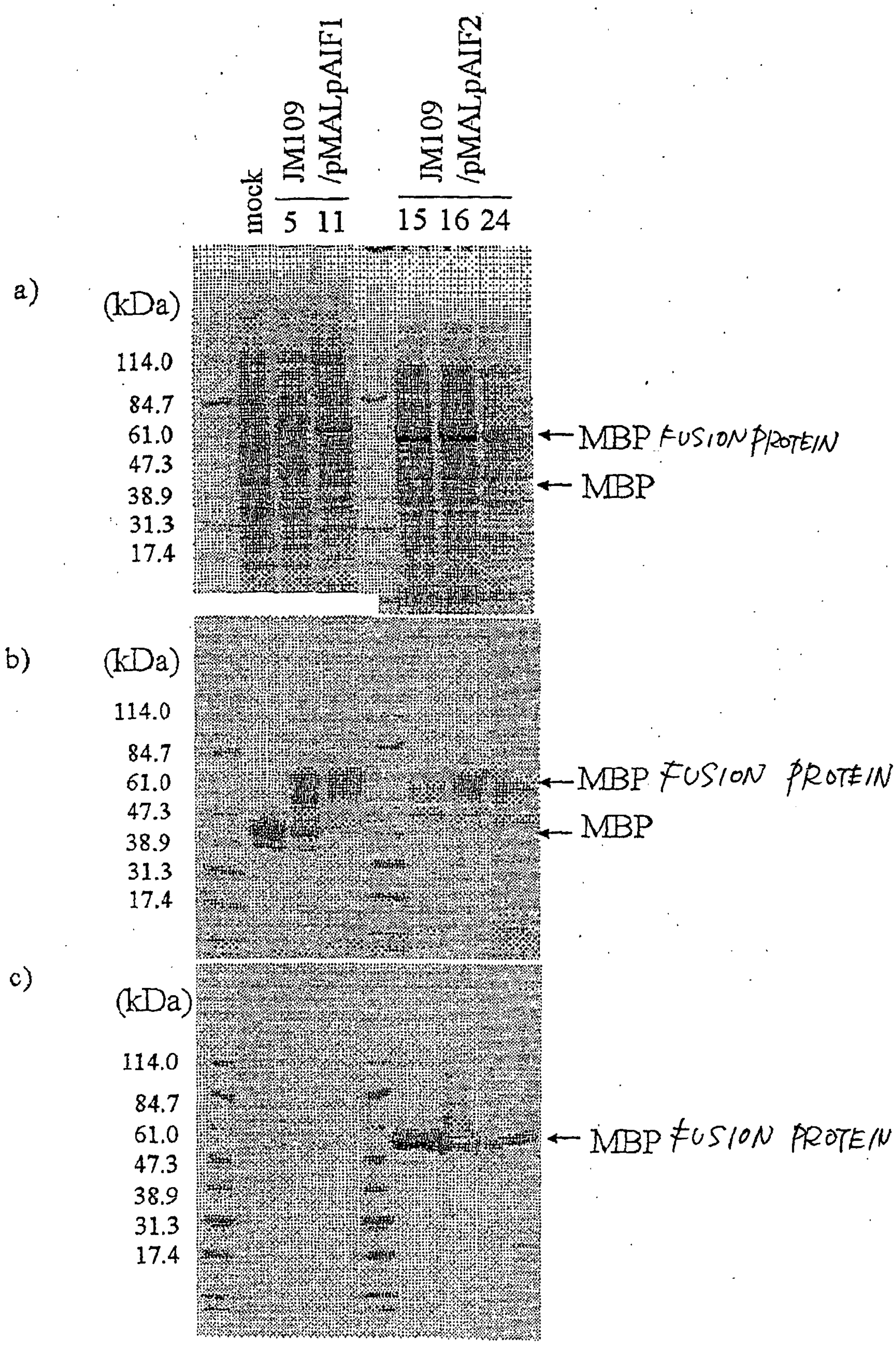
mock
JM109
/pMALpAIF1
5 11
JM109
/pMALpAIF2
15 16 24
a)
(kDa)
114.0
84.7
61.0
47.3
38.9
31.3
17.4
MBP FUSION PROTEIN
MBP
b)
(kDa)
114.0
84.7
61.0
47.3
38.9
31.3
17.4
MBP FUSION PROTEIN
MBP
c)
(kDa)
114.0
84.7
61.0
47.3
38.9
31.3
17.4
MBP FUSION PROTEIN

FIG.31

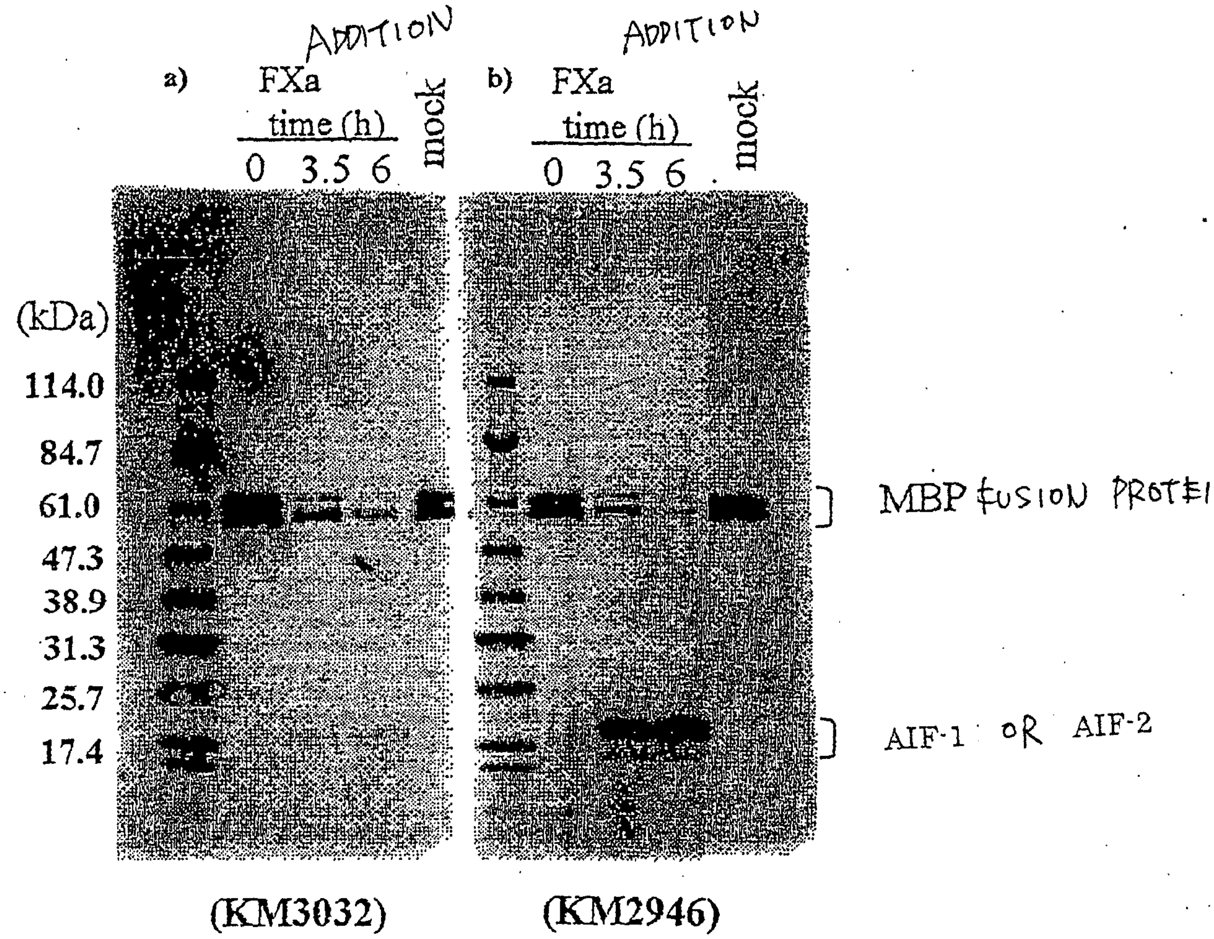
ADDITION
a) FXa
time (h)
0 3.5 6
mock
ADDITION
b) FXa
time (h)
0 3.5 6
mock
(kDa)
114.0
84.7
61.0
47.3
38.9
31.3
25.7
17.4
MBP FUSION PROTEI
AIF-1 OR AIF-2
(KM3032)
(KM2946)

FIG. 32

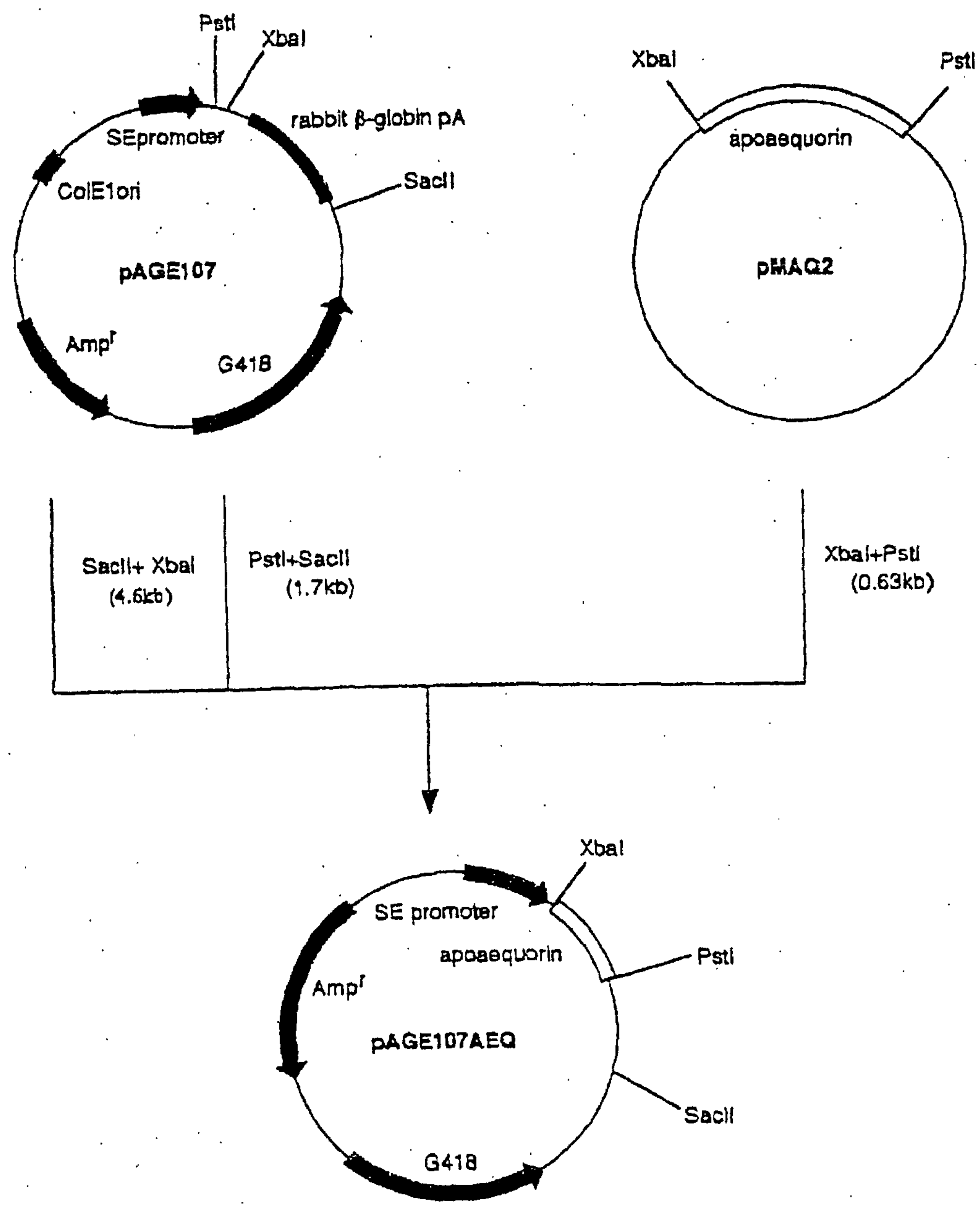

PstI
XbaI
SEpromoter
rabbit β-globin pA
ColE1ori
SacII
pAGE107
Ampr
G418
XbaI
PstI
apoaequorin
pMAQ2
SacII+ XbaI (4.6kb)
PstI+SacII (1.7kb)
XbaI+PstI (0.63kb)
XbaI
SE promoter
apoaequorin
PstI
Ampr
pAGE107AEQ
SacII
G418

FIG. 33

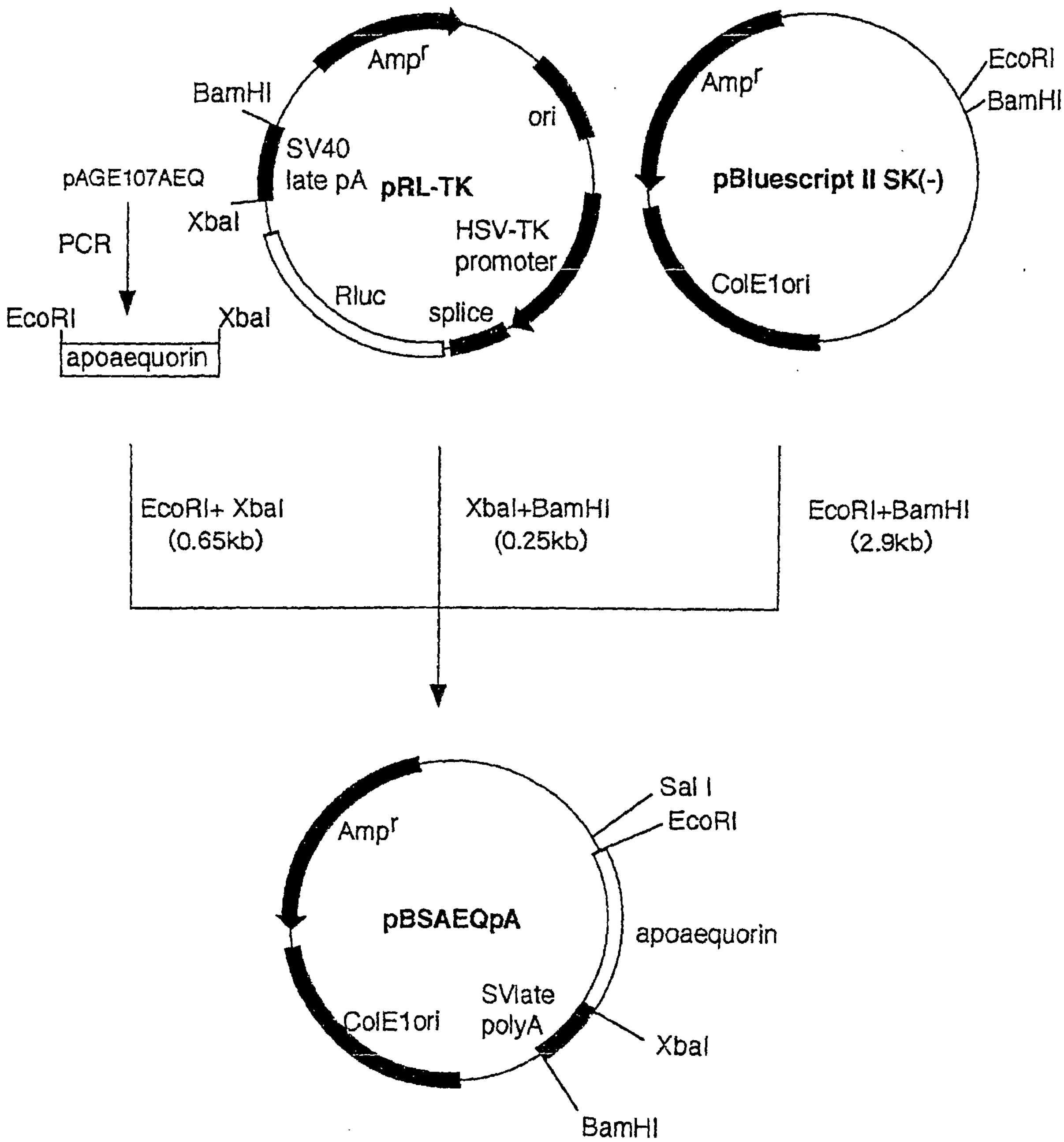
pAGE107AEQ
PCR
EcoRI
XbaI
apoaequorin
BamHI
SV40
late pA
pRL-TK
Amp^r
ori
HSV-TK
promoter
Rluc
splice
XbaI
pBluescript II SK(-)
Amp^r
EcoRI
BamHI
ColE1ori
EcoRI+ XbaI
(0.65kb)
XbaI+BamHI
(0.25kb)
EcoRI+BamHI
(2.9kb)
pBSAEQpA
Amp^r
Sal I
EcoRI
apoaequorin
SVlate
polyA
XbaI
BamHI
ColE1ori

FIG.34

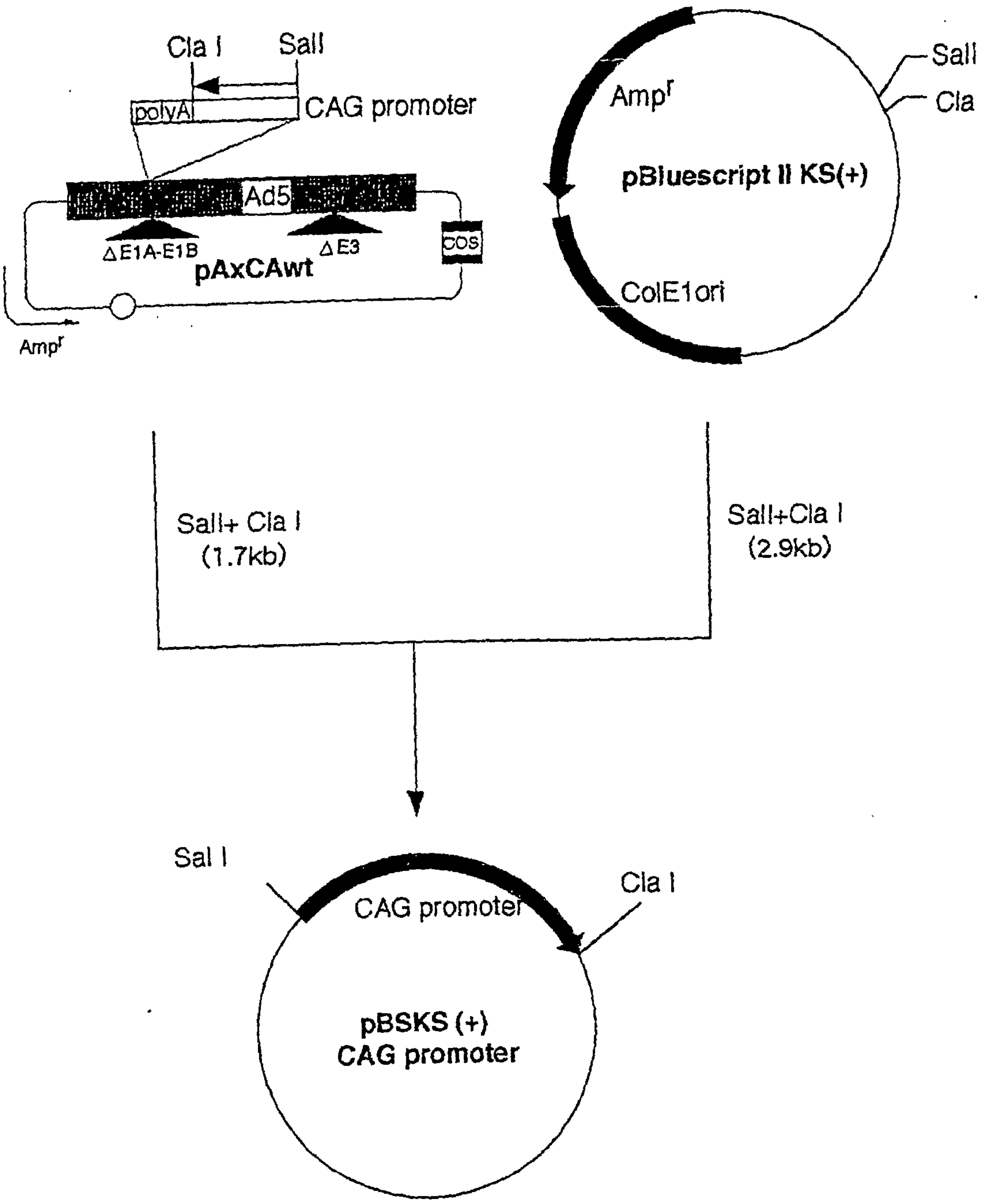
Cla I
SalI
CAG promoter
polyA
Ad5
ΔE1A-E1B
ΔE3
cos
pAxCAwt
Ampr
SalI
Cla I
Ampr
pBluescript II KS(+)
ColE1ori
SalI+ Cla I
(1.7kb)
SalI+Cla I
(2.9kb)
Sal I
Cla I
CAG promoter
pBSKS (+)
CAG promoter

FIG. 35

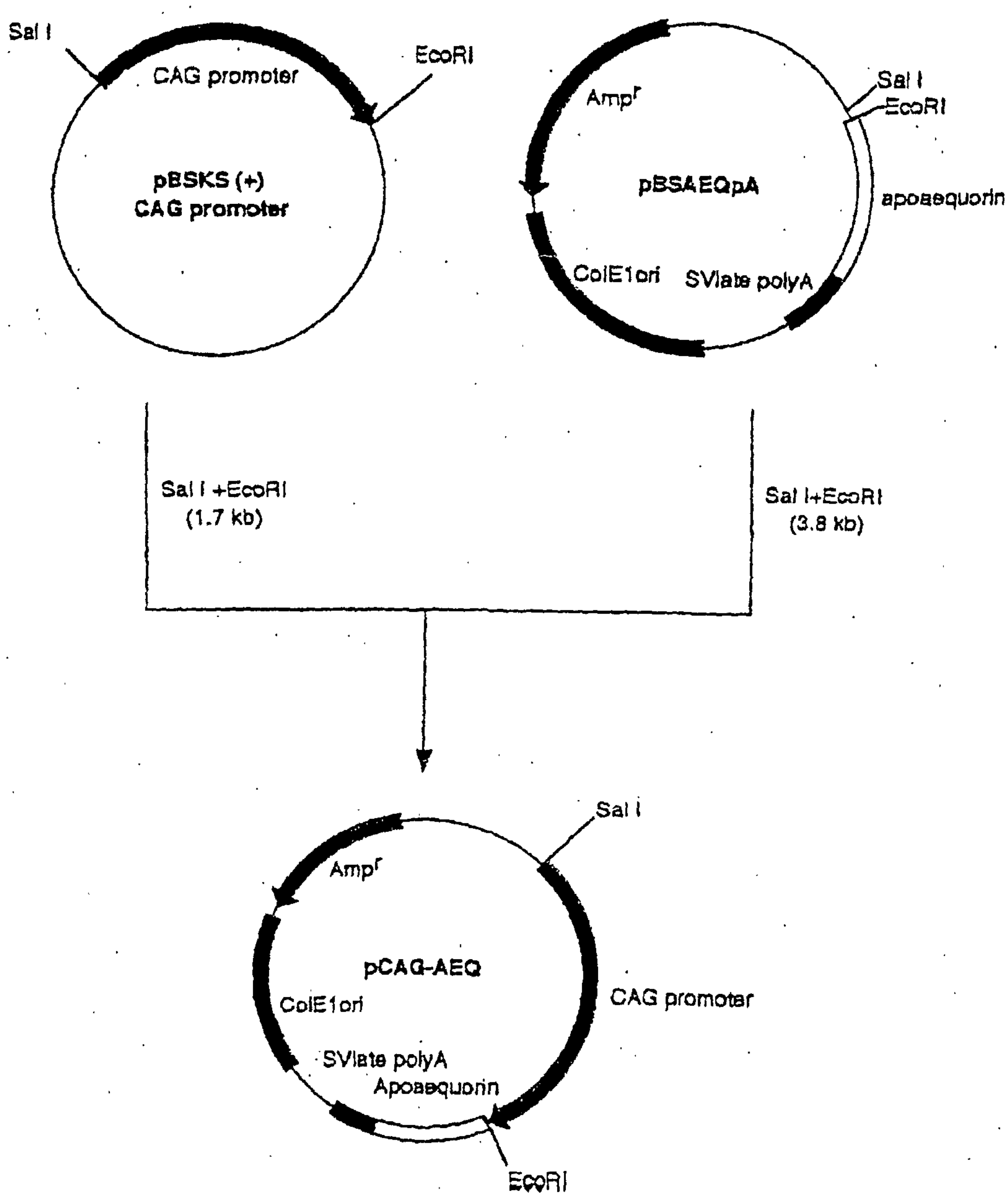
Sal I
CAG promoter
EcoRI
pBSKS (+)
CAG promoter
Amp$^r$
Sal I
EcoRI
pBSAEQpA
apoaequorin
ColE1ori
SVlate polyA
Sal I +EcoRI
(1.7 kb)
Sal I+EcoRI
(3.8 kb)
Sal I
Amp$^r$
pCAG-AEQ
ColE1ori
CAG promoter
SVlate polyA
Apoaequorin
EcoRI

FIG.36

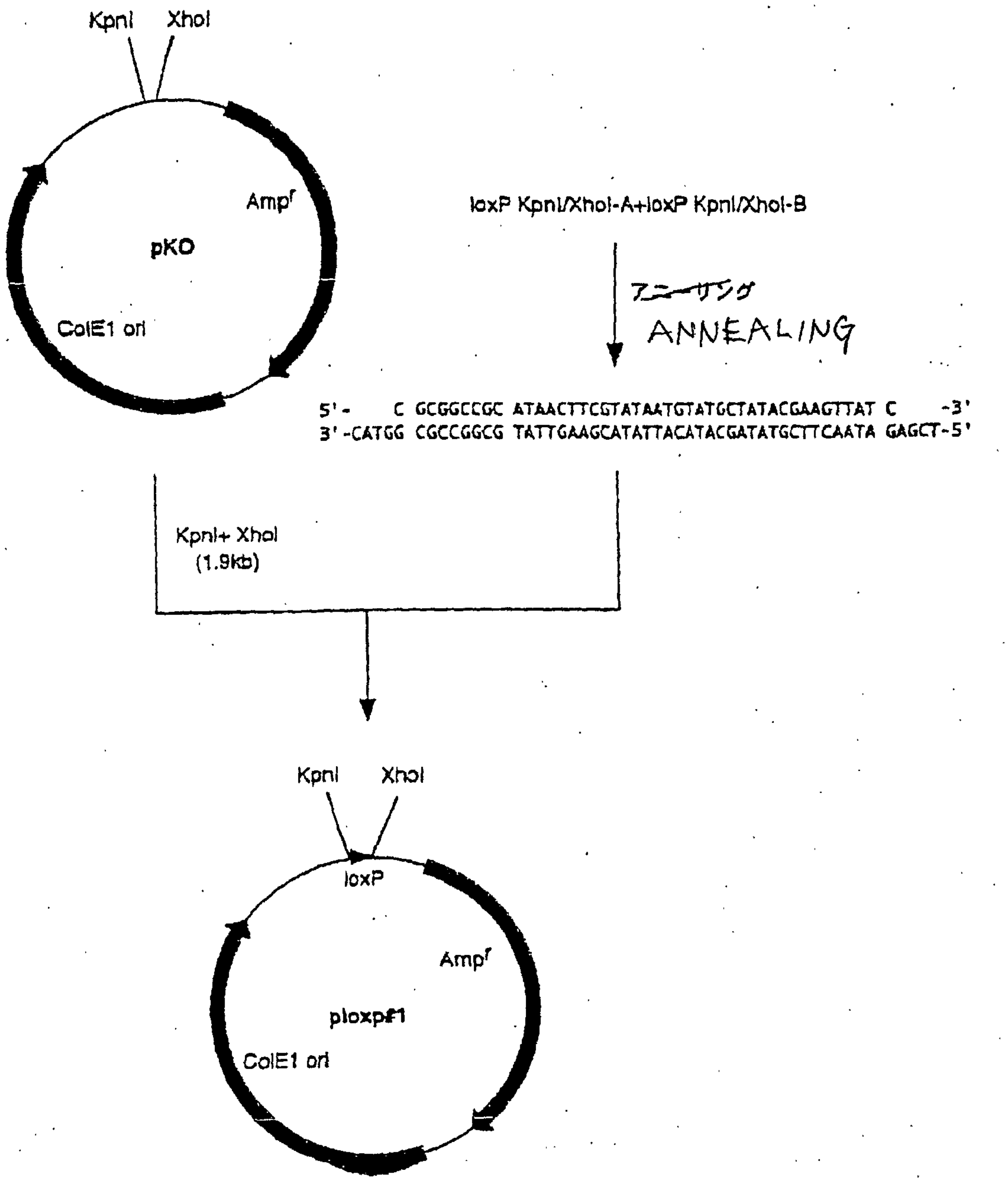
KpnI
XhoI
Amp^r
pKO
ColE1 ori
loxP KpnI/XhoI-A+loxP KpnI/XhoI-B
アニーリング
ANNEALING
5'-     C GCGGCCGC ATAACTTCGTATAATGTATGCTATACGAAGTTAT C     -3'
3'-CATGG CGCCGGCG TATTGAAGCATATTACATACGATATGCTTCAATA GAGCT-5'
KpnI+ XhoI
(1.9kb)
KpnI
XhoI
loxP
Amp^r
ploxp#1
ColE1 ori

FIG.37

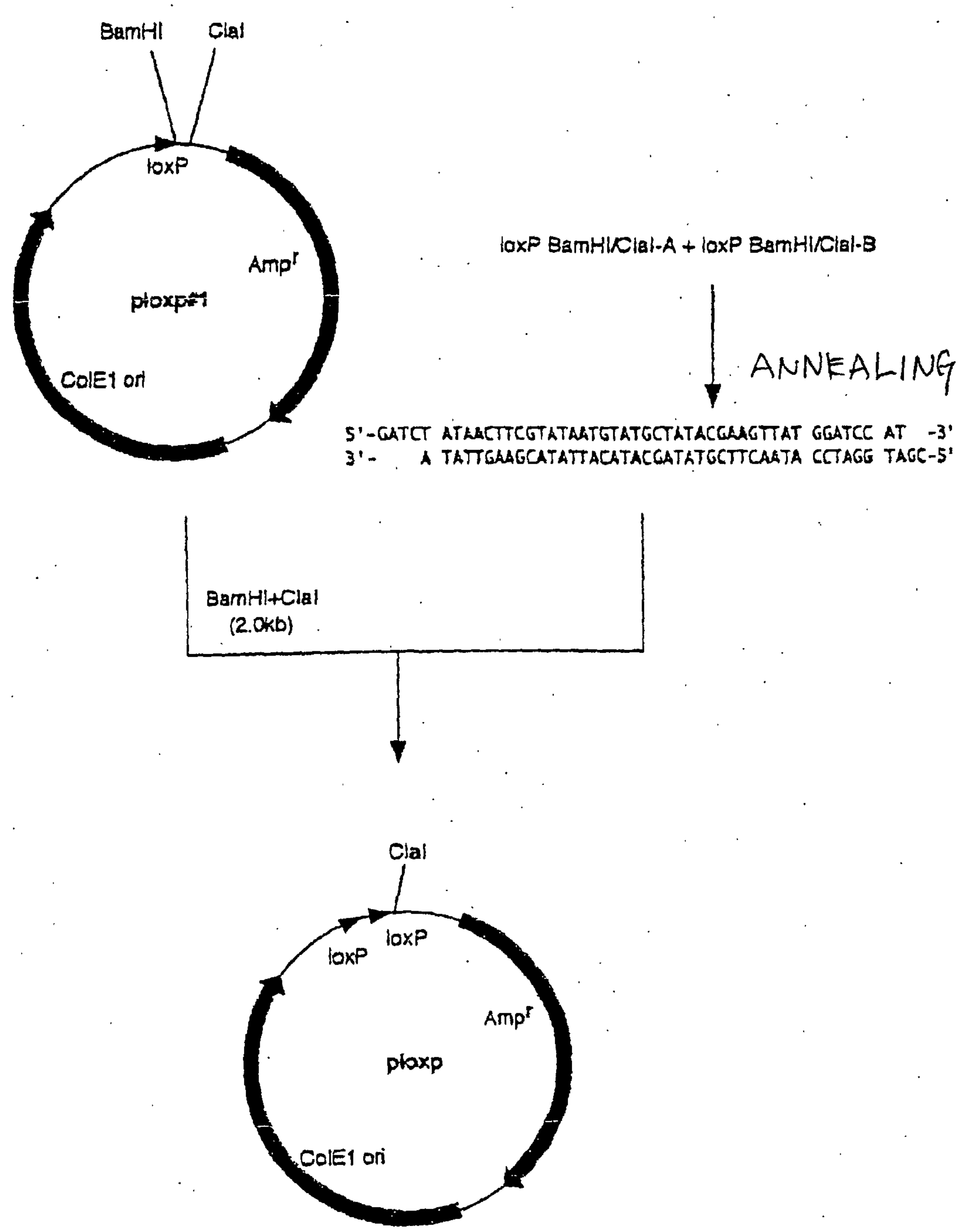
BamHI
ClaI
loxP
Amp$^r$
ploxp#1
ColE1 ori
loxP BamHI/ClaI-A + loxP BamHI/ClaI-B
ANNEALING
5'-GATCT ATAACTTCGTATAATGTATGCTATACGAAGTTAT GGATCC AT -3'
3'- A TATTGAAGCATATTACATACGATATGCTTCAATA CCTAGG TAGC-5'
BamHI+ClaI
(2.0kb)
ClaI
loxP
loxP
Amp$^r$
ploxp
ColE1 ori

FIG. 38

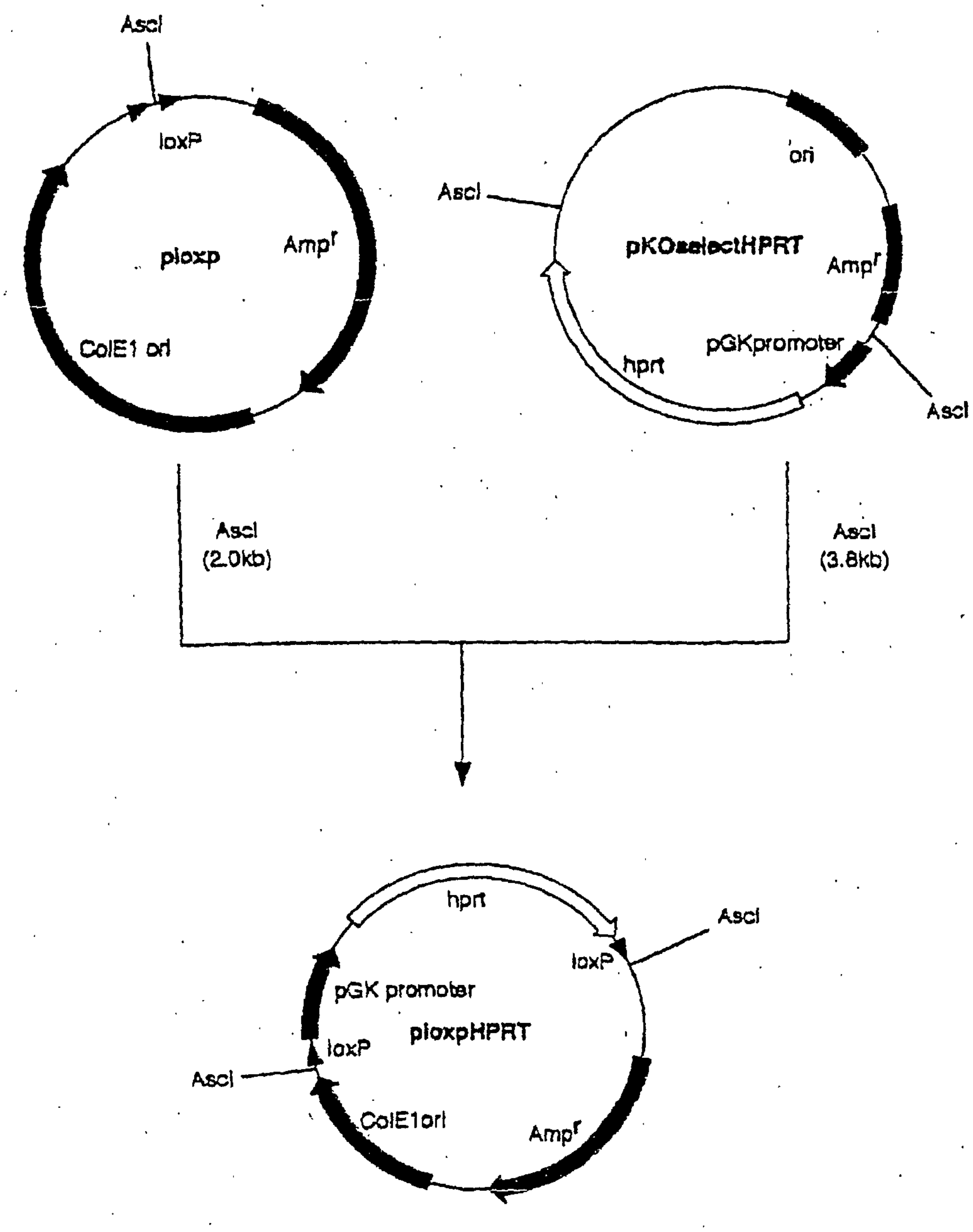
AscI
loxP
ploxp
Ampr
ColE1 ori
AscI
ori
pKOselectHPRT
Ampr
hprt
pGKpromoter
AscI
AscI
(2.0kb)
AscI
(3.8kb)
hprt
AscI
loxP
pGK promoter
ploxpHPRT
loxP
AscI
ColE1ori
Ampr

FIG. 39

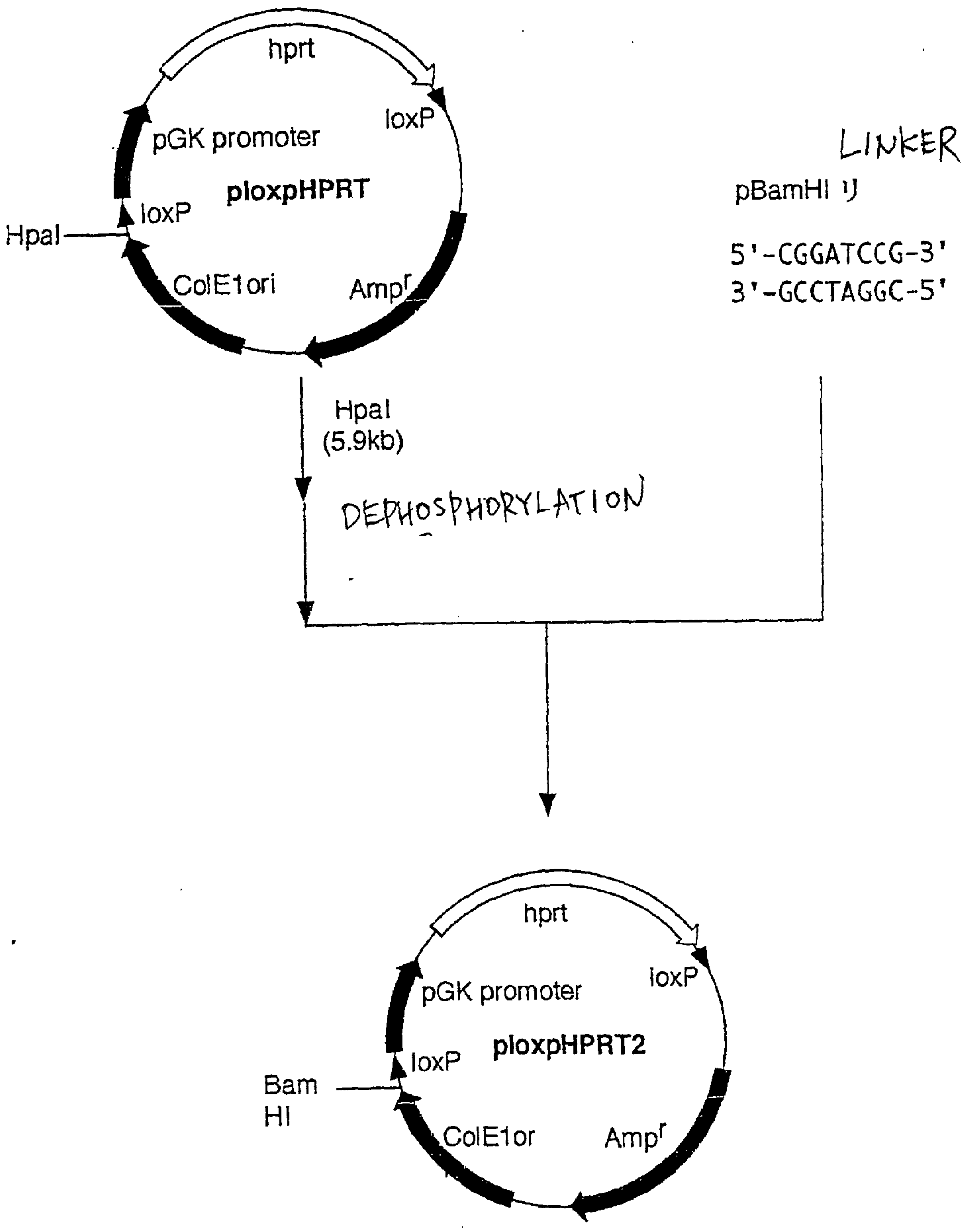
hprt
loxP
pGK promoter
ploxpHPRT
loxP
HpaI
ColE1ori
Amp^r
LINKER
pBamHI リ
5'-CGGATCCG-3'
3'-GCCTAGGC-5'
HpaI
(5.9kb)
DEPHOSPHORYLATION
hprt
pGK promoter
loxP
ploxpHPRT2
loxP
Bam
HI
ColE1or
Amp^r

FIG. 40

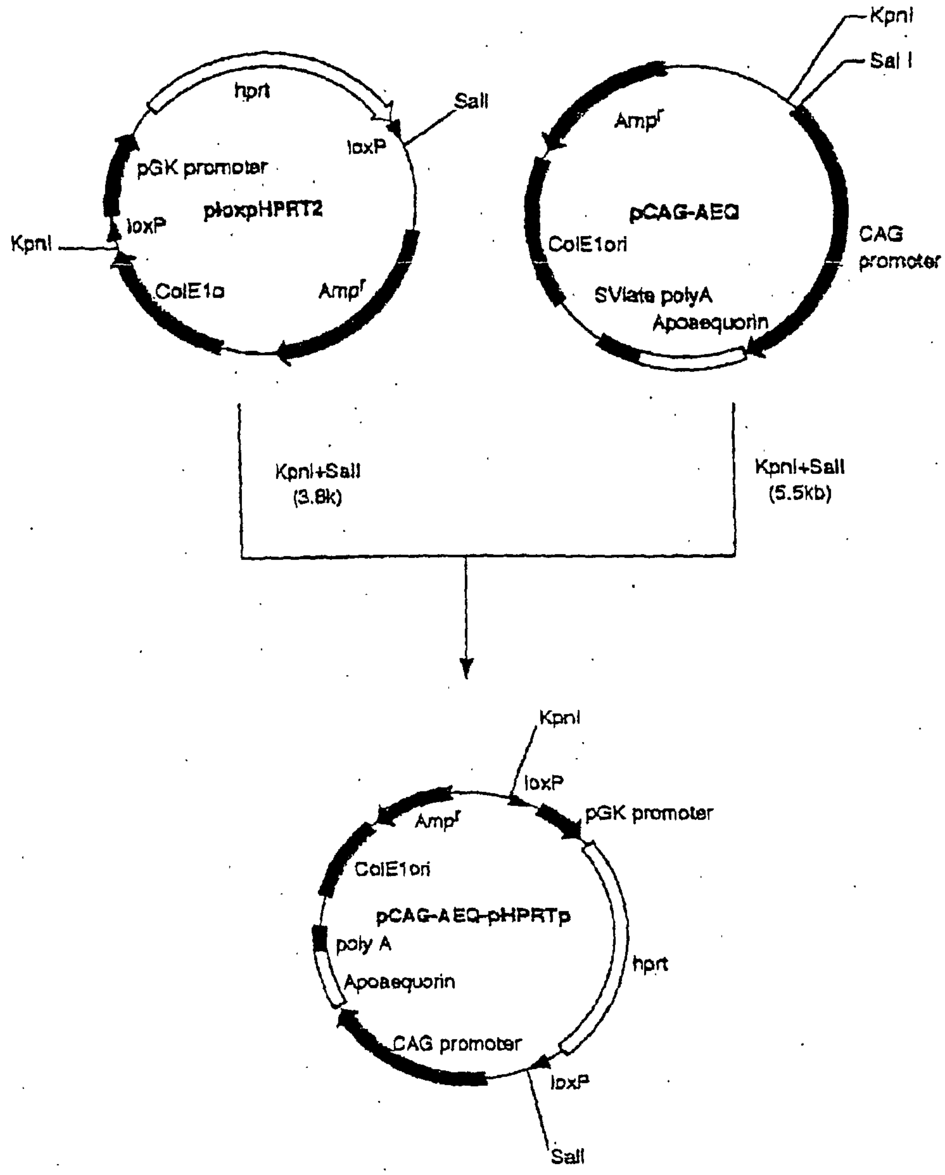
hprt
SalI
loxP
pGK promoter
ploxpHPRT2
loxP
KpnI
ColE1o
Ampr
KpnI
Sal I
Ampr
pCAG-AEQ
ColE1ori
CAG promoter
SVlate polyA
Apoaequorin
KpnI+SalI (3.8k)
KpnI+SalI (5.5kb)
KpnI
loxP
pGK promoter
Ampr
ColE1ori
pCAG-AEQ-pHPRTp
poly A
hprt
Apoaequorin
CAG promoter
loxP
SalI

FIG.41

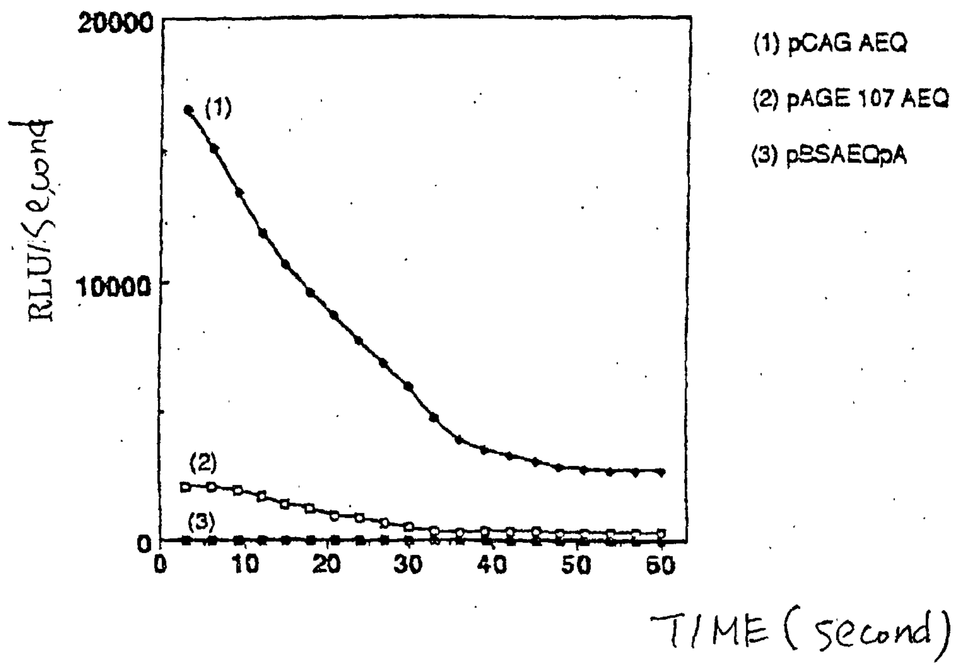
RLU/Second
20000
10000
0
(1)
(2)
(3)
0
10
20
30
40
50
60
TIME (second)
(1) pCAG AEQ
(2) pAGE 107 AEQ
(3) pBSAEQpA

FIG.42

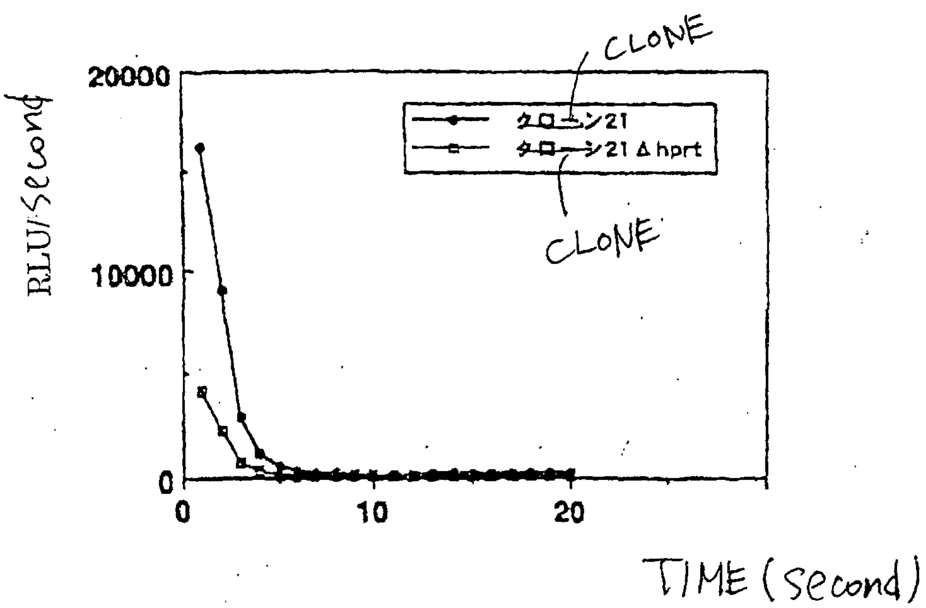
RLU/Second
20000
10000
0
0
10
20
TIME (second)
クローン21
クローン21 Δ hprt
CLONE
CLONE

**INTERNATIONAL SEARCH REPORT**

International application No. PCT/JP01/06633

A. CLASSIFICATION OF SUBJECT MATTER
Int.Cl[7] C12N15/09, C07K7/08, C07K14/47, C07K16/18, C12N5/10, C12P21/08, C12Q1/68, A61K38/17, A61K39/395, A61K48/00, A61P1/00, 3/10, 9/00, 9/08, 9/10, 11/06, 13/12, 17/06, 19/02, 25/02, 27/06, 27/14, A61P27/16, 35/00, 37/06, 37/08, G01N33/15, G01N33/50, G01N33/53, G01N33/566, A01K67/027
According to International Patent Classification (IPC) or to both national classification and IPC

B. FIELDS SEARCHED
Minimum documentation searched (classification system followed by classification symbols)
Int.Cl[7] C12N15/09, C07K7/08, C07K14/47, C07K16/18

Documentation searched other than minimum documentation to the extent that such documents are included in the fields searched

Electronic data base consulted during the international search (name of data base and, where practicable, search terms used)
SwissProt/PIR/Geneseq, WPI (DIALOG), BIOSIS (DIALOG)

C. DOCUMENTS CONSIDERED TO BE RELEVANT

| Category* | Citation of document, with indication, where appropriate, of the relevant passages | Relevant to claim No. |
|---|---|---|
| A | EP 879882 A1 (SmithKline Beecham Corporation), 25 November, 1998 (25.11.98), Sequence Identification No.: 2 & JP 11-184 A | 1-37,39-52 |
| A | Utans, U. et al., "Cloning and Characterization of Allograft Inflammatory factor-1: A Novel Macrophage factor Identified in Rat Cardiac allografts with Chronic Rejection", J. Clin. Invest., Vol.95, No.6, pages 2954 to 2962, (1995) | 1-37,39-52 |
| A | Chen, Z. W. et al., "Identification, isolation, and characterization of daintain (allograft inflammatory factor 1), a macrophage polypeptide with effects on insulin secretion and abundantly present in the pancreas of prediabetic BB rats", Proc. Natl. Acad. Sci. USA, Vol.94, No.25, pages 13879 to 13884, (1997) | 1-37,39-52 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 October, 2001 (03.10.01) | 23 October, 2001 (23.10.01) |
| Name and mailing address of the ISA/ Japanese Patent Office | Authorized officer |
| Facsimile No. | Telephone No. |

Form PCT/ISA/210 (second sheet) (July 1992)

INTERNATIONAL SEARCH REPORT

International application No.
PCT/JP01/06633

C (Continuation). DOCUMENTS CONSIDERED TO BE RELEVANT

| Category* | Citation of document, with indication, where appropriate, of the relevant passages | Relevant to claim No. |
|---|---|---|
| A | WO 98/31800 A2 (Human Genome Sciences, Inc.), 23 July, 1998 (23.07.98), Fig. 6 & US 6046031 A & JP 2001-509029 A | 1-37,39-52 |

Form PCT/ISA/210 (continuation of second sheet) (July 1992)

## INTERNATIONAL SEARCH REPORT

International application No.
PCT/JP01/06633

**Box I Observations where certain claims were found unsearchable (Continuation of item 1 of first sheet)**

This international search report has not been established in respect of certain claims under Article 17(2)(a) for the following reasons:

1. ☒ Claims Nos.: 38
because they relate to subject matter not required to be searched by this Authority, namely:
It provides an invention relating to diagnostic methods to be practiced on the human or animal body.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)